# EUROPEAN PATENT APPLICATION

(11) **EP 3 777 863 A1**
(43) Date of publication of application: **17.02.2021**
(21) Application number: 20196183.6
(22) Date of filing: 11.09.2015
(51) Int. Cl.: A61K 31/55, A61K 31/4164, A61K 31/496, A61P 17/00, A61K 45/06, A61K 9/20, A61K 31/195, A61K 31/415, A61K 31/4178, A61K 31/4439, A61P 1/16, A61P 3/10, A61P 13/12, A61P 31/14, A61P 31/18, A61P 31/20, A61P 19/04

(54) **CENICRIVIROC COMBINATION THERAPY FOR THE TREATMENT OF FIBROSIS**

(30) Priority: 12.09.2014 US 201462049591 P; 06.11.2014 US 201462076264 P
(62) Divisional of application: 15840102.6
(71) Applicant: Tobira Therapeutics, Inc., South San Francisco, CA 94080 (US)
(72) Inventor: Lefebvre, Eric, South San Francisco, CA California 94080 (US)
(74) Representative: J A Kemp LLP

(57) **Abstract**

Cenicriviroc (CVC) is an orally active antagonist of ligand binding to C-C chemokine receptor type 5 (CCR5) and C-C chemokine receptor type 2 (CCR2). CVC blocks the binding of RANTES, MIP-1α, and MIP-1β to CCR5, and of MCP-1/CCL2 to CCR2. Methods of treating fibrosis and related conditions comprising co-administration of CVC with FXR agonists, high dose vitamin E (>400 iU/d), a peroxisome proliferator-activated receptor alpha (PPAR-α) agonist, PPAR-γ agonist, PPAR-δ agonist and/or chemokine antagonists are provided herein.

## Description

### RELATED APPLICATIONS

This application claims the benefit of U.S. Application No. 62/076,264, filed November 6, 2014, and U.S. Application No. 62/049,591, filed September 12, 2014, the contents of each is incorporated herein by reference in their entireties.

### FIELD

The present disclosure relates to pharmaceutical compositions containing cenicriviroc, methods for the preparation thereof, and their use in a combination therapy for the treatment of inflammation and connective tissue diseases and disorders, such as fibrosis including NASH.

### BACKGROUND

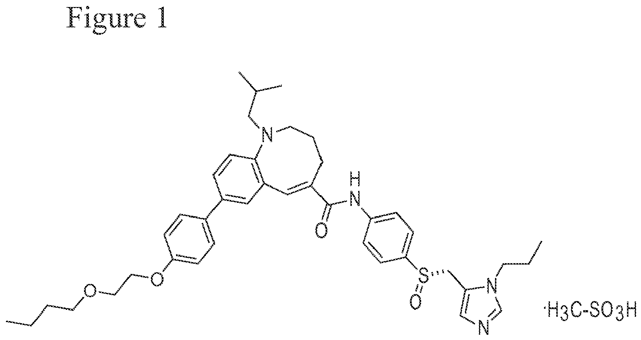

Cenicriviroc (also known as CVC) is the common name of (S,E)-8-(4-(2-Butoxyethoxy)phenyl)-1-(2-methylpropyl)-N-(4-(((1-propyl-1H-imidazol-5-yl)methyl)sulfinyl)phenyl)-1,2,3,4-tetrahydrobenzo[b]azocine-5-carboxamide. The chemical structure of cenicriviroc mesylate appears in Figure 1. Cenicriviroc binds to and inhibits the activity of the C-C chemokine receptor type 2 (CCR2) and C-C chemokine receptor type 5 (CCR5) receptors (24). These receptors not only play a role in entry of viruses such as Human Immunodeficiency Virus (HIV) into the cell, but also are important for the recruitment of immune cells to sites of injury. Inhibition of this receptor's activity may have an anti-inflammatory effect. More recently, the role that inflammation plays in the development of fibrosis has been examined [30]. It has been shown that C-C chemokine receptor type 2 (CCR2) and CCR5 may play a role in promoting hepatic fibrosis [3, 4, 5, 31 32].

### SUMMARY OF THE INVENTION

In one embodiment, the invention provides a method of treating fibrosis or a fibrotic disease or condition in a subject in need thereof comprising co-administering to the subject a therapeutically effective amount of cenicriviroc or a salt or solvate thereof; and one or more additional active agents. In a further embodiment, the additional active agent is an anti-inflammatory agent. In a further embodiment, the additional active agent is a chemokine receptor antagonist. In a further embodiment, the additional active agent inhibits the binding of a chemokine to a chemokine receptor. In a further embodiment, the additional active agent inhibits the binding of ligand to CCR1. In a further embodiment, the additional active agent inhibits the binding of CCR5 ligands to CCR1. In a further embodiment, the additional active agent is selected from the group consisting of a farnesoid X receptor (FXR) agonist, high dose vitamin E (> 400 iU/d), and a peroxisome proliferator-activated receptor alpha, gamma, and delta (PPAR-α, -γ, and-δ) agonist. In another further embodiment, the additional active agent is selected from the group consisting of obeticholic acid, pioglitazone, 3-[2-[2-Chloro-4-[[3-(2,6-dichlorophenyl)-5-(1-methylethyl)-4-isoxazolyl]methoxy]phenyl]ethenyl]benzoic acid (GW4064), 2-methyl-2-[[4-[2-[[(cyclohexylamino)carbonyl](4-cyclohexylbutyl)amino]ethyl]phenyl]thio]-propanoic acid (GW7647), and 2-[2,6 dimethyl-4-[3-[4-(methylthio)phenyl]-3-oxo-1(E)-propenyl]phenoxyl]-2-methylpropanoic acid (GFT505).

In one embodiment, the fibrosis or fibrotic disease or condition is liver fibrosis or renal fibrosis. In a further embodiment, the liver fibrosis is associated with non-alcoholic steatohepatitis (NASH). In another embodiment, the liver fibrosis is associated with non-alcoholic fatty liver disease (NAFLD). In a further embodiment, the liver fibrosis is associated with emerging cirrhosis. In another further embodiment, the liver fibrosis comprises non-cirrhotic hepatic fibrosis. In one embodiment, the subject is infected by human immunodeficiency virus (HIV). In another embodiment, the subject has a disease or condition selected from the group consisting of alcoholic liver disease, HIV and HCV co-infection, viral hepatitis (such as HBV or HCV infection), type 2 diabetes mellitus (T2DM), metabolic syndrome (MS), and a combination thereof.

In one embodiment, the present invention provides a method of treating NASH in a subject in need thereof comprising co-administering to the subject a therapeutically effective amount of cenicriviroc, or a salt or solvate thereof; wherein the NASH is associated with type 2 diabetes mellitus (T2DM); and one or more additional active agents.

In one embodiment, the present invention provides a method of treating NASH in a subject in need thereof comprising co-administering to the subject a therapeutically effective amount of cenicriviroc, or a salt or solvate thereof; wherein the NASH is associated with metabolic syndrome (MS); and one or more additional active agents.

In one embodiment, the present invention provides a method of treating NASH in a subject in need thereof comprising co-administering to the subject a therapeutically effective amount of cenicriviroc, or a salt or solvate thereof; and one or more additional active agents; wherein the NASH is associated with HIV and HCV co-infection.

In one embodiment, the additional active agent is selected from the group consisting of a farnesoid X receptor (FXR) agonist and a peroxisome proliferator-activated receptor alpha (PPAR-α and -δ) agonist. In a further embodiment, the additional active agent is selected from the group consisting of obeticholic acid, 3-[2-[2-Chloro-4-[[3-(2,6-dichlorophenyl)-5-(1-methylethyl)-4-isoxazolyl]methoxy]phenyl]ethenyl]benzoic acid (GW4064), 2-methyl-2-[[4-[2-[[(cyclohexylamino)carbonyl](4-cyclohexylbutyl)amino]ethyl]phenyl]thio]-propanoic acid (GW7647), and 2-[2,6 dimethyl-4-[3-[4-(methylthio)phenyl]-3-oxo-1(E)-propenyl]phenoxyl]-2-methylpropanoic acid (GFT505).

In one embodiment, the cenicriviroc or a salt or solvate thereof is formulated as a pharmaceutical composition comprising cenicriviroc or a salt or solvate thereof and fumaric acid. In one embodiment, the cenicriviroc or salt or solvate thereof is formulated as an oral composition. In one embodiment, the cenicriviroc or salt or solvate thereof is administered once per day or twice per day. In another embodiment, the co-administration comprises simultaneous administration, sequential administration, overlapping administration, interval administration, continuous administration, or a combination thereof. In a further embodiment, the co-administration is carried out for one or more treatment cycles. In another embodiment, the co-administration is carried out for 1 to 24 treatment cycles. In a further embodiment, each of the treatment cycle comprises about 7 or more days. In yet a further embodiment, each of the treatment cycle comprises about 28 or more days. In another embodiment, the co-administration comprises one or more treatment cycles, and each treatment cycle comprises about 28 days.

In one embodiment, the co-administration comprises oral administration, parenteral administration, or a combination thereof. In a further embodiment, the parenteral administration comprises intravenous administration, intraarterial administration, intramuscular administration, subcutaneous administration, intraosseous administration, intrathecal administration, or a combination thereof. In one embodiment, cenicriviroc or a salt or solvate thereof is administered orally; and the additional active agent is administered orally or parenterally.

In one embodiment, the co-administration comprises simultaneous administration. In a further embodiment, cenicriviroc or a salt or solvate thereof and the additional active agent are co-administered simultaneously for about 28 days or more.

In another embodiment, the invention provides a method further comprising detecting a level of one or more biological molecules in the subject treated for fibrosis or the fibrotic disease or condition, and determining a treatment regimen based on an increase or decrease in the level of one or more biological molecules, wherein the biological molecule is selected from the group consisting of lipopolysaccharide (LPS), LPs-binding protein (LBP), 16S rDNA, sCD14, intestinal fatty acid binding protein (I-FABP), zonulin-1, Collagen 1a1 and 3a1, TGF-β, fibronectin-1, hs-CRP, IL-1β, IL-6, IL-33, fibrinogen, MCP-1, MIP-1α and -1β, RANTES, sCD163, TGF-β, TNF-α, a biomarker of hepatocyte apoptosis such as CK-18 (caspase-cleaved and total), and a combination thereof.

In another embodiment, the method further comprises detecting a level of one or biological molecules in the subject treated for fibrosis or the fibrotic disease or condition, wherein an increase or decrease in the level of one or more biological molecules compared to a predetermined standard level is predictive of the treatment efficacy of fibrosis or the fibrotic disease or condition, wherein the biological molecule is selected from the group consisting of lipopolysaccharide (LPS), LPS-binding protein (LBP), 16S rDNA, sCD14, intestinal fatty acid binding protein (I-FABP), zonulin-1, Collagen 1a1 and 3a1, TGF-β, fibronectin-1, hs-CRP, IL-1β, IL-6, IL-33, fibrinogen, MCP-1, MIP-1α and -1β, RANTES, sCD163, TGF-β, TNF-α, a biomarker of hepatocyte apoptosis such as CK-18 (caspase-cleaved and total), α2-macroglobulin, apolipoprotein A1, haptoglobin, hyaluronic acid, hydroxyproline, N-terminal propeptide of collagen type III, tissue inhibitors of metalloproteinases, and a combination thereof. In one embodiment, the one or more biological molecules are measured in a biological sample from a subject treated for fibrosis or the fibrotic disease or condition. In another embodiment, the biological sample is selected from blood, skin, hair follicles, saliva, oral mucous, vaginal mucous, sweat, tears, epithelial tissues, urine, semen, seminal fluid, seminal plasma, prostatic fluid, pre-ejaculatory fluid (Cowper's fluid), excreta, biopsy, ascites, cerebrospinal fluid, lymph, brain, and tissue extract sample or biopsy sample.

The present invention also provides a pharmaceutical composition comprising a therapeutically effective amount of cenicriviroc, or a salt or solvate thereof; and one or more additional active agents. In one embodiment, the pharmaceutical composition further comprises one or more pharmaceutically acceptable excipients. In a further embodiment, the pharmaceutically acceptable excipient comprises fumaric acid.

In one embodiment, the present invention provides a combination package comprising
(a) at least one individual dose of cenicriviroc, or a salt or solvate thereof; and
(b) at least one individual dose of one or more additional active agent.
In another embodiment, the combination package further comprises an instruction document providing a protocol for co-administering (a) and (b).

In one embodiment, the present invention provides a method of distributing an antifibrotic agent comprising distributing to a subject a predetermined amount of a first pharmaceutical composition comprising cenicriviroc, or a salt or solvate thereof, in combination with a predetermined amount of a second pharmaceutical composition comprising at least one or more active agents. In a further embodiment, the present invention provides a method of distributing an antifibrotic agent comprising distributing to a subject a predetermined amount of a first pharmaceutical composition comprising cenicriviroc, or a salt or solvate thereof, in combination with an instruction of administering the first pharmaceutical composition with a predetermined amount of a second pharmaceutical composition comprising at least one or more active agents.

### BRIEF DESCRIPTION OF THE DRAWINGS

Figure 1 is the chemical formula of cenicriviroc mesylate.
Figure 2 is a graph comparing the absolute bioavailability, in beagle dogs, of cenicriviroc mesylate compounded as an oral solution with that of cenicriviroc mesylate prepared by wet granulation and mixed with various acid solubilizer excipients.
Figure 3 is a graph of the total impurity and degradant content of different cenicriviroc formulations subjected to accelerated stability testing at 40° C and 75% relative humidity when packaged with a desiccant.
Figure 4 is a dynamic vapor sorption isotherm for different cenicriviroc formulations.
Figure 5 shows the study schematic of the evaluation of CVC in mouse UUO model of renal fibrosis. Vehicle control and CVC administered BID; anti-TGF-β1 antibody, compound 1D11 (positive control) administered i.p. QD, once daily; CVC, cenicriviroc; ip, intraperitoneal; PBS, phosphate buffered saline; QD, once daily; TGF, transforming growth factor; UUO, unilateral ureter occlusion
Figure 6 shows the change in body weight (Day 5) in each treatment group in mouse UUO model of renal fibrosis.
Figure 7 shows the Collagen Volume Fraction (CVF; % area) score in each treatment group in mouse UUO model of renal fibrosis. Data presented exclude a single outlier from an animal in the CVC 20 mg/kg/day group, which had a CVF value >2 standard deviations higher than any other animal in the group.
Figure 8A-B shows the mRNA expression from renal cortical tissue of sham-surgery
Figure 9 shows the change in body weight until week 9 in animals treated with Cenicriviroc (low or high dose).
Figure 10A-C shows the change in liver and body weight until week 9 in animals treated with Cenicriviroc (low or high dose). Panel A shows the change in body weight, Panel B shows the change in liver weight, and Panel C shows the change in the liver-to body weight ratio.
Figure 11A-F shows the whole blood and biochemistry of animals treated with Cenicriviroc (low or high dose) at week 9. Panel A shows Whole blood glucose, Panel B shows Plasma ALT, Panel C shows Plasma MCP-1, Panel D shows Plasma MIP-1β, Panel E shows Liver triglyceride, and Panel F shows Liver hydroxyproline.
Figure 12 shows the HE-stained liver sections of animals treated with Cenicriviroc (low or high dose) at week 9.
Figure 13 shows the NAFLD Activity score of animals treated with Cenicriviroc (low or high dose) at week 9.
Figure 14 shows representative photomicrographs of Sirius red-stained liver sections of animals treated with Cenicriviroc (low or high dose) at week 9.
Figure 15 shows representative photomicrographs of F4/80-immunostained liver sections of animals treated with Cenicriviroc (low or high dose) at week 9.
Figure 16 shows the percentages of inflammation area of animals treated with Cenicriviroc (low or high dose) at week 9.
Figure 17 shows representative photomicrographs of F4/80 and CD206 double-immunostained liver sections of animals treated with Cenicriviroc (low or high dose) at week 9.
Figure 18 shows the percentages of F4/80 and CD206 double positive cells of F4/80 positive cells of animals treated with Cenicriviroc (low or high dose) at week 9.
Figure 19 shows the representative photomicrographs of F4/80 and CD16/32 double-immunostained liver sections of animals treated with Cenicriviroc (low or high dose) at week 9.
Figure 20 shows the percentages of F4/80 and CD16/32 double positive cells of F4/80 positive cells of animals treated with Cenicriviroc (low or high dose) at week 9.
Figure 21 shows the M1/M2 ratio of animals treated with Cenicriviroc (low or high dose) at week 9.
Figure 22 shows representative photomicrographs of oil red-stained liver sections of animals treated with Cenicriviroc (low or high dose) at week 9.
Figure 23 shows the percentages of fat deposition area of animals treated with Cenicriviroc (low or high dose) at week 9.
Figure 24 shows representative photomicrographs of TUNEL-positive cells in livers of animals treated with Cenicriviroc (low or high dose) at week 9.
Figure 25 shows percentages of TUNEL-positive cells of animals treated with Cenicriviroc (low or high dose) at week 9.
Figure 26 shows quantitative RT-PCR of animals treated with Cenicriviroc (low or high dose) at week 9. The levels of TNF-α, MCP-1, Collagen Type 1, and TIMP-1 were measured.

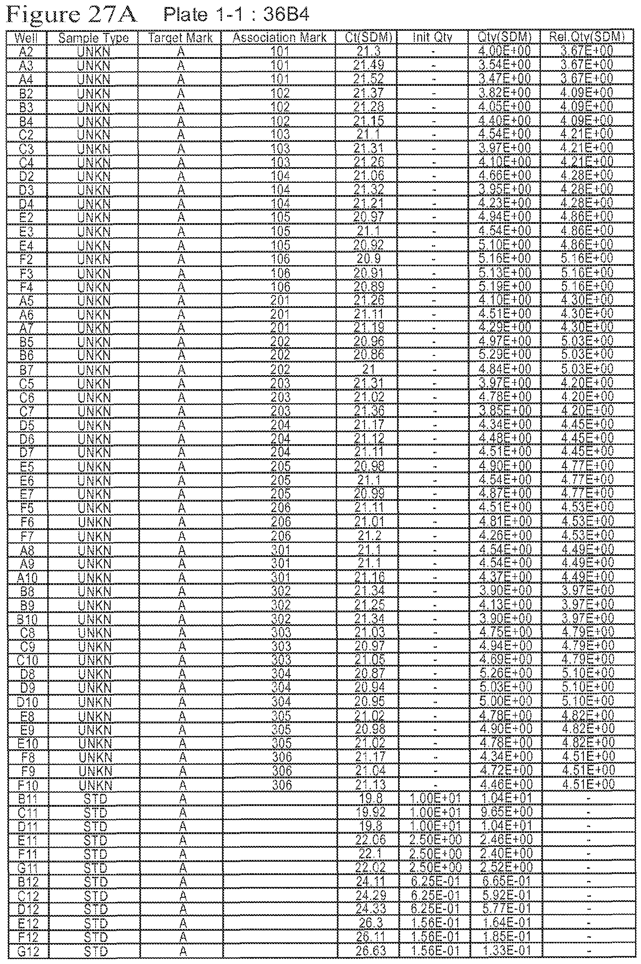

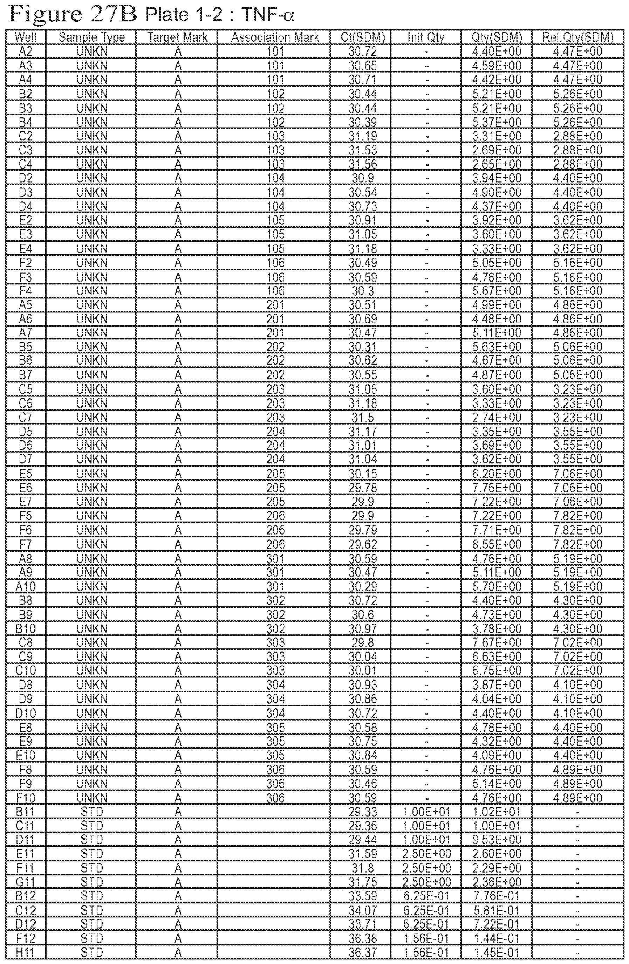

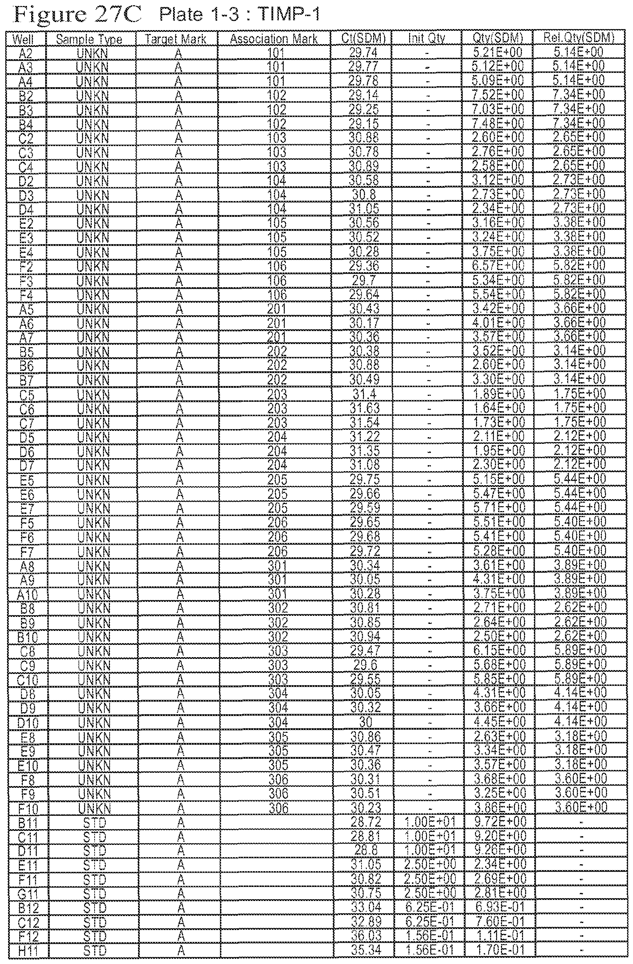

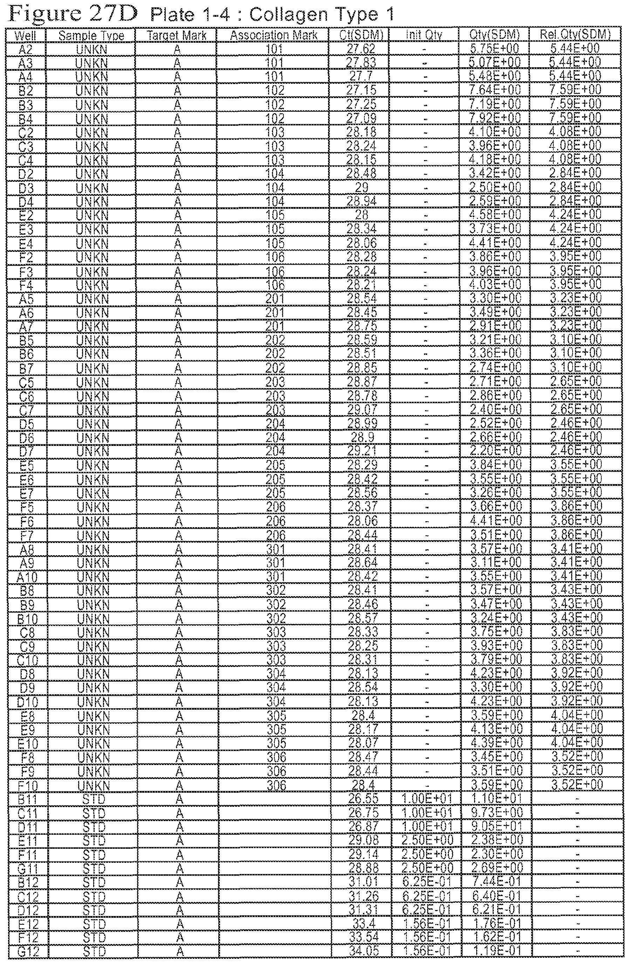

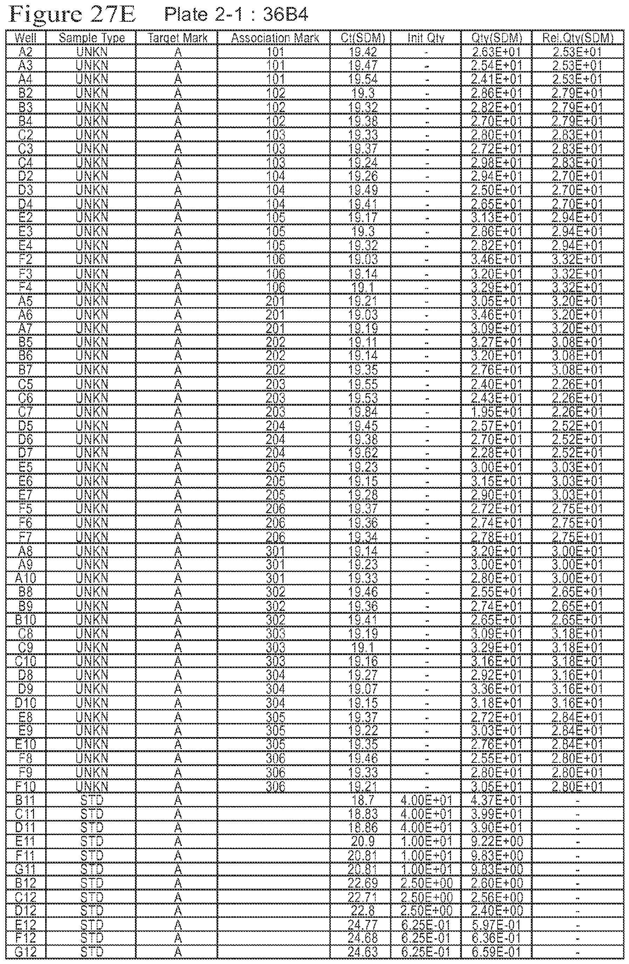

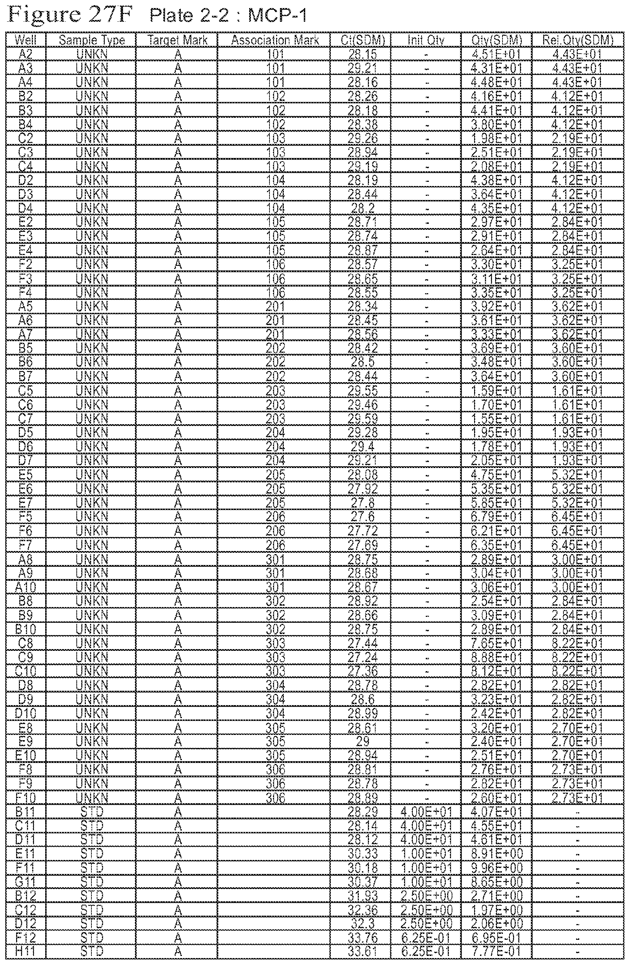

Figure 27A-F shows raw data for quantitative RT-PCR of animals treated with Cenicriviroc (low or high dose) at week 9. Panel A shows the levels of 36B4, Panel B shows the levels of TNF-α, Panel C shows the levels of TIMP-1, Panel D shows the levels of collagen type 1, Panel E shows the levels of 36B4, and Panel f shows the levels of MCP-1.
Figure 28 shows the body weight changes of animals treated with Cenicriviroc (low or high dose) from 6 to 18 weeks.
Figure 29 shows the survival curve of animals treated with Cenicriviroc (low or high dose) from 6 to 18 weeks.
Figure 30A-C shows the body weight and liver weight at of animals treated with Cenicriviroc (low or high dose) at week 18. Panel A shows Body weight, Panel B shows Liver weight, and Panel C shows Liver-to-body weight ratio.
Figure 31A-C shows macroscopic appearance of livers of animals treated with Cenicriviroc (low or high dose) at week 18. Panel A shows the livers of animals treated with vehicle only, Panel B shows the livers of animals treated with low-dose Cenicriviroc, and Panel C shows the livers of animals treated with high-dose Cenicriviroc.
Figure 32 shows the number of visible tumor nodules of animals treated with Cenicriviroc (low or high dose) at week 18.
Figure 33 shows the maximum diameter of visible tumor nodules of animals treated with Cenicriviroc (low or high dose) at week 18.
Figure 34 shows representative photomicrographs of HE-stained liver sections of animals treated with Cenicriviroc (low or high dose) at week 18.
Figure 35 shows representative photomicrographs of GS-immunostained liver sections of animals treated with Cenicriviroc (low or high dose) at week 18.
Figure 36 shows representative photomicrographs of CD31-immunostained liver sections of animals treated with Cenicriviroc (low or high dose) at week 18.
Figure 37 shows percentages of CD31-positive area of animals treated with Cenicriviroc (low or high dose) at week 18.
Figure 38 Proportion of Subjects With HIV-1 RNA <50 Copies/mL Over Time up to Week 48 - Snapshot Algorithm - ITT - Study 202.
Figure 39 shows the LS mean changes from baseline in sCD14 levels (106 pg/mL) over time up to Week 48 - ITT.
Figure 40 shows the CVC (Pooled Data)- and EFV-treated subjects grouped according to APRI and FIB-4 fibrosis index scores at baseline, Week 24, and Week 48.
Figure 41 shows the scatter plot of change from baseline APRI versus change from baseline sCD14 - Week 48 (ITT).
Figure 42 shows a scatter plot of change from baseline FIB-4 versus change from baseline sCD14 - Week 48 (ITT).
Figure 43 shows the study design for studying the combination treatment comprising CVC and an additional therapeutic agent.

### DETAILED DESCRIPTION

It should be understood that singular forms such as "a," "an," and "the" are used throughout this application for convenience, however, except where context or an explicit statement indicates otherwise, the singular forms are intended to include the plural. Further, it should be understood that every journal article, patent, patent application, publication, and the like that is mentioned herein is hereby incorporated by reference in its entirety and for all purposes. All numerical ranges should be understood to include each and every numerical point within the numerical range, and should be interpreted as reciting each and every numerical point individually. The endpoints of all ranges directed to the same component or property are inclusive, and intended to be independently combinable.

### Definitions:

Except for the terms discussed below, all of the terms used in this Application are intended to have the meanings that one of skill in the art at the time of the invention would ascribe to them.

"About" includes all values having substantially the same effect, or providing substantially the same result, as the reference value. Thus, the range encompassed by the term "about" will vary depending on context in which the term is used, for instance the parameter that the reference value is associated with. Thus, depending on context, "about" can mean, for example, ±15%, ±10%, ±5%, ±4%, ±3%, ±2%, ±1%, or ± less than 1%. Importantly, all recitations of a reference value preceded by the term "about" are intended to also be a recitation of the reference value alone. Notwithstanding the preceding, in this application the term "about" has a special meaning with regard to pharmacokinetic parameters, such as area under the curve (including AUC, AUCₜ, and AUC_{∞}) Cₘₐₓ, Tₘₐₓ, and the like. When used in relationship to a value for a pharmacokinetic parameter, the term "about" means from 80% to 125% of the reference parameter.

"Cenicriviroc" refers to the chemical compound (*S*)-8-[4-(2-Butoxyethoxy)phenyl]-1-isobutyl-*N*-(4-{[(1-propyl-1*H*-imidazol-5-yl)methyl]sulfinyl}phenyl)-1,2,3,4-tetrahydro-1-benzazocine-5-carboxamide (structure shown below). Details of the composition of matter of cenicriviroc are disclosed in US Patent Application Publication No. 2012/0232028 which is hereby incorporated by reference in its entirety for all purposes. Details of related formulations are disclosed in US Application No. 61/823,766 which is hereby incorporated by reference in its entirety for all purposes.

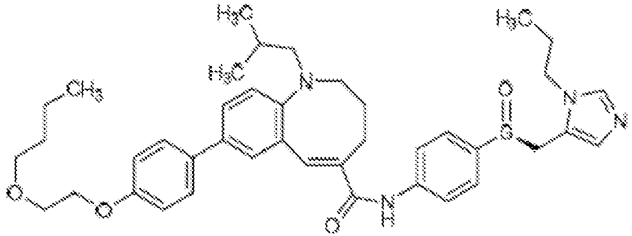

"Compound of the present invention" or "the present compound" refers to cenicriviroc or a salt or solvate thereof.

"Substantially similar" means a composition or formulation that resembles the reference composition or formulation to a great degree in both the identities and amounts of the composition or formulation.

"Pharmaceutically acceptable" refers to a material or method that can be used in medicine or pharmacy, including for veterinary purposes, for example, in administration to a subject.

"Salt" and "pharmaceutically acceptable salt" includes both acid and base addition salts. "Acid addition salt" refers to those salts that retain the biological effectiveness and properties of the free bases, which are not biologically or otherwise undesirable, and which are formed with inorganic acids and organic acids. "Base addition salt" refers to those salts that retain the biological effectiveness and properties of the free acids, which are not biologically or otherwise undesirable, and which are prepared from addition of an inorganic base or an organic base to the free acid. Examples of pharmaceutically acceptable salts include, but are not limited to, mineral or organic acid addition salts of basic residues such as amines; alkali or organic addition salts of acidic residues; and the like, or a combination comprising one or more of the foregoing salts. The pharmaceutically acceptable salts include salts and the quaternary ammonium salts of the active agent. For example, acid salts include those derived from inorganic acids such as hydrochloric, hydrobromic, sulfuric, sulfamic, phosphoric, nitric and the like; other acceptable inorganic salts include metal salts such as sodium salt, potassium salt, cesium salt, and the like; and alkaline earth metal salts, such as calcium salt, magnesium salt, and the like, or a combination comprising one or more of the foregoing salts. Pharmaceutically acceptable organic salts includes salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethane disulfonic, oxalic, isethionic, HOOC-(CH₂)ₙ-COOH where n is 0-4, and the like; organic amine salts such as triethylamine salt, pyridine salt, picoline salt, ethanolamine salt, triethanolamine salt, dicyclohexylamine salt, N,N'-dibenzylethylenediamine salt, and the like; and amino acid salts such as arginate, asparginate, glutamate, and the like; or a combination comprising one or more of the foregoing salts.

In one embodiment, the acid addition salt of cenicriviroc is cenicriviroc mesylate, e.g., (S)-8-[4-(2-Butoxyethoxy)phenyl]-1-isobutyl-*N*-(4-{[(1-propyl-1*H-*imidazol-5-yl)methyl]sulfinyl}phenyl)-1,2,3,4-tetrahydro-1-benzazocine-5-carboxamide monomethanesulfonoate. In one embodiment, the cenicriviroc mesylate is a crystalline material, such as a pale greenish-yellow crystalline powder. In one embodiment, the cenicriviroc mesylate is freely soluble in glacial acetic acid, methanol, benzyl alcohol, dimethylsulfoxide, and N,N-dimethylformamide; soluble in pyridine and acetic anhydride; and sparingly soluble in 99.5% ethanol; slightly soluble in acetonitrile, 1-octanol, and tetrahydrofuran; and practically insoluble in ethyl acetate and diethylether. In one embodiment, the cenicriviroc mesylate is freely soluble in aqueous solution from pH 1 to 2; sparingly soluble at pH 3 and practically insoluble from pH 4 to 13 and in water.

"Solvate" means a complex formed by solvation (the combination of solvent molecules with molecules or ions of the active agent of the present invention), or an aggregate that consists of a solute ion or molecule (the active agent of the present invention) with one or more solvent molecules. In the present invention, the preferred solvate is hydrate.

"Pharmaceutical composition" refers to a formulation of a compound of the disclosure and a medium generally accepted in the art for the delivery of the biologically active compound to mammals, e.g., humans. Such a medium includes all pharmaceutically acceptable carriers, diluents or excipients therefor.

"Treating" includes ameliorating, mitigating, and reducing the instances of a disease or condition, or the symptoms of a disease or condition.

"Administering" includes any mode of administration, such as oral, subcutaneous, sublingual, transmucosal, parenteral, intravenous, intra-arterial, buccal, sublingual, topical, vaginal, rectal, ophthalmic, otic, nasal, inhaled, intramuscular, intraosseous, intrathecal, and transdermal, or a combination thereof. "Administering" can also include prescribing or filling a prescription for a dosage form comprising a particular compound. "Administering" can also include providing directions to carry out a method involving a particular compound or a dosage form comprising the compound.

"Therapeutically effective amount" means the amount of an active substance that, when administered to a subject for treating a disease, disorder, or other undesirable medical condition, is sufficient to have a beneficial effect with respect to that disease, disorder, or condition. The therapeutically effective amount will vary depending on the chemical identity and formulation form of the active substance, the disease or condition and its severity, and the age, weight, and other relevant characteristics of the patient to be treated. Determining the therapeutically effective amount of a given active substance is within the ordinary skill of the art and typically requires no more than routine experimentation.

### Fibrosis:

Fibrosis is the formation of excess fibrous connective tissue in an organ or tissue in a reparative or reactive process. This can be a reactive, benign, or pathological state. The deposition of connective tissue in the organ and/or tissue can obliterate the architecture and function of the underlying organ or tissue. Fibrosis is this pathological state of excess deposition of fibrous tissue, as well as the process of connective tissue deposition in healing.

Fibrosis is similar to the process of scarring, in that both involve stimulated cells laying down connective tissue, including collagen and glycosaminoglycans. Cytokines which mediate many immune and inflammatory reactions play a role in the development of fibrosis. Hepatocyte damage resulting from factors such as fat accumulation, viral agents, excessive alcohol consumption, hepatoxins, inevitably triggers an inflammatory immune response. The increased production of cytokines and chemokines in the liver leads to recruitment of pro-inflammatory monocytes (precursor cells) that subsequently mature into pro-inflammatory macrophages. Pro-inflammatory macrophages are pro-fibrogenic in nature and ultimately lead to the activation of hepatic stellate cells (HSCs) that are primarily responsible for the deposition of extracellular matrix (ECM).

Infiltration of various immune cell populations, resulting in inflammation, is a central pathogenic feature following acute- and chronic liver injury. Chronic liver inflammation leads to continuous hepatocyte injury which can lead to fibrosis, cirrhosis, ESLD, and HCC. Interactions between intra-hepatic immune cells lead to increased activation and migration of Kupffer cells and HSCs and are critical events for developing liver fibrosis. Additionally, there is increasing evidence of the role of CCR2 and CCR5 in the pathogenesis of liver fibrosis [1-7,9, 31]. These members of the C-C chemokine family are expressed by pro-fibrogenic cells including pro-inflammatory monocytes and macrophages, Kupffer cells, and HSCs [1-4]. CCR2 signaling plays an important role in the pathogenesis of renal fibrosis through regulation of bone marrow-derived fibroblasts [8]. CCR2- and CCR5-positive monocytes as well as CCR5-positive T lymphocytes are attracted by locally released MCP-1 and RANTES, and can contribute to chronic interstitial inflammation in the kidney [10, 11]. In rodents, CVC has high distribution in the liver, mesenteric lymph node, and intestine also described as the gut-liver axis. Disruption of the intestinal microbiota and its downstream effects on the gut-liver axis both play an important role in metabolic disorders such as obesity, non-alcoholic fatty liver disease (NAFLD) and non-alcoholic steatohepatitis (NASH) [16, 23].

**Table 1 lists chemokines expressed by liver cells [30].**

| **Cell type** | **Chemokine** |
|---|---|
| Hepatocytes | MCP-1 (CCL2) _{[38]}, MIP-1α (CCL3) _{[74]}, RANTES (CCL5) _{[16,74]}, MIP-3β (CCL19) _{[75]}, SLC (CCL21) _{[75]}, Mig (CXCL9) _{[64]}, IP-10 (CXCL10) _{[64]}, CXCL16 _{[76]}, LEC (CCL16) _{[77]}, IL-8 (CXCL8) _{[78]} and Eotaxin (CCL11) _{[41]} |
| Stellate cells | MCP-1 (CCL2) _{[52,60]}, MIP-1α (CCL3) _{[60]}, MIP-1β (CCL4) _{[60]}, CX₃CL1 _{[59]}, KC (CXCL1) _{[60]}, MIP-2 (CXCL2) _{[60]}, IP-10 (CXCL10) _{[60]} and SLC (CCL21) _{[70]} |
| Kupffer cells | MCP1 (CCL2) _{[52,38,60,79]}, MIP-1α (CCL3) _{[80]} and MIP-3α (CCL20) _{[56]} |
| Liver endothelial cells | MCP-1 (CCL2) _{[52]}, IL-8 (CXCL8) _{[81,76]}, CXCL16 _{[75]}, Mig (CXCL9) _{[69]}, IP-10 (CXCL10) _{[69]}, CXCL16 _{[65]}, CX₃CL1 _{[82]}, SLC (CCL21) _{[83]}, Eotaxin (CCL11) _{[41]} and TECK (CCL25) _{[73]} |
| * Summarizes selected experimental data from humans and mice/rats regarding the expression of chemokines by different resident hepatic cell populations upon activation or following liver injury. IP: Interferon-inducible protein; KC: Kupffer cell; LEC: Liver-expressed chemokine; MCP: Monocyte chemoattractant protein; MIP: Macrophage inflammatory protein; SLC: Secondary lymphoid-organ chemokine; TECK: Thymus-expressed chemokine | |

The activation of Hepatic stellate cells (HSCs) plays an important role in the pathogenesis of hepatic fibrosis. Following liver injury, hepatic stellate cells (HSCs) become activated and express a combination of matrix metalloproteinases (MMPs) and their specific tissue inhibitors (TIMPs) [32]. In the early phases of liver injury, HSCs transiently express MMP-3, MMP-13, and uroplasminogen activator (uPA) and exhibit a matrix-degrading phenotype. Degradation of the extracellular matrix does not appear to be CCR2 or CCR5 dependent.

Activated HSCs can amplify the inflammatory response by inducing infiltration of mono- and polymorphonuclear leucocytes. Infiltrating monocytes and macrophages participate in the development of fibrosis via several mechanisms, including increased secretion of cytokines and generation of oxidative stress-related products. Activated HSCs can express CCR2 and CCR5 and produce chemokines that include MCP-1, MIP-1α, MIP-1β and RANTES. CCR2 promotes HSC chemotaxis and the development of hepatic fibrosis. In human liver diseases, increased MCP-1 is associated with macrophage recruitment and severity of hepatic fibrosis and primary biliary cirrhosis. CCR5 stimulates HSC migration and proliferation.

In the later stages of liver injury and HSC activation, the pattern changes and the cells express a combination of MMPs that have the ability to degrade normal liver matrix, while inhibiting degradation of the fibrillar collagens that accumulate in liver fibrosis. This pattern is characterized by the combination of pro-MMP-2 and membrane type 1 (MT1)-MMP expression, which drive pericellular generation of active MMP-2 and local degradation of normal liver matrix. In addition there is a marked increase in expression of TIMP-1 leading to a more global inhibition of degradation of fibrillar liver collagens by interstitial collagenases (MMP-1/MMP-13). In liver injury associated with chronic alcoholic liver disease, the production of TNF-α, IL-1, IL-6, as well as the chemokine IL-8/CXCL8 is increased. TNF-α is also an important mediator of non-alcoholic fatty liver disease. These pathways play a significant role in the progression of liver fibrosis. Inhibiting the activation of HSCs and accelerating the clearance of activated HSCs may be effective strategies for resolution of hepatic fibrosis.

Chemokine families play important regulatory roles in inflammation. Members of this family include, but are not limited to CXC receptors and ligands including but not limited to CXCR1, CXCR2, CXCR3, CXCR4, CXCR5, CXCR6, CXCR7, CXCR8, CXCR9, CXCR10, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, and CXCL17; the CC chemokines and receptors including but not limited to CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCR1, CCR2, CCR3, CCR4, CCR5, CCR6, CCR7, CCR8, CCR, and CCR10; the C chemokines including but not limited to XCL1, XCL2, and XCR1; and the CX3C chemokines including but not limited to CS3CL1 and CX3CR1. These molecules may be upregulated in fibrotic organs or tissues. In further embodiments, these molecules may be downregulated in fibrotic organs or tissues. In further embodiments, the molecules in the signaling pathways of these chemokines may be upregulated in fibrotic organs or tissues. In further embodiments, the molecules in the signaling pathways of these chemokines may be downregulated in fibrotic organs or tissues.

Fibrosis can occur in many tissues within the body including but not limited to, the lungs, liver, bone marrow, joints, skin, digestive tract, lymph nodes, blood vessels, or heart and typically is a result of inflammation or damage. Non-limiting examples of fibrosis, or a fibrotic disease and/or condition, include Pulmonary fibrosis, Idiopathic pulmonary fibrosis, Cystic fibrosis, Cirrhosis, Endomyocardial fibrosis, myocardial infarction, Atrial Fibrosis, Mediastinal fibrosis, Myelofibrosis, Retroperitoneal fibrosis, Progressive massive fibrosis, complications from pneumoconiosis, Nephrogenic systemic fibrosis, Crohn's Disease, Keloid, Scleroderma/systemic sclerosis, Arthrofibrosis, Peyronie's disease, Dupuytren's contracture, fibrosis associated with atherosclerosis, lymph node fibrosis, emerging cirrhosis, non-cirrhotic hepatic fiborsis, renal fibrosis, and adhesive capsulitis.

### Embodiments of Therapeutic Utilities:

The present invention provides a combination therapy for treating fibrosis and/or fibrotic diseases and/or conditions. Anti-fibrotic effects of CVC in animal studies were observed when CVC treatment was initiated at the onset of liver injury (TAA) or soon after (TAA; HFD) but not once cirrhosis was established (TAA). This suggests that anti-fibrotic effects of CVC may be more pronounced in populations with established liver fibrosis and at significant risk of disease progression. These include: Non-alcoholic steatohepatitis (NASH) associated with type 2 diabetes mellitus (T2DM) and metabolic syndrome (MS), HIV and HCV co-infection, or HCV infection, alcoholic liver disease, viral hepatitis (such as HBV or HCV infection), emerging cirrhosis, non-cirrhotic hepatic fibrosis, and a combination thereof.

### NASH

The combination therapy disclosed herein may be used to treat liver fibrosis resulting from Nonalcoholic Steatohepatitis (NASH), a common liver disease that affects 2 to 5 percent of Americans. Although liver damage due to NASH has some of the characteristics of alcoholic liver disease, it occurs in people who drink little or no alcohol. The major feature in NASH is fat in the liver, along with inflammation and hepatocyte damage (ballooning). NASH can be severe and can lead to cirrhosis, in which the liver is permanently damaged and scarred and no longer able to work properly.Nonalcoholic fatty liver disease (NAFLD) is a common, often "silent", liver disease associated with obesity related disorders, such as type-2 diabetes and metabolic syndrome, occurring in people who drink little or no alcohol and is characterized by the accumulation of fat in the liver with no other apparent causes. [32-43] At the beginning of the NAFLD spectrum is simple steatosis, which is characterized by a build-up of fat within the liver. Liver steatosis without inflammation is usually benign and slow or non-progressive. NASH is a more advanced and severe subtype of NAFLD where steatosis is complicated by liver-cell injury and inflammation, with or without fibrosis.

The rising prevalence of obesity-related disorders has contributed to a rapid increase in the prevalence of NASH. Approximately 10% to 20% of subjects with NAFLD will progress to NASH [44].

NAFLD is the most common cause of chronic liver disease. [45] Most US studies report a 10% to 35% prevalence rate of NAFLD; however, these rates vary with the study population and the method of diagnosis. [46] Since approximately one-third of the US population is considered obese, the prevalence of NAFLD in the US population is likely to be about 30%.[46] One study has found that NAFLD affects approximately 27% to 34% of Americans, or an estimated 86 to 108 million patients. [44] NAFLD is not unique to the US. Reports from the rest of the world, including Brazil, China, India, Israel, Italy, Japan, Korea, Sri Lanka, and Taiwan, suggest that the prevalence rate ranges from 6% to 35% (median of 20%). [46] A study by the Gastroenterological Society of Australia/Australian Liver Association has found that NAFLD affects an estimated 5.5 million Australians, including 40% of all adults aged ≥ 50 years. [47] An Australian study of severely obese patients found that 25% of these patients had NASH. [48]

Liver biopsy is required to make a definitive diagnosis of NASH. In a US study of middle-aged individuals, the prevalence of histologically confirmed NASH was 12.2%.[49] Current estimates place NASH prevalence at approximately 9 to 15 million in the US (3% to 5% of the US population), with similar prevalence in the EU and China.[46, 50] The prevalence of NASH in the obese population ranges from 10% to 56% (median of 33%). [46] In an autopsy series of lean individuals from Canada, the prevalence of steatohepatitis and fibrosis was 3% and 7%, respectively.[46] The prevalence of NASH is also increasing in developing regions, which has been attributed to people in these regions starting to adopt a more sedentary lifestyle and westernized diet [51] consisting of processed food with high fat and sugar/fructose content.[52]

NASH is a serious chronic liver disease defined by the presence of hepatic steatosis and inflammation with hepatocyte injury, with or without fibrosis. [34] Chronic liver inflammation is a precursor to fibrosis, which can progress to cirrhosis, end-stage liver disease and hepatocellular carcinoma. In addition to insulin resistance, altered lipid storage and metabolism, accumulation of cholesterol within the liver, oxidative stress resulting in increased hepatic injury, and bacterial translocation[34,53-56] secondary to disruption of gut microbiota (associated with high fructose-containing diet) have all been implicated as important co-factors contributing to progression of NASH.[57-60] Due to the growing epidemic of obesity and diabetes, NASH is projected to become the most common cause of advanced liver disease and the most common indication for liver transplantation.[46, 61-63] The burden of NASH, combined with a lack of any approved therapeutic interventions, represents an unmet medical need.

In further embodiments, liver fibrosis is associated with emerging cirrhosis. In some embodiments, the cirrhosis is associated with alcohol damage. In further embodiments, the cirrhosis is associated with a hepatitis infection, including but not limited to hepatitis B and hepatitis C infections, primary biliary cirrhosis (PBC), primary sclerosing cholangitis, HIV infection, or fatty liver disease. In some embodiments, the present invention provides for methods of treating subjects at risk of developing liver fibrosis or cirrhosis.

In another embodiment, the fibrosis comprises non-cirrhotic hepatic fibrosis. In another further embodiment, the subject is infected by human immunodeficiency virus (HIV). In yet a further embodiment, the subject is infected with a hepatitis virus, including but not limited to HCV (hepatitis C virus). In further embodiment, the subject has diabetes. In a further embodiment, the subject has type 2 diabetes. In a further embodiment, the subject has type 1 diabetes. In a further embodiment, the subject has metabolic syndrome (MS). In a further embodiment, the subject has alcoholic liver disease. In a futher embodiment, the subject has viral hepatitis. In one embodiment, the viral hepatitis is caused by HBV infection. In another embodiment, the viral hepatitis is caused by HCV infection. In further embodiments, the subject has one or more of these diseases or disorders. In a further embodiment, the subject is at risk of developing one or more of these diseases. In a further embodiment, the subject has insulin resistance. In further embodiments, the subject has increased blood glucose concentrations, high blood pressure, elevated cholesterol levels, elevated triglyceride levels, or is obese. In a further embodiment, the subject has Polycystic ovary syndrome.

In one embodiment, the invention provides a method of treatment, wherein the cenicriviroc or a salt or solvate thereof is coadministered with one or more additional active agents. In a further embodiment, the additional active agent is an anti-inflammatory agent. In a further embodiment, the additional active agent is a chemokine receptor antagonist. In a further embodiment, the additional active agent inhibits the binding of a chemokine to a chemokine receptor. In a further embodiment, the additional active agent inhibits the binding of ligand to CCR1. In a further embodiment, the additional active agent inhibits the binding of CCR5 ligands to CCR1. In a further embodiment, the one or more additional therapeutic agents can suppress hepatic apolipoprotein CIII expression, suppress cholesterol 7 alpha-hydroxylase (CYP7A1) expression, induce high-density lipoprotein-mediated transhepatic cholesterol efflux , protect against cholestatic liver damage, attenuate liver inflammation and/or fibrosis, decrease hepatic lipid accumulation, and/or inhibit proinflammatory and/or profibrotic gene expression. In one embodiment, the one or more additional therapeutic agents are selected from the group including, but not limited to, a farnesoid X receptor (FXR) agonist, a peroxisome proliferator-activated receptor alpha (PPAR-α) agonist, PPAR-γ agonist, PPAR-δ agonist, high dose vitamin E (> 400 iU/d), obeticholic acid, 3-[2-[2-Chloro-4-[[3-(2,6-dichlorophenyl)-5-(1-methylethyl)-4-isoxazolyl]methoxy]phenyl]ethenyl]benzoic acid (GW4064), 2-methyl-2-[[4-[2-[[(cyclohexylamino)carbonyl](4-cyclohexylbutyl)amino]ethyl]phenyl]thio]-propanoic acid (GW7647), and 2-[2,6 dimethyl-4-[3-[4-(methylthio)phenyl]-3-oxo-1(E)-propenyl]phenoxyl]-2-methylpropanoic acid (GFT505), 3-(3,4-Difluorobenzoyl)-1,2,3,6-tetrahydro-1,1-dimethylazepino[4,5-b]indole-5-carboxylic acid 1-methylethyl ester (WAY-36245), Bile Acid Derivatives (e.g. INT-767, INT-777), Azepino[4,5-b]indoles, 1-[(4-Chlorophenyl)methyl]-3-[(1,1-dimethylethyl)thio]-α,α-dimethyl-5-(1-methylethyl)-1H-Indole-2-propanoic acid (MK886), N-((2S)-2-((((1Z)-1-Methyl-3-oxo-3-(4-(trifluoromethyl)phenyl)prop-1-enyl)amino)-3-(4-(2-(5-methyl-2-phenyl-1,3-oxazol-4-yl)ethoxy)phenyl)propyl)propanamide (GW6471), 2-[2,6 dimethyl-4-[3-[4-(methylthio)phenyl]-3-oxo-1(E)-propenyl]phenoxyl]-2-methylpropanoic acid (GFT505), or a combination thereof.

Certain embodiments include methods for monitoring and/or predicting the treatment efficacy of the present treatment as described herein. Such methods include detecting the level of one or more biological molecules, such as for example, biomarkers, in a subject (or in a biological sample from the subject) treated for fibrosis or a fibrotic disease or condition, wherein an increase or decrease in the level of one or more biological molecules compared to a predetermined standard level indicates or is predictive of the treatment efficacy of the present treatment.

In one embodiment, the invention provides a method of treatment, comprising detecting the level of one or more biological molecules in the subject treated for fibrosis or the fibrotic disease or condition, and determining a treatment regimen based on an increase or decrease in the level of one or more biological molecules, wherein the biological molecule is selected from the group consisting of lipopolysaccharide (LPS), LPS-binding protein (LBP), 16S rDNA, sCD14, intestinal fatty acid binding protein (I-FABP), zonulin-1, Collagen 1a1 and 3a1, TGF-β, fibronectin-1, hs-CRP, IL-1β, IL-6, IL-33, fibrinogen, MCP-1, MIP-1α and -1β, RANTES, sCD163, TGF-β, TNF-α, a biomarker of hepatocyte apoptosis such as CK-18 (caspase-cleaved and total), or biomarkers of bacterial translocation such as LPS, LBP, sCD14, and I-FABP, α2-macroglobulin, apolipoprotein A1, haptoglobin, hyaluronic acid, hydroxyproline, N-terminal propeptide of collagen type III, tissue inhibitors of metalloproteinases, or a combination thereof.

In one embodiment, the invention provides a method of treatment, comprising detecting the level of one or biological molecules in the subject treated for fibrosis or the fibrotic disease or condition, wherein an increase or decrease in the level of one or more biological molecules compared to a predetermined standard level is predictive of the treatment efficacy of fibrosis or the fibrotic disease or condition.

In a further embodiment, the one or more biological molecules are measured in a biological sample from a subject treated for fibrosis or the fibrotic disease or condition. In yet a further embodiment, the biological sample is selected from blood, skin, hair follicles, saliva, oral mucous, vaginal mucous, sweat, tears, epithelial tissues, urine, semen, seminal fluid, seminal plasma, prostatic fluid, pre-ejaculatory fluid (Cowper's fluid), excreta, biopsy, ascites, cerebrospinal fluid, lymph, brain, and tissue extract sample or biopsy sample.

### CDAA Mouse Model of NASH

The choline-deficient, L-amino acid defined (CDAA) diet has been used as a rodent model of NASH, and is characterized by steatosis, inflammatory cell infiltration and fibrosis (Nakae et al. (1995) Toxic. Pathol. 23(5):583-590). NASH can result by inhibition of the fatty acid oxidation in hepatocytes. Mice on the CDAA diet do not gain weight or have changes in peripheral insulin sensitivity (Kodama et al (2009) Gastroenterology 137(4):1467-1477). The CDAA model, like the MCD model, can be used to study the inflammatory and fibrotic elements of the NASH spectrum.

### Combination Therapy:

The compound of the invention may be used alone or in combination with one or more additional active agents. The one or more additional active agents may be any compound, molecule, or substance which can exert therapeutic effect to a subject in need thereof. The one or more additional active agents may be "co-administered", i.e, administered together in a coordinated fashion to a subject, either as separate pharmaceutical compositions or admixed in a single pharmaceutical composition. By "co-administered", the one or more additional active agents may also be administered simultaneously with the present compound, or be administered separately with the present compound, including at different times and with different frequencies. The one or more additional active agents may be administered by any known route, such as orally, intravenously, subcutaneously, intramuscularly, nasally, and the like; and the therapeutic agent may also be administered by any conventional route. In many embodiments, at least one and optionally both of the one or more additional active agents may be administered orally.

These one or more additional active agents include, but are not limited to, agents that suppress hepatic apolipoprotein CIII expression, suppress cholesterol 7 alpha-hydroxylase (CYP7A1) expression, induce high-density lipoprotein-mediated transhepatic cholesterol efflux , protect against cholestatic liver damage, attenuate liver inflammation and/or fibrosis, decrease hepatic lipid accumulation, inhibit proinflammatory and/or profibrotic gene expression, a farnesoid X receptor (FXR) agonist, a peroxisome proliferator-activated receptor alpha (PPAR-α and delta) agonist, and/or peroxisome proliferator-activated receptor gamma (PPAR-γ) agonist, anti-inflammatory agents, chemokine receptor antagonists, or combination thereof. When two or more medicines are used in combination, dosage of each medicine is commonly identical to the dosage of the medicine when used independently, but when a medicine interferes with metabolism of other medicines, the dosage of each medicine is properly adjusted. Each medicine may be administered simultaneously or separately in a time interval of less than 12 hours. A dosage form as described herein, such as a capsule, can be administered at appropriate intervals. For example, once per day, twice per day, three times per day, and the like. In particular, the dosage form is administered once or twice per day. Even more particularly, the dosage form is administered once per day. Also, more particularly, the dosage form is administered twice per day.

In one embodiment, the one or more additional therapeutic agents include, but are not limited to, a farnesoid X receptor (FXR) agonist, high dose vitamin E (> 400 iU/d), a peroxisome proliferator-activated receptor alpha (PPAR-α) agonist, PPAR-γ agonist, and PPAR-δ agonist, obeticholic acid, pioglitazone, 3-[2-[2-Chloro-4-[[3-(2,6-dichlorophenyl)-5-(1-methylethyl)-4-isoxazolyl]methoxy]phenyl]ethenyl]benzoic acid (GW4064), 2-methyl-2-[[4-[2-[[(cyclohexylamino)carbonyl](4-cyclohexylbutyl)amino]ethyl]phenyl]thio]-propanoic acid (GW7647), and 2-[2,6 dimethyl-4-[3-[4-(methylthio)phenyl]-3-oxo-1(E)-propenyl]phenoxyl]-2-methylpropanoic acid (GFT505), 3-(3,4-Difluorobenzoyl)-1,2,3,6-tetrahydro-1,1-dimethylazepino[4,5-b]indole-5-carboxylic acid 1-methylethyl ester (WAY-36245), Bile Acid Derivatives (e.g. INT-767, INT-777), Azepino[4,5-b]indoles, 1-[(4-Chlorophenyl)methyl]-3-[(1,1-dimethylethyl)thio]-α,α-dimethyl-5-(1-methylethyl)-1H-Indole-2-propanoic acid (MK886), N-((2S)-2-(((1Z)-1-Methyl-3-oxo-3-(4-(trifluoromethyl)phenyl)prop-1-enyl)amino)-3-(4-(2-(5-methyl-2-phenyl-1,3-oxazol-4-yl)ethoxy)phenyl)propyl)propanamide (GW6471), 2-[2,6 dimethyl-4-[3-[4-(methylthio)phenyl]-3-oxo-1(E)-propenyl]phenoxyl]-2-methylpropanoic acid (GFT505), or a combination thereof.

In one embodiment the additional therapeutic agents include an anti-inflammatory agent. In a further embodiment, the additional active agent is a chemokine receptor antagonist. In a further embodiment, the additional active agent inhibits the binding of a chemokine ligand to a chemokine receptor. In a further embodiment, the additional active agent inhibits the binding of ligand to CCR1. In a further embodiment, the additional active agent inhibits the binding of CCR5 ligands to CCR1. In one embodiment, chemokine ligands include, but are not limited to MCP-1 (CCL2), MIP-1α (CCL3), RANTES (CCL5), MIP-3β (CCL19), SLC (CCL21), Mig (CXCL9), IP-10 (CXCL10), CSCL16, LEC (CCL16), IL-8 (CXCL8), Eotaxin (CCL11), MIP-1β (CCL4), CX₃CL1, KC (CXCL1), MIP-2 (CXCL2), MIP-3α (CCL20), CXCL16, TECK (CCL25), CCL6, CCL7, CCL8, CCL9, CCL10, CCL12, CCL13, CCL14, CCL15, CCL17, and CCL18 or combinations thereof. In one embodiment, chemokine receptors include, but are not limited to CCR1, CCR2, CCR3, CCR4, CCR5, CCR6, CCR7, CCR8, CCR9, and CCR10; the C chemokines including but not limited to XCL1, XCL2, and XCR1; and the CX3C chemokines including but not limited to CS3CL1 and CX3CR1 or combinations thereof. In one exemplary embodiment, the additional therapeutic agent inhibits binding of CCR5 ligands (e.g. MIP-1α, RANTES) to CCR1. In one embodiment, the additional therapeutic agent is Aplaviroc, Vicriviroc, Maraviroc, a chemokine peptide derivative, a small molecule inhibitor, an antibody, Met-RANTES, AOP-RANTES, RANTES(3-68), Eotaxin(3-74), Met-Ckbeta7, I-Tac/E0H1, CPWYFWPC-Peptide, a small molecule derived from compound J113863, a small molecule trans-isomer of compound J113863, SB-328437 (Glaxo-SmithKline), RO116-9132-238 (Roche Bioscience), Compound 25 (Merck), A-122058 (Abbott Laboratories), DPCA37818, DPC168, Compound 115 (Bristol-Myers Squibb), Piperidine antagonist, CP-481,715 (Pfizer), MLN3897 (Millennium/Sanofi Aventis), BX471 (structure shown below, Berlex/Scherring AG), AZD-4818 (Astra-Zeneca), BMS-817399 (Bristol-Myers Squibb), CAM-3001 (Medimmune), CCX354-C (Chemo-Centryx), CCx915/MK-0812, INCB8696 (InCyte), RO5234444, GW766994, JC1, BKT140, propagermanium, Shikonin, BX471, and/or YM-344031.

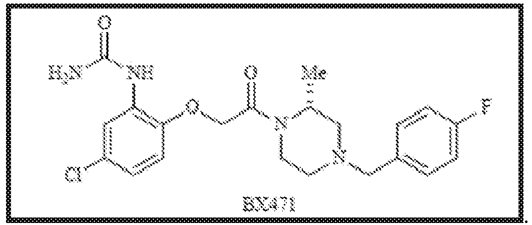

### Method of Co-administration

In one aspect, the present invention provides a method of treating fibrosis or fibrotic disease or condition in a patient in need thereof. The method comprises co-administering to a patient in need thereof a therapeutically effective amount of at least one additional therapeutic agent disclsoed above; and at least one compound of CVC as described above, or a salt, solvate, ester and/or prodrug thereof. The term "patient" or "subject" includes humans and animals, preferably mammals.

In one embodiment, the method further comprises co-administering an additional therapeutic agent. That is, the method comprising co-administering to a patient in need thereof a therapeutically effective amount of at least one additional therapeutic agent; and at least one CVC as described above, or a salt, solvate, ester and/or prodrug thereof. The additional active agent can be any compound, agent, molecule, composition, or medication that has biological activity or therapeutic effect. The additional active agent may (1) agonize farnesoid X receptor (FXR); (2) agonize peroxisome proliferator-activated receptor alpha (PPAR-α); (3) suppress hepatic apolipoprotein CIII expression; (4) suppress cholesterol 7 alpha-hydroxylase (CYP7A1) expression; (5) induce high-density lipoprotein-mediated transhepatic cholesterol efflux; (6) protect against cholestatic liver damage; (7) attenuate liver inflammation and/or fibrosis; (8) decrease hepatic lipid accumulation; and/or (9) inhibit proinflammatory and/or profibrotic gene expression, (10) act as an anti-inflammatory agent, and/or (11) inhibit chemokine binding. Preferably, the additional active agent is a farnesoid X receptor (FXR) or peroxisome proliferator-activated receptor alpha (PPAR-α) agonist, or chemokine antagonist. As used herein, ther term "farnesoid X receptor (FXR)" agonist is a drug or molecule that activates the farnesoid X receptor (FXR). As used herein, the term "peroxisome proliferator-activated receptor alpha (PPAR-α)" is a drug or moleucle that activates the peroxisome proliferator-activated receptor alpha (PPAR-α). As used herein, the term "chemokine antagonist" is a drug or molecule that inhibits, decreases, abrogates, or blocks binding of a chemokine to one or more of its cognate receptors. Examples of FXR and PPAR-α agonists include, but are not limited to, obeticholic acid, 3-[2-[2-Chloro-4-[[3-(2,6-dichlorophenyl)-5-(1-methylethyl)-4-isoxazolyl]methoxy]phenyl]ethenyl]benzoic acid (GW4064), 2-methyl-2-[[4-[2-[[(cyclohexylamino)carbonyl](4-cyclohexylbutyl)amino]ethyl]phenyl]thio]-propanoic acid (GW7647), and 2-[2,6 dimethyl-4-[3-[4-(methylthio)phenyl]-3-oxo-1(E)-propenyl]phenoxyl]-2-methylpropanoic acid (GFT505), 3-(3,4-Difluorobenzoyl)-1,2,3,6-tetrahydro-1,1-dimethylazepino[4,5-b]indole-5-carboxylic acid 1-methylethyl ester (WAY-36245), Bile Acid Derivatives (e.g. INT-767, INT-777), Azepino[4,5-b]indoles, 1-[(4-Chlorophenyl)methyl]-3-[(1,1-dimethylethyl)thio]-α,α-dimethyl-5-(1-methylethyl)-1H-Indole-2-propanoic acid (MK886), N-((2S)-2-(((1Z)-1-Methyl-3-oxo-3-(4-(trifluoromethyl)phenyl)prop-1-enyl)amino)-3-(4-(2-(5-methyl-2-phenyl-1,3-oxazol-4-yl)ethoxy)phenyl)propyl)propanamide (GW6471), 2-[2,6 dimethyl-4-[3-[4-(methylthio)phenyl]-3-oxo-1(E)-propenyl]phenoxyl]-2-methylpropanoic acid (GFT505), or a combination thereof. Examples of chemokine antagonists include but are not limited to, Aplaviroc, Vicriviroc, Maraviroc, Met-RANTES, AOP-RANTES, RANTES(3-68), Eotaxin(3-74), Met-Ckbeta7, I-Tac/EOH1, CPWYFWPC-Peptide, a small molecule derived from compound J113863, Small molecule trans-isomer of compound J113863, SB-328437 (Glaxo-SmithKline), RO116-9132-238 (Roche Bioscience), Compound 25 (Merck), A-122058 (Abbott Laboratories), DPCA37818, DPC168, Compound 115 (Bristol-Myers Squibb), Piperidine antagonist, CP-481,715 (Pfizer), MLN3897 (Millennium/Sanofi Aventis), BX471 (Berlex/Scherring AG), AZD-4818 (Astra-Zeneca), BMS-817399 (Bristol-Myers Squibb), CAM-3001 (Medimmune), CCX354-C (Chemo-Centryx), CCx915/MK-0812, INCB8696 (InCyte), RO5234444, GW766994, JC1, BKT140, propagermanium, Shikonin, BX471, and/or YM-344031.

The term "therapeutically effective amount", as used herein, denotes an amount that can produce one or more intended biological effects in a patient, such as ameliorating, relieving, improving, or remedying the conditions, symptoms, and/or effects of the fibrosis and/or fibrotic disease or condition in a subject. The amount refers to the amount, given in combination, of (a) an additional therapeutic agent and (b) CVC , or a salt, solvate, ester and/or prodrug thereof. The term "therapeutically effective amount" may also refer to the amount, given in combination, of (a) an additional therapeutic, and (b) CVC, or a salt, solvate, ester and/or prodrug thereof. It is understood to one skilled in the art that the therapeutically effective amount may vary for each patient depending on the individual patient's condition.

In one embodiment, CVC, or a salt, solvate, ester and/or prodrug thereof, is administered at a dose from about 30 mg/day to about 500 mg/day. In one embodiment, the additional therapeutic agent is administered at a dose from about 5 mg/m2 to about 3 g/m2.

The administered dose may be expressed in units of mg/m2/day in which a patient's body surface area (BSA) may be calculated in m2 using various available formulae using the patient's height and weight. The administered dose may alternatively be expressed in units of mg/day which does not take into consideration the patient's BSA. It is straightforward to convert from one unit to another given a patient's height and weight.

The term "co-administration" or "coadministration" refers to administration of (a) an additional therapeutic agent and (b) CVC, or a salt, solvate, ester and/or prodrug thereof, together in a coordinated fashion. For example, the co-administration can be simultaneous administration, sequential administration, overlapping administration, interval administration, continuous administration, or a combination thereof.

In one embodiment, the co-administration is carried out for one or more treatment cycles. By "treatment cycle", it is meant a pre-determined period of time for co-administering the additional therapeutic agent and CVC, or a salt, solvate, ester and/or prodrug thereof. Typically, the patient is examined at the end of each treatment cycle to evaluate the effect of the present combination therapy. In one embodiment, the co-administration is carried out for 1 to 48 treatment cycles. In another embodiment, the co-administration is carried out for 1 to 36 treatment cycles. In another embodiment, the co-administration is carried out for 1 to 24 treatment cycles.

In one embodiment, each of the treatment cycle has about 3 or more days. In another embodiment, each of the treatment cycle has from about 3 days to about 60 days. In another embodiment, each of the treatment cycle has from about 5 days to about 50 days. In another embodiment, each of the treatment cycle has from about 7 days to about 28 days. In another embodiment, each of the treatment cycle has 28 days. In one embodiment, the treatment cycle has about 29 days. In another embodiment, the treatment cycle has about 30 days. In another embodiment, the treatment cycle has about 31 days. In another embodiment, the treatment cycle has about a month-long treatment cycle. In another embodiment, the treatment cycle is any length of time from 3 weeks to 6 weeks. In another embodiment, the treatment cycle is any length of time from 4 weeks to 6 weeks. In yet another embodiment, the treatment cycle is 4 weeks. In another embodiment, the treatment cycle is one month. In another embodiment, the treatment cycle is 5 weeks. In another embodiment, the treatment cycle is 6 weeks.

Depending on the patient's condition and the intended therapeutic effect, the dosing frequency for each of the additional therapeutic agent, and CVC, or a salt, solvate, ester and/or prodrug thereof, may vary from once per day to six times per day. That is, the dosing frequency may be once per day, twice per day, three times per day, four times per day, five times per day, or six times per day.

There may be one or more void days in a treatment cycle. By "void day", it is meant a day when neither the additional therapeutic agent nor CVC, or a salt, solvate, ester and/or prodrug thereof, is administered. In other words, none of the additional therapeutic agent and the CVC, or a salt, solvate, ester and/or prodrug thereof, is administered on a void day. Any treatment cycle must have at least one non-void day. By "non-void day", it is meant a day when at least one of the additional therapeutic agent and the CVC, or a salt, solvate, ester and/or prodrug thereof, is administered.

By "simultaneous administration", it is meant that the additional therapeutic agent and the CVC, or a salt, solvate, ester and/or prodrug thereof, are administered on the same day. For the simultaneous administration, the additional therapeutic agent and the CVC, or a salt, solvate, ester and/or prodrug thereof, can be administered at the same time or one at a time.

In one embodiment of the simultaneous administration, the CVC, or a salt, solvate, ester and/or prodrug thereof, is administered from 1 to 4 times per day for 7 to 28 days; and the additional therapeutic agent is administered 1 to 4 times per day for 7 to 28 days. In another embodiment of the simultaneous administration, the CVC, or a salt, solvate, ester and/or prodrug thereof, is administered once per day for 28 days; and the additional therapeutic agent is administered once per day for 28 days.

By "sequential administration", it is meant that during a period of two or more days of continuous co-administration without any void day, only one of the additional therapeutic agent and the CVC, or a salt, solvate, ester and/or prodrug thereof, is administered on any given day.

In one embodiment of the sequential administration, the CVC, or a salt, solvate, ester and/or prodrug thereof, is administered from 1 to 4 times per day for 7 to 21 days; and the additional therapeutic agent is administered 1 to 4 times per day for 7 to 21 days. In another embodiment of the sequential administration, the CVC, or a salt, solvate, ester and/or prodrug thereof, is administered from 1 to 4 times per day for 14 days; and the additional therapeutic agent is administered 1 to 4 times per day for 14 days.

In one specific embodiment of the sequential administration, the present method comprises one or more treatment cycle and each of the treatment cycle has 28 days, wherein the additional therapeutic agent is administered once per day for 7 days, and the CVC, or a salt, solvate, ester and/or prodrug thereof, is administered once per day for 21 days. The 7 days for administering the additional therapeutic agent and the 21 days for administering the CVC, or a salt, solvate, ester and/or prodrug thereof, are independently consecutive or non-consecutive. For example, in a consecutive administration, the 7 days for administering the additional therapeutic agent can be day 1 to day 7 in the treatment cycle, and the 21 days for administering the CVC, or a salt, solvate, ester and/or prodrug thereof, can be day 8 to day 28 in the treatment cycle.

By "overlapping administration", it is meant that during a period of two or more days of continuous co-administration without any void day, there is at least one day of simultaneous administration and at least one day when only one of the additional therapeutic agent and the CVC, or a salt, solvate, ester and/or prodrug thereof, is administered.

In one embodiment of overlapping administration, the CVC, or a salt, solvate, ester and/or prodrug thereof, is administered from 1 to 4 times per day for 28 days; and the additional therapeutic agent is administered 1 to 4 times per day for 7 to 14 days.

In one specific embodiment of overlapping administration, the present method comprises one or more treatment cycle and each of the treatment cycle has 28 days, wherein the additional therapeutic agent is administered once per day for 7 days, and the CVC, or a salt, solvate, ester and/or prodrug thereof, is administered once per day for 28 days. The 7 days for administering the additional therapeutic agent can be consecutive or non-consecutive. For example, in a consecutive administration, the 7 days for administering the additional therapeutic agent can be day 1 to day 7 in the treatment cycle.

By "interval administration", it is meant a period of co-administration with at least one void day. By "continuous administration", it is meant a period of co-administration without any void day. The continuous administration may be simultaneous, sequential, or overlapping, as described above.

In one embodiment of interval administration, the CVC, or a salt, solvate, ester and/or prodrug thereof, is administered from 1 to 4 times per day for 7 to 21 days; the addition a therapeutic agent is administered 1 to 4 times per day for 7 to 21 days, and there are 1 to 14 void days in the treatment cycle.

In one specific embodiment of interval administration, the present method comprises one or more treatment cycle and each of the treatment cycle has 28 days, wherein the additional therapeutic agent is administered once per day for 7 days, the CVC, or a salt, solvate, ester and/or prodrug thereof, is administered once per day for 7 to 14 days, and there are 7 to 14 void days in the treatment cycle. For example, the additional therapeutic agent is administered once per day from day 1 to day 7; the CVC, or a salt, solvate, ester and/or prodrug thereof, is administered once per day from day 15 to day 28; and day 7 to day 14 are void days in the treatment cycle.

In the present method, the co-administration comprises oral administration, parenteral administration, or a combination thereof. Examples of the parentaeral administration include, but are not limited to intravenous (IV) administration, intraarterial administration, intramuscular administration, subcutaneous administration, intraosseous administration, intrathecal administration, or a combination thereof. The additional therapeutic agent and the CVC, or a salt, solvate, ester and/or prodrug thereof, can be independently administered orally or parenterally. In one embodiment, the CVC, or a salt, solvate, ester and/or prodrug thereof, is administered orally; and the additional therapeutic agent is administered parenterally. The parenteral administration may be conducted via injection or infusion.

In one embodiment of the present method, combination therapy comprises CVC, or a salt, solvate, ester and/or prodrug therof and the additional therapeutic agent comprises farnesoid X receptor (FXR) agonist. In one embodiment of the present method, combination therapy comprises CVC, or a salt, solvate, ester and/or prodrug therof and the additional therapeutic agent comprises high dose vitamin E (> 400 iU/d). In one embodiment of the present method, combination therapy comprises CVC, or a salt, solvate, ester and/or prodrug therof and the additional therapeutic agent comprises a peroxisome proliferator-activated receptor alpha (PPAR-α) agonist. In one embodiment of the present method, combination therapy comprises CVC, or a salt, solvate, ester and/or prodrug therof and the additional therapeutic agent comprises a PPAR-γ agonist. In one embodiment of the present method, combination therapy comprises CVC, or a salt, solvate, ester and/or prodrug therof and the additional therapeutic agent comprises a PPAR-δ agonist. In one embodiment of the present method, combination therapy comprises CVC, or a salt, solvate, ester and/or prodrug therof and the additional therapeutic agent comprises 3-[2-[2-Chloro-4-[[3-(2,6-dichlorophenyl)-5-(1-methylethyl)-4-isoxazolyl]methoxy]phenyl]ethenyl]benzoic acid (GW4064). In one embodiment of the present method, combination therapy comprises CVC, or a salt, solvate, ester and/or prodrug therof and the additional therapeutic agent comprises 2-methyl-2-[[4-[2-[[(cyclohexylamino)carbonyl](4-cyclohexylbutyl)amino]ethyl]phenyl]thio]-propanoic acid (GW7647). In one embodiment of the present method, combination therapy comprises CVC, or a salt, solvate, ester and/or prodrug therof and the additional therapeutic agent comprises pioglitazone. In one embodiment of the present method, combination therapy comprises CVC, or a salt, solvate, ester and/or prodrug thereof and the additional therapeutic agent comprises a chemokine antagonist.

In one embodiment, for one treatment cycle, the additional therapeutic agent is administered for 7 consecutive days before the administration of CVC, or a salt, solvate, ester and/or prodrug thereof for 14 consecutive days. In another embodiment, for one treatment cycle, CVC, or a salt, solvate, ester and/or prodrug thereof is administered for 14 consecutive days before the administration of the additional therapeutic agent for 7 consecutive days. In yet another embodiment, for one treatment cycle, the additional therapeutic agent is administered for the first 7 consecutive days and CVC, or a salt, solvate, ester and/or prodrug thereof is administered for the first 14 consecutive days. In yet another embodiment, for one treatment cycle, the additional therapeutic agent is administered for the first 7 consecutive days, and CVC, or a salt, solvate, ester and/or prodrug thereof is administered for the first 28 consecutive days. In yet another embodiment, for one treatment cycle, CVC, or a salt, solvate, ester and/or prodrug thereof is administered for 28 consecutive days and the additional therapeutic agent is administered for 7 consecutive days that overlap with CVC, or a salt, solvate, ester and/or prodrug thereof administration. In another embodiment, the additional therapeutic agent is administered on days 1 through 7 and CVC, or a salt, solvate, ester and/or prodrug thereof is administered on days 1 through 14, of a treatment cycle. In another embodiment, the additional therapeutic agent is administered on days 1 through 7 and CVC, or a salt, solvate, ester and/or prodrug thereof is administered on days 1 through 28, of a treatment cycle. In another embodiment, the additional therapeutic agent is administered on days 1 to 7 and CVC, or a salt, solvate, ester and/or prodrug thereof is administered on days 8 through 21, of a treatment cycle. In yet another embodiment, CVC, or a salt, solvate, ester and/or prodrug thereof is administered on days 1 through 14 and the additional therapeutic agent is administered on days 15 through 21, of a treatment cycle. In another embodiment, the treatment cycle is 28 days, 29 days, 30 days or 31 days. In another embodiment, the treatment cycle is any length of time from 4 weeks to 6 weeks long.

In one embodiment, the additional therapeutic agent is administered daily by 24-hour continuous infusion. In another embodiment, the additional therapeutic agent is administered every 12 hours by intravenous infusion over 1 to 2 hours. In another embodiment, the additional therapeutic agent is administered twice daily subcutaneously. In another embodiment, in one treatment cycle, the additional therapeutic agent is administered every other day for a total of 3 days of administration. In another embodiment, in one treatment cycle, the additional therapeutic agent is administered every other day for a total of 4 days of administration. In another embodiment, in one treatment cycle, the additional therapeutic agent is administered on days 1, 3 and 5. In yet another embodiment, in one treatment cycle, the additional therapeutic agent is administered on days 1, 3, 5 and 7.

In another embodiment, in one treatment cycle, CVC, or a salt, solvate, ester and/or prodrug thereof is administered for the first 14 consecutive days, a first additional therapeutic agent is administered for 7 consecutive days following the completion of CVC, or a salt, solvate, ester and/or prodrug thereof administration and an a second additional therapeutic agent is administered for 3 days overlapping with the first additional therapeutic compound administration. In another embodiment, in one treatment cycle, CVC, or a salt, solvate, ester and/or prodrug thereof is administered for the first 28 consecutive days, a first additional therapeutic agent is administered for 7 consecutive days following completion of 14 days of administration of CVC, or a salt, solvate, ester and/or prodrug thereof administration and second additional therapeutic agent is administered for 3 days overlapping with the first additional therapeutic agent administration.

In another embodiment, in one treatment cycle, a first additional therapeutic agent is administered for the first 7 consecutive days, a second additional therapeutic agent is administered for 3 consecutive days overlapping with the first additional therapeutic agent administration and CVC, or a salt, solvate, ester and/or prodrug thereof is administered for the first 28 consecutive days. In another embodiment, in one treatment cycle, a first additional therapeutic agent is administered for the first 7 consecutive days, a second additional therapeutic agent is administered for 3 consecutive days overlapping with the first additional therapeutic agent administration and CVC, or a salt, solvate, ester and/or prodrug thereof is administered for the first 14 consecutive days. In another embodiment, in one treatment cycle, a first additional therapeutic agent is administered on days 1 through 7 and a second additional therapeutic agent is administered on days 1 through 3 and CVC, or a salt, solvate, ester and/or prodrug thereof is administered on days 1 through 14. In another embodiment, in one treatment cycle, a first additional therapeutic agent is administered on days 1 through 7 and a second additional therapeutic agent is administered on days 1 through 3 and CVC, or a salt, solvate, ester and/or prodrug thereof is administered on days 1 through 28. In another embodiment, a first additional therapeutic agent is administered on days 1 through 7 and a second additional therapeutic agent is administered on days 1 through 3 and CVC, or a salt, solvate, ester and/or prodrug thereof is administered on days 1 through 7 and 15 through 21.

In another embodiment, for one treatment cycle, CVC, or a salt, solvate, ester and/or prodrug thereof is orally administered at 200 mg/day on days 1 through 14 and a first additional therapeutic agent is administered intravenously at 100 mg/m² /day on days 1 through 7 and a second additional therapeutic agent is administered intravenously at 60 mg/m²/day on days 1 through 3. In yet another embodiment, for one treatment cycle, CVC, or a salt, solvate, ester and/or prodrug thereof is orally administered at 200 mg/day on days 1 through 7 and days 15 through 21 and a first additional therapeutic agent is administered intravenously at 100 mg/m²/day on days 1 through 7 and a second additional therapeutic agent is administered intravenously at 60 mg/m²/day on days 1 through 3. In another embodiment, for one treatment cycle, CVC, or a salt, solvate, ester and/or prodrug thereof is orally administered at 60 mg/day on days 1 through 14 and a first additional therapeutic agent is administered intravenously at 100 mg/m²/day on days 1 through 7 and a second additional therapeutic agent is administered intravenously at 60 mg/m²/day on days 1 through 3. In another embodiment, for one treatment cycle, CVC, or a salt, solvate, ester and/or prodrug thereof is orally administered at 60 mg/day on days 1 through 28 and a first additional therapeutic agent is administered intravenously at 100 mg/m²/day on days 1 through 7 and a second additional therapeutic agent is administered intravenously at 60 mg/m²/day on days 1 through 3. In yet another embodiment, for one treatment cycle, CVC, or a salt, solvate, ester and/or prodrug thereof is orally administered at 60 mg/day on days 1 through 7 and days 15 through 21 and a first additional therapeutic agent is administered intravenously at 100 mg/m2/day on days 1 through 7 and a second additional therapeutic agent is administered intravenously at 60 mg/m²/day on days 1 through 3.

In one specific embodiment, for one treatment cycle, a first additional therapeutic agent is administered intravenously at 100 mg/m²/day on days 1 through 7 and a second additional therapeutic agent is administered intravenously at 60 mg/m²/day on days 1 through 3 and CVC, or a salt, solvate, ester and/or prodrug thereof is orally administered at 200 mg/day on days 8 through 21.

In yet another embodiment, the combination regimen is given as first line therapy (for example, to patients unfit for standard treatment of hepatic or renal fibrosis). In another embodiment, the combination regimen is given as second line therapy (for example, to patients who have hepatic or renal fibrosis after receiving prior therapy).

### Pharmaceutical Compositions, Dosages and Administration:

In one aspect, the present invention provides a pharmaceutical composition and a combination package that are useful for treating fibrosis and/or fibrotic diseases or conditions.

The compositions are intended to be administered by a suitable route, including, but not limited to, orally, parenterally, rectally, topically and locally. For oral administration, capsules and tablets can be formulated. The compositions are in liquid, semi-liquid or solid form and are formulated in a manner suitable for each route of administration.

In one embodiment, the pharmaceutical composition comprises a therapeutically effective amount of (a) an additional therapeutic agent; and (b) CVC, or a salt, solvate, ester and/or prodrug thereof. Ingredients (a) and (b) are pharmaceutically active ingredients. The pharmaceutical composition may comprise additional active ingredients besides (a) and (b). For example, the pharmaceutical composition may comprise the additional active agent as described above. In one embodiment, the additional active agent is a FXR or PPAR-α agonist. In one embodiment, the additional active agent is a chemokine antagonist.

In one specific embodiment, the pharmaceutical composition comprises a FXR agonist and CVC, or a salt, solvate, ester and/or prodrug thereof. In another specific embodiment, the pharmaceutical composition comprises a PPAR-α agonist and CVC, or a salt, solvate, ester and/or prodrug thereof. In one specific embodiment, the pharmaceutical composition comprises a chemokine antagonist and CVC, or a salt, solvate, ester and/or prodrug thereof.

A dosage of a particular subject can be determined according to the subject's age, weight, general health conditions, sex, meal, administration time, administration route, excretion rate and the degree of particular disease conditions to be treated by taking into consideration of these and other factors.

The present invention provides a method of treatment, wherein the cenicriviroc or a salt or solvate thereof is formulated as an oral composition.

The present invention provides a method of treatment, wherein the cenicriviroc or a salt or solvate thereof is administered, for example, once per day or twice per day. The dosage form can be administered for a duration of time sufficient to treat the fibrotic disease or condition.

In the case of oral administration, a daily dosage is in a range of about 5 to 1000 mg, preferably about 10 to 600 mg, and more preferably about 10 to 300 mg, most preferably about 15 to 200 mg as the active ingredient (i.e. as the compound of the invention) per an adult of body weight of 50 kg, and the medicine may be administered, for example, once, or in 2 to 3 divided doses a day.

The cenicriviroc or a salt or solvate thereof may be formulated into any dosage form suitable for oral or injectable administration. When the compound is administered orally, it can be formulated into solid dosage forms for oral administration, for example, tablets, capsules, pills, granules, and so on. It also can be formulated into liquid dosage forms for oral administration, such as oral solutions, oral suspensions, syrups and the like. The term "tablets" as used herein, refers to those solid preparations which are prepared by homogeneously mixing and pressing the compounds and suitable auxiliary materials into circular or irregular troches, mainly in common tablets for oral administration, including also buccal tablets, sublingual tablets, buccal wafer, chewable tablets, dispersible tablets, soluble tablets, effervescent tablets, sustained-release tablets, controlled-release tablets, enteric-coated tablets and the like. The term "capsules" as used herein, refers to those solid preparations which are prepared by filling the compounds, or the compounds together with suitable auxiliary materials into hollow capsules or sealing into soft capsule materials. According to the solubility and release property, capsules can be divided into hard capsules (regular capsules), soft capsules (soft shell capsules), sustained-release capsules, controlled-release capsules, enteric-coated capsules and the like. The term "pills" as used herein, refers to spherical or near-spherical solid preparations which are prepared by mixing the compounds and suitable auxiliary materials via suitable methods, including dropping pills, dragee, pilule and the like. The term "granules" as used herein, refers to dry granular preparations which are prepared by mixing the compounds and suitable auxiliary materials and have a certain particle size. Granules can be divided into soluble granules (generally referred to as granules), suspension granules, effervescent granules, enteric-coated granules, sustained-release granules, controlled-release granules and the like. The term "oral solutions" as used herein, refers to a settled liquid preparation which is prepared by dissolving the compounds in suitable solvents for oral administration. The term "oral suspensions" as used herein, refers to suspensions for oral administration, which are prepared by dispersing the insoluble compounds in liquid vehicles, also including dry suspension or concentrated suspension. The term "syrups" as used herein, refers to a concentrated sucrose aqueous solution containing the compounds. The injectable dosage form can be produced by the conventional methods in the art of formulations, and aqueous solvents or non-aqueous solvents may be selected. The most commonly used aqueous solvent is water for injection, as well as 0.9% sodium chloride solution or other suitable aqueous solutions. The commonly used non-aqueous solvent is vegetable oil, mainly soy bean oil for injection, and others aqueous solutions of alcohol, propylene glycol, polyethylene glycol, and *etc.*

In one embodiment, a pharmaceutical composition comprising cenicriviroc or a salt thereof and fumaric acid is provided. In certain embodiments, the cenicriviroc or salt thereof is cenicriviroc mesylate.

In further embodiments, the weight ratio of cenicriviroc or salt thereof to fumaric acid is from about 7:10 to about 10:7, such as from about 8:10 to about 10:8, from about 9:10 to about 10:9, or from about 95:100 to about 100:95. In other further embodiments, the fumaric acid is present in an amount of from about 15% to about 40%, such as from about 20% to about 30%, or about 25%, by weight of the composition. In other further embodiments, the cenicriviroc or salt thereof is present in an amount of from about 15% to about 40%, such as from about 20% to about 30%, or about 25%, by weight of the composition.

In other further embodiments, the composition of cenicriviroc or a salt thereof and fumaric acid further comprises one or more fillers. In more specific embodiments, the one or more fillers are selected from microcrystalline cellulose, calcium phosphate dibasic, cellulose, lactose, sucrose, mannitol, sorbitol, starch, and calcium carbonate. For example, in certain embodiments, the one or more fillers is microcrystalline cellulose. In particular embodiments, the weight ratio of the one or more fillers to the cenicriviroc or salt thereof is from about 25:10 to about 10:8, such as from about 20:10 to about 10:10, or about 15:10. In other particular embodiments, the one or more fillers are present in an amount of from about 25% to about 55%, such as from about 30% to about 50% or about 40%, by weight of the composition. In other further embodiments, the composition further comprises one or more disintegrants. In more specific embodiments, the one or more disintegrants are selected from cross-linked polyvinylpyrrolidone, cross-linked sodium carboxymethyl cellulose, and sodium starch glycolate. For example, in certain embodiments, the one or more disintegrants is cross-linked sodium carboxymethyl cellulose. In particular embodiments, the weight ratio of the one or more disintegrants to the cenicriviroc or salt thereof is from about 10:10 to about 30:100, such as about 25:100. In other particular embodiments, the one or more disintegrants are present in an amount of from about 2% to about 10%, such as from about 4% to about 8%, or about 6%, by weight of the composition. In other further embodiments, the composition further comprises one or more lubricants. In more specific embodiments, the one or more lubricants are selected from talc, silica, stearin, magnesium stearate, and stearic acid. For example, in certain embodiments, the one or more lubricants is magnesium stearate. In particular embodiments, the one or more lubricants are present in an amount of from about 0.25% to about 5%, such as from about 0.75% to about 3%, or about 1.25%, by weight of the composition.

In other further embodiments, the composition of cenicriviroc or a salt thereof and fumaric acid is substantially similar to that of Table 2. In other further embodiments, the composition of cenicriviroc or a salt thereof and fumaric acid is substantially similar to that of Tables 3 and 4. In other further embodiments, any of the compositions of cenicriviroc or a salt thereof and fumaric acid is produced by a process involving dry granulation. In other further embodiments, any of the compositions of cenicriviroc or a salt thereof and fumaric acid has a water content of no more than about 4% by weight, such as no more than 2% by weight, after six weeks exposure to about 40° C at about 75% relative humidity when packaged with desiccant. In other further embodiments, any of the above-mentioned compositions has a total impurity level of no more than about 2.5%, such as no more than 1.5%, after 12 weeks of exposure to 40° C at 75% relative humidity when packaged with desiccant. In other further embodiments, the cenicriviroc or salt thereof of any of the above-mentioned compositions has a mean absolute bioavailability after oral administration that is substantially similar to the bioavailability of the cenicriviroc or salt thereof in a solution after oral administration. In yet further embodiments, the cenicriviroc or salt thereof has an absolute bioavailability of about 10% to about 50%, such as about 27%, in beagle dogs.

In another embodiment, a pharmaceutical formulation is provided that comprises a composition of cenicriviroc or a salt thereof and fumaric acid. In further embodiments, the composition in the formulation can be in the form of a granulate. In other further embodiments, the composition in the formulation is disposed in a capsule shell. In other further embodiments, the composition of the formulation is disposed in a sachet. In other further embodiments, the composition of the formulation is a tablet or a component of a tablet. In still other further embodiments, the composition of the formulation is one or more layers of a multi-layered tablet. In other further embodiments, the formulation comprises one or more additional pharmaceutically inactive ingredients. In other further embodiments, the formulation is substantially similar to that of Table 9. In other further embodiments, a tablet having a composition substantially similar to of Table 9 is provided. In other further embodiments, any of the above embodiments are coated substrates. In another embodiment, methods for preparing any of the above-mentioned embodiments are provided. In further embodiments, the method comprises admixing cenicriviroc or a salt thereof and fumaric acid to form an admixture, and dry granulating the admixture. In other further embodiments, the method further comprises admixing one or more fillers with the cenicriviroc or salt thereof and fumaric acid to form the admixture. In other further embodiments, the method further comprises admixing one or more disintegrants with the cenicriviroc or salt thereof and fumaric acid to form the admixture. In other further embodiments, the method further comprises admixing one or more lubricants with the cenicriviroc or salt thereof and fumaric acid to form the admixture. In other further embodiments, the method further comprises compressing the dry granulated admixture into a tablet. In other further embodiments, the method comprises filling a capsule with the dry granulated admixture.

Further, the compound of the invention can be included or used in combination with blood for transfusion or blood derivatives. In one embodiment, the compound of the invention can be included or used in combination with one or more agents that purge latent HIV reservoirs and added to blood for transfusion or blood derivatives. Usually, blood for transfusion or blood derivatives are produced by mixing blood obtained form plural persons and, in some cases, uninfected cells are contaminated with cells infected with HIV virus. In such a case, uninfected cells are likely to be infected with HIV virus. When the compound of the present invention is added to blood for transfusion or blood derivatives along with one or more agents that purge latent HIV reservoirs, infection and proliferation of the virus can be prevented or controlled. Especially, when blood derivatives are stored, infection and proliferation of the virus is effectively prevented or controlled by addition of the compound of the present invention. In addition, when blood for transfusion or blood derivatives contaminated with HIV virus are administered to a person, infection and proliferation of the virus in the person's body can be prevented by adding the compound of the invention to the blood or blood derivatives in combination with one or more agents that purge latent HIV reservoirs. For example, usually, for preventing HIV infectious disease upon using blood or blood derivatives by oral administration, a dosage is in a range of about 0.02 to 50 mg/kg, preferably about 0.05 to 30 mg/kg, and more preferably about 0.1 to 10 mg/kg as the CCR5/CCR2 antagonist per an adult of body weight of about 60 kg, and the medicine may be administered once or 2 to 3 doses a day. As a matter of course, although the dosage range can be controlled on the basis of unit dosages necessary for dividing the daily dosage, as described above, a dosage of a particular subject can be determined according to the subject's age, weight, general health conditions, sex, meal, administration time, administration route, excretion rate and the degree of particular disease conditions to be treated by taking into consideration of these and other factors. In this case, the administration route is also appropriately selected and, the medicine for preventing HIV infectious disease of the present invention may be added directly to blood for transfusion or blood derivatives before transfusion or using blood derivatives. In such a case, desirably, the medicine of the present invention is mixed with blood or blood derivatives immediately to 24 hours before, preferably immediately to 12 hours before, more preferably immediately to 6 hours before transfusion or using blood derivatives.

Aside from blood for transfusion or blood derivatives, when the compositions of the invention is administered together with the blood for transfusion or blood derivatives and/or other active agents, the medicine is administered preferably at the same time of, to 1 hour before transfusion or using the blood derivatives. More preferably, for example, the medicine is administered once to 3 times per day and the administration is continued 4 weeks.

In another embodiment, the pharmaceutical composition further comprises a pharmaceutically acceptable excipient. The pharmaceutically acceptable excipient can be any inert or slightly active substance used in preparing a pharmaceutical composition as a vehicle, carrier, or medium of administration for the active ingredients. In one embodiment, the pharmaceutically acceptable excipient is a release-rate controlling ingredient. By "release-rate controlling ingredient", it is meant an ingredient that can control the release rate of the active ingredients. The pharmaceutically acceptable excipient can be used with the active ingredients to formulate suitable pharmaceutical preparations such as solutions, suspensions, tablets, dispersible tablets, pills, capsules, powders, sustained release formulations or elixirs, for oral administration or in sterile solutions or suspensions for parenteral administration, as well as transdermal patch preparation and dry powder inhalers. Typically the active ingredients described above are formulated into pharmaceutical compositions using techniques and procedures well known in the art.

In one embodiment, the combination package comprises (a) at least one individual dose of an additional therapeutic agent, and (b) at least one individual dose of CVC or a salt, solvate, ester and/or prodrug thereof. In another embodiment, the combination package further comprises an instruction document providing a protocol for co-administering (a) and (b).

Typically, the compositions are formulated for single dosage administration. To formulate a composition, the weight fraction of (a) and (b) is dissolved, suspended, dispersed or otherwise mixed in a selected vehicle at an effective concentration such that the treated condition is relieved or ameliorated. Pharmaceutical carriers, vehicles, or other medium suitable for administration of (a) and (b) provided herein include any such carriers known to those skilled in the art to be suitable for the particular mode of administration.

In addition, (a) and (b) may be formulated as the only pharmaceutically active ingredient in the composition or may be combined with other active ingredients. Liposomal suspensions, including tissue- targeted liposomes, such as tumor-targeted liposomes, may also be suitable as pharmaceutically acceptable carriers. These may be prepared according to methods known to those skilled in the art. For example, liposome formulations may be prepared as known in the art. Briefly, liposomes such as multilamellar vesicles (MLVs) may be formed by drying down egg phosphatidyl choline and brain phosphatidyl serine (7:3 molar ratio) on the inside of a flask. A solution of a compound provided herein in phosphate buffered saline lacking divalent cations (PBS) is added and the flask shaken until the lipid film is dispersed. The resulting vesicles are washed to remove unencapsulated compound, pelleted by centrifugation, and then resuspended in PBS.

The active ingredients are included in the pharmaceutically acceptable excipient in an amount sufficient to exert a therapeutically useful effect with minimal or no undesirable side effects on the patient treated. The concentration of active ingredients in the pharmaceutical composition will depend on absorption, inactivation and excretion rates of the active compounds, the physicochemical characteristics of the compound, the dosage schedule, and amount administered as well as other factors known to those of skill in the art.

Typically a therapeutically effective dosage should produce a serum concentration of active ingredient of from about 0.1 ng/ml to about 50 to about 100 µg/ml. The pharmaceutical compositions typically should provide a dosage of from about 0.001 mg to about 2000 mg of compound per kilogram of body weight per day. Pharmaceutical dosage unit forms are prepared to provide from about 1 mg to about 1000 mg and in certain embodiments, from about 10 mg to about 500 mg, from about 20 mg to about 250 mg or from about 25 mg to about 100 mg of the essential active ingredient or a combination of essential ingredients per dosage unit form. In certain embodiments, the pharmaceutical dosage unit forms are prepared to provide about 1 mg, 20 mg, 25 mg, 50 mg, 100 mg, 250 mg, 500 mg, 1000 mg or 2000 mg of the essential active ingredient. In certain embodiments, the pharmaceutical dosage unit forms are prepared to provide about 50 mg of the essential active ingredient.

The active ingredients may be administered at once, or may be divided into a number of smaller doses to be administered at intervals of time. It is understood that the precise dosage and duration of treatment is a function of the disease being treated and may be determined empirically using known testing protocols or by extrapolation from in vivo or in vitro test data. It is to be noted that concentrations and dosage values may also vary with the severity of the condition to be alleviated and/or the age, bodyweight, and other health concerns of the patient. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

Thus, effective concentrations or amounts of the active ingredients described herein or pharmaceutically acceptable derivatives thereof are mixed with a suitable pharmaceutical carrier, vehicle, or other medium for systemic, topical or local administration to form pharmaceutical compositions. Compounds are included in an amount effective for ameliorating one or more symptoms of, or for treating or preventing fibrosis and/or fibrotic diseases or conditions. The concentration of active compound in the composition will depend on absorption, inactivation, excretion rates of the active compound, the dosage schedule, amount administered, particular formulation as well as other factors known to those of skill in the art.

Solutions or suspensions used for parenteral, intradermal, subcutaneous, or topical application can include any of the following components: a sterile diluent, such as water for injection, saline solution, fixed oil, polyethylene glycol, glycerine, propylene glycol, dimethyl acetamide or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; and agents for the adjustment of tonicity such as sodium chloride or dextrose. Parenteral preparations can be enclosed in ampules, disposable syringes or single or multiple dose vials made of glass, plastic or other suitable material.

In instances in which the active ingredients exhibit insufficient solubility, methods for solubilizing compounds may be used. Such methods are known to those of skill in this art, and include, but are not limited to, using cosolvents, such as dimethylsulfoxide (DMSO), using surfactants, such as TWEEN®, or dissolution in aqueous sodium bicarbonate.

Upon mixing or addition of the active ingredients, the resulting mixture may be a solution, suspension, emulsion or the like. The form of the resulting mixture depends upon a number of factors, including the intended mode of administration and the solubility of the compound in the selected carrier or vehicle. In one embodiment, the effective concentration is sufficient for ameliorating the symptoms of the disease, disorder or condition treated and may be empirically determined.

The pharmaceutical compositions are provided for administration to humans and animals in unit dosage forms, such as tablets, capsules, pills, powders, granules, sterile parenteral solutions or suspensions, and oral solutions or suspensions, and oil-water emulsions containing suitable quantities of the compounds or pharmaceutically acceptable derivatives thereof. The pharmaceutically therapeutically active compounds and derivatives thereof are typically formulated and administered in unit-dosage forms or multiple-dosage forms. Unit-dose forms as used herein refer to physically discrete units suitable for human and animal subjects and packaged individually as is known in the art. Each unit-dose contains a predetermined quantity of the therapeutically active compound sufficient to produce the desired therapeutic effect, in association with the required pharmaceutical carrier, vehicle or diluent. Examples of unit-dose forms include ampules and syringes and individually packaged tablets or capsules. Unit-dose forms may be administered in fractions or multiples thereof. A multiple-dose form is a plurality of identical unit-dosage forms packaged in a single container to be administered in segregated unit-dose form. Examples of multiple-dose forms include vials, bottles of tablets or capsules or bottles of pints or gallons. Hence, multiple dose form is a multiple of unit-doses which are not segregated in packaging.

Controlled-release preparations can also be prepared. Suitable examples of controlled-release preparations include semipermeable matrices of solid hydrophobic polymers containing the active ingredients provided herein, which matrices are in the form of shaped articles, e.g., films, or microcapsule. Examples of controlled-release matrices include polyesters, hydrogels (for example, poly(2-hydroxyethyl-methacrylate), or poly(vinylalcohol)), polylactides, copolymers of L-glutamic acid and ethyl-L-giutamate, non- degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated active ingredients remain in the body for a long time, they may denature or aggregate as a result of exposure to moisture at 37 °C, resulting in a loss of biological activity and possible changes in their structure. Rational strategies can be devised for stabilization depending on the mechanism of action involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions. Other examples of controlled-release preparations include coated compositions. For example, the coating may function as a barrier to decrease the release rate of the active ingredients; or the coating is enteric, i.e., pratically insoluble in an acidic environment and thereby delays the release of the active ingredients until the composition reaches the lower GI tract wherein the pH environment is neutral or basic.

Dosage forms or compositions containing active ingrediensts in the range of 0.005% to 100% with the balance made up from non-toxic excipient may be prepared. For oral administration, a pharmaceutically acceptable non-toxic composition is formed by the incorporation of any of the normally employed excipients, such as, for example pharmaceutical grades of mannitol, lactose, starch, magnesium stearate, talcum, cellulose derivatives, sodium crosscarmellose, glucose, sucrose, magnesium carbonate or sodium saccharin. Such compositions include solutions, suspensions, tablets, capsules, powders and sustained release formulations, such as, but not limited to, implants and microencapsulated delivery systems, and biodegradable, biocompatible polymers, such as collagen, ethylene vinyl acetate, polyanhydrides, polyglycolic acid, polyorthoesters, polylactic acid and others. Methods for preparation of these compositions are known to those skilled in the art. The contemplated compositions may contain about 0.001%-100% active ingredients, in certain embodiments, about 0.1-85%, typically about 75-95%.

The active ingredients or pharmaceutically acceptable derivatives may be prepared with carriers that protect the active ingredients against rapid elimination from the body, such as time release formulations or coatings. The compositions may include other active compounds to obtain desired combinations of properties. The active ingredients provided herein, or pharmaceutically acceptable derivatives thereof as described herein, may also be advantageously administered for therapeutic or prophylactic purposes together with another pharmacological agent known in the general art to be of value in treating one or more of the diseases or medical conditions referred to hereinabove, such as fibrosis and/or fibrotic diseases or conditions. It is to be understood that such combination therapy constitutes a further aspect of the compositions and methods of treatment provided herein.

### Compositions for oral administration

Oral pharmaceutical dosage forms are either solid, gel or liquid. The solid dosage forms are tablets, capsules, granules, and bulk powders. Types of oral tablets include compressed, chewable lozenges and tablets which may be enteric-coated, sugar-coated or film-coated. Capsules may be hard or soft gelatin capsules, while granules and powders may be provided in non-effervescent or effervescent form with the combination of other ingredients known to those skilled in the art.

In certain embodiments, the formulations are solid dosage forms, such as capsules or tablets. The tablets, pills, capsules, troches and the like can contain any of the following ingredients, or compounds of a similar nature: a binder; a diluent; a disintegrating agent; a lubricant; a glidant; a sweetening agent; and a flavoring agent.

Examples of binders include microcrystalline cellulose, gum tragacanth, glucose solution, acacia mucilage, gelatin solution, sucrose and starch paste. Lubricants include talc, starch, magnesium or calcium stearate, lycopodium and stearic acid. Diluents include, for example, lactose, sucrose, starch, kaolin, salt, mannitol and dicalcium phosphate. Glidants include, but are not limited to, colloidal silicon dioxide. Disintegrating agents include crosscarmellose sodium, sodium starch glycolate, alginic acid, corn starch, potato starch, bentonite, methylcellulose, agar and carboxymethylcellulose. Coloring agents include, for example, any of the approved certified water soluble FD and C dyes, mixtures thereof; and water insoluble FD and C dyes suspended on alumina hydrate. Sweetening agents include sucrose, lactose, mannitol and artificial sweetening agents such as saccharin, and any number of spray dried flavors. Flavoring agents include natural flavors extracted from plants such as fruits and synthetic blends of compounds which produce a pleasant sensation, such as, but not limited to peppermint and methyl salicylate. Wetting agents include propylene glycol monostearate, sorbitan monooleate, diethylene glycol monolaurate and polyoxyethylene laural ether. Emetic-coatings include fatty acids, fats, waxes, shellac, ammoniated shellac and cellulose acetate phthalates. Film coatings include hydroxyethylcellulose, sodium carboxymethylcellulose, polyethylene glycol 4000 and cellulose acetate phthalate.

If oral administration is desired, the active ingredients could be provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

When the dosage unit form is a capsule, it can contain, in addition to material of the above type, a liquid carrier such as a fatty oil. In addition, dosage unit forms can contain various other materials which modify the physical form of the dosage.unit, for example, coatings of sugar and other enteric agents. The active ingredients can also be administered as components of an elixir, suspension, syrup, wafer, sprinkle, chewing gum or the like. A syrup may contain, in addition to the active compounds, sucrose as a sweetening agent and certain preservatives, dyes and colorings and flavors. The active ingredients can also be mixed with other active materials which do not impair the desired action, or with materials that supplement the desired action, such as antacids, H2 blockers, and diuretics.

Pharmaceutically acceptable excipients included in tablets are binders, lubricants, diluents, disintegrating agents, coloring agents, flavoring agents, and wetting agents. Enteric-coated tablets, because of the enteric-coating, resist the action of stomach acid and dissolve or disintegrate in the neutral or alkaline intestines. Sugar-coated tablets are compressed tablets to which different layers of pharmaceutically acceptable substances are applied. Film-coated tablets are compressed tablets which have been coated with a polymer or other suitable coating. Multiple compressed tablets are compressed tablets made by more than one compression cycle utilizing the pharmaceutically acceptable substances previously mentioned. Coloring agents may also be used in the above dosage forms. Flavoring and sweetening agents are used in compressed tablets, sugar-coated, multiple compressed and chewable tablets. Flavoring and sweetening agents are especially useful in the formation of chewable tablets and lozenges. Liquid oral dosage forms include aqueous solutions, emulsions, suspensions, solutions and/or suspensions reconstituted from non-effervescent granules and effervescent preparations reconstituted from effervescent granules. Aqueous solutions include, for example, elixirs and syrups. Emulsions are either oil-in-water or water-in-oil.

Elixirs are clear, sweetened, hydroalcoholic preparations. Pharmaceutically acceptable excipients used in elixirs include solvents. Syrups are concentrated aqueous solutions of a sugar, for example, sucrose, and may contain a preservative. An emulsion is a two-phase system in which one liquid is dispersed in the form of small globules throughout another liquid. harmaceutically acceptable excipients used in emulsions are non-aqueous liquids, emulsifying agents and preservatives. Suspensions use pharmaceutically acceptable suspending agents and preservatives. Pharmaceutically acceptable substances used in non-effervescent granules, to be reconstituted into a liquid oral dosage form, include diluents, sweeteners and wetting agents. Pharmaceutically acceptable substances used in effervescent granules, to be reconstituted into a liquid oral dosage form, include organic acids and a source of carbon dioxide. Coloring and flavoring agents are used in all of the above dosage forms. Solvents include glycerin, sorbitol, ethyl alcohol and syrup.

Examples of preservatives include glycerin, methyl and propylparaben, benzoic add, sodium benzoate and alcohol. Examples of non-aqueous liquids utilized in emulsions include mineral oil and cottonseed oil. Examples of emulsifying agents include gelatin, acacia, tragacanth, bentonite, and surfactants such as polyoxyethylene sorbitan monooleate. Suspending agents include sodium carboxymethylcellulose, pectin, tragacanth, Veegum and acacia. Diluents include lactose and sucrose. Sweetening agents include sucrose, syrups, glycerin and artificial sweetening agents such as saccharin. Wetting agents include propylene glycol monostearate, sorbitan monooleate, diethylene glycol monolaurate and polyoxyethylene lauryl ether. Organic adds include citric and tartaric acid. Sources of carbon dioxide include sodium bicarbonate and sodium carbonate. Coloring agents include any of the approved certified water soluble FD and C dyes, and mixtures thereof. Flavoring agents include natural flavors extracted from plants such fruits, and synthetic blends of compounds which produce a pleasant taste sensation.

For a solid dosage form, the solution or suspension, in for example propylene carbonate, vegetable oils or triglycerides, is encapsulated in a gelatin capsule. For a liquid dosage form, the solution, e.g., for example, in a polyethylene glycol, may be diluted with a sufficient quantity of a pharmaceutically acceptable liquid carrier, e.g., water, to be easily measured for administration.

Alternatively, liquid or semi-solid oral formulations may be prepared by dissolving or dispersing the active compound or salt in vegetable oils, glycols, triglycerides, propylene glycol esters (e.g., propylene carbonate) and other such carriers, and encapsulating these solutions or suspensions in hard or soft gelatin capsule shells. Other useful formulations include, but are not limited to, those containing a compound provided herein, a dialkylated mono- or poly-alkylene glycol, including, but not limited to, 1 ,2- dimethoxymethane, diglyme, triglyme, tetraglyme, polyethylene glycol-350- dimethyl ether, polyethylene glycol-550-dimethyl ether, polyethylene glycol-750-dimethyl ether wherein 350, 550 and 750 refer to the approximate average molecular weight of the polyethylene glycol, and one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, thiodipropionic acid and its esters, and dithiocarbamates.

Other formulations include, but are not limited to, aqueous alcoholic solutions including a pharmaceutically acceptable acetal. Alcohols used in these formulations are any pharmaceutically acceptable water- miscible solvents having one or more hydroxyl groups, including, but not limited to, propylene glycol and ethanol. Acetals include, but are not limited to, di(lower alkyl) acetals of lower alkyl aldehydes such as acetaldehyde diethyl acetal.

In all embodiments, tablets and capsules formulations may be coated as known by those of skill in the art in order to modify or sustain dissolution of the active ingredient. Thus, for example, they may be coated with a conventional enterically digestible coating, such as phenylsalicylate, waxes and cellulose acetate phthalate.

### Injectables, solutions and emulsions

Parenteral administration, generally characterized by injection, either subcutaneously, intramuscularly or intravenously is also contemplated herein. Injectables can be prepared in conventional forms, either as liquid solutions or suspensions, solid forms suitable for solution or suspension in liquid prior to injection, or as emulsions. Suitable excipients are, for example, water, saline, dextrose, glycerol or ethanol. In addition, if desired, the pharmaceutical compositions to be administered may also contain minor amounts of non-toxic auxiliary substances such as wetting or emulsifying agents, pH buffering agents, stabilizers, solubility enhancers, and other such agents, such as for example, sodium acetate, sorbitan monolaurate, triethanolamine oleate and cyclodextrins. In one embodiment, the composition is administered as an aqueous solution with hydroxypropyl-beta-cyclodextrin (HPBCD) as an excipient. In one embodiment, the aqueous solution contains about 1 % to about 50% HPBCD. In one embodiment, the aqueous solution contains about 1%, 3%, 5%, 10% or about 20% HPBCD.

Implantation of a slow-release or sustained-release system, such that a constant level of dosage is maintained is also contemplated herein. Briefly, a compound provided herein is dispersed in a solid inner matrix, e.g., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, e.g., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, - ethylene/vinyl acetate/vinyl alcohol terpolymer, and ethylene/viπyloxyethanol copolymer, that is insoluble in body fluids. The compound diffuses through the outer polymeric membrane in a release rate controlling step. The percentage of active compound contained in such parenteral compositions is highly dependent on the specific nature thereof, as well as the activity of the compound and the needs of the subject.

Parenteral administration of the compositions includes intravenous, subcutaneous and intramuscular administrations. Preparations for parenteral administration include sterile solutions ready for injection, sterile dry soluble products, such as lyophilized powders, ready to be combined with a solvent just prior to use, including hypodermic tablets, sterile suspensions ready for injection, sterile dry insoluble products ready to be combined with a vehicle just prior to use and sterile emulsions. The solutions may be either aqueous or nonaqueous.

If administered intravenously, suitable carriers include physiological saline or phosphate buffered saline (PBS), and solutions containing thickening and solubilizing agents, such as glucose, polyethylene glycol, and polypropylene glycol and mixtures thereof.

Pharmaceutically acceptable excipients used in parenteral preparations include aqueous vehicles, nonaqueous vehicles, antimicrobial agents, isotonic agents, buffers, antioxidants, local anesthetics, suspending and dispersing agents, emulsifying agents, sequestering or chelating agents and other pharmaceutically acceptable substances.

Examples of aqueous vehicles include Sodium Chloride Injection, Ringers Injection, Isotonic Dextrose Injection, Sterile Water Injection, Dextrose and Lactated Ringers Injection. Nonaqueous parenteral vehicles include fixed oils of vegetable origin, cottonseed oil, corn oil, sesame oil and peanut oil. Antimicrobial agents in bacteriostatic or fungistatic concentrations must be added to parenteral preparations packaged in multiple-dose containers which include phenols or cresofs, mercurials, benzyl alcohol, chlorobutanol, methyl and propyl p-hydroxybenzoic acid esters, thimerosal, benzalkonium chloride and benzethonium chloride. Isotonic agents include sodium chloride and dextrose. Buffers include phosphate and citrate. Antioxidants include sodium bisulfate. Local anesthetics include procaine hydrochloride. Suspending and dispersing agents include sodium carboxymethylcelluose, hydroxypropyl methylcellulose and polyvinylpyrrolidone. Emulsifying agents include Polysorbate 80 (TWEEN® 80). A sequestering or chelating agent of metal ions include EDTA. Pharmaceutical carriers also include ethyl alcohol, polyethylene glycol and propylene glycol for water miscible vehicles and sodium hydroxide, hydrochloric acid, citric acid or lactic acid for pH adjustment. The concentration of the pharmaceutically active compound is adjusted so that an injection provides an effective amount to produce the desired pharmacological effect. The exact dose depends on the age, weight and condition of the patient or animal as is known in the art. The unit-dose parenteral preparations are packaged in an ampule, a vial or a syringe with a needle. All preparations for parenteral administration must be sterile, as is known and practiced in the art.

Illustratively, intravenous or intraarterial infusion of a sterile aqueous solution containing an active compound is an effective mode of administration. Another embodiment is a sterile aqueous or oily solution or suspension containing an active material injected as necessary to produce the desired pharmacological effect.

Injectables are designed for local and systemic administration. Typically a therapeutically effective dosage is formulated to contain a concentration of at least about 0.1% w/w up to about 90% w/w or more, such as more than 1 % w/w of the active compound to the treated tissue(s). The active ingredients may be administered at once, or may be divided into a number of smaller doses to be administered at intervals of time. It is understood that the precise dosage and duration of treatment is a function of the tissue being treated and may be determined empirically using known testing protocols or by extrapolation from in vivo or in vitro test data. It is to be noted that concentrations and dosage values may also vary with the age of the individual treated. It is to be further understood that for any particular subject, specific dosage regimens should be adjusted over time according to the individual need and the professional judgment of the person administering or supervising the administration of the formulations, and that the concentration ranges set forth herein are exemplary only and are not intended to limit the scope or practice of the claimed formulations.

The compound may be suspended in micronized or other suitable form or may be derivatized to produce a more soluble active product or to produce a prodrug. The form of the resulting mixture depends upon a number of factors, including the intended mode of administration and the solubility of the compound in the selected carrier or vehicle. The effective concentration is sufficient for ameliorating the symptoms of the condition and may be empirically determined.

### Sustained Release Compositions

Active ingredients provided herein can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719, 5,674,533, 5,059,595, 5,591 ,767, 5,120,548, 5,073,543, 5,639,476, 5,354,556, 5,639,480, 5,733,566, 5,739,108, 5,891,474, 5,922,356, 5,972,891 , 5,980,945, 5,993,855, 6,045,830, 6,087,324, 6,113,943, 6,197,350, 6,248,363, 6,264,970, 6,267,981 , 6,376,461 ,6,419,961 , 6,589,548, 6,613,358, 6,699,500 and 6,740,634, each of which is incorporated herein by reference. Such dosage forms can be used to provide slow or controlled-release of one or more active ingredients using, for example, hydropropylmethyl cellulose, other polymer matrices, gels, permeable membranes, osmotic systems, multilayer coatings, micraparticles, liposomes, microspheres, or a combination thereof to provide the desired release profile in varying proportions. Suitable controlled-release formulations known to those of ordinary skill in the art, including those described herein, can be readily selected for use with the active ingredients provided herein.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (e.g., adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

In certain embodiments, the agent may be administered using intravenous infusion, an implantable osmotic pump, a transdermal patch, liposomes, or other modes of administration. In one embodiment, a pump may be used. In another embodiment, polymeric materials can be used. In yet another embodiment, a controlled release system can be placed in proximity of the therapeutic target, i.e., thus requiring only a fraction of the systemic dose. In some embodiments, a controlled release device is introduced into a subject in proximity of the site of inappropriate immune activation or a tumor. The active ingredient can be dispersed in a solid inner matrix, e.g., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcoho! and cross-linked partially hydrolyzed polyvinyl acetate, that is surrounded by an outer polymeric membrane, e.g., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The active ingredient then diffuses through the outer polymeric membrane in a release rate controlling step. The percentage of active ingredient contained in such parenteral compositions is highly dependent on the specific nature thereof, as well as the needs of the subject.

### Targeted Formulations

The active ingredients provided herein, or pharmaceutically acceptable derivatives thereof, may also be formulated to be targeted to a particular tissue, receptor, or other area of the body of the subject to be treated. Many such targeting methods are well known to those of skill in the art. All such targeting methods are contemplated herein for use in the instant compositions. For non-limiting examples of targeting methods, see, e.g., U.S. Patent Nos. 6,316,652, 6,274,552, 6,271,359, 6,253,872, 6,139,865, 6,131 ,570, 6,120,751 , 6,071 ,495, 6,060,082, 6,048,736, 6,039,975, 6,004,534, 5,985,307, 5,972,366, 5,900,252, 5,840,674, 5,759,542 and 5,709,874.

In one embodiment, liposomal suspensions, including tissue- targeted liposomes, such as tumor-targeted liposomes, may also be suitable as pharmaceutically acceptable carriers. These may be prepared according to methods known to those skilled in the art. Briefly, liposomes such as multilamellar vesicles (MLVs) may be formed by drying down egg phosphatidyl choline and brain phosphatidyl serine (7:3 molar ratio) on the inside of a flask. A solution of a compound provided herein in phosphate buffered saline lacking divalent cations (PBS) is added and the flask shaken until the lipid film is dispersed. The resulting vesicles are washed to remove unencapsulated compound, pelleted by centrifugation, and then resuspended in PBS.

### Method of Distribution

In one aspect, the present invention provides a method of distributing an antifibrotic agent. The term "antifibrotic agent" denotes a chemical agent to treat or control disease, particularly fibrosis and/or fibrotic diseases or conditions.

In one embodiment, the method comprises distributing to a subject a predetermined amount of a first pharmaceutical composition in combination with a predetermined amount of a second pharmaceutical composition. The first pharmaceutical composition comprises CVC, or a salt, solvate, ester and/or prodrug thereof, and the second pharmaceutical composition comprises an additional therapeutic agent. In one specific embodiment, the first pharmaceutical composition comprises CVC or a salt, solvate, ester and/or prodrug thereof, and the second pharmaceutical composition comprises a FXR agonist. In another specific embodiment, the first pharmaceutical composition comprises CVC or a salt, solvate, ester and/or prodrug thereof, and the second pharmaceutical composition comprises a PPAR-α agonist. In one specific embodiment, the first pharmaceutical composition comprises CVC or a salt, solvate, ester and/or prodrug thereof, and the second pharmaceutical composition comprises a chemokine antagonist. The term "antifibrotic agent" denotes a chemical agent to treat or control disease, particularly fibrosis and/or fibrotic diseases or conditions. In another embodiment, the method comprises distributing a predetermined amount of a third pharmaceutical composition in combination with the first and second pharmaceutical compositions. The third pharmaceutical composition comprises an additional therapeutic agent as described hereinabove.

In another embodiment, the method comprises distributing to a subject a predetermined amount of a first pharmaceutical composition in combination with an instruction of administering the first pharmaceutical composition with a predetermined amount of a second pharmaceutical composition. In another embodiment, the method comprises distributing to a subject a predetermined amount of a first pharmaceutical composition in combination with an instruction of administering the first pharmaceutical composition with a predetermined amount of a second pharmaceutical composition and a predetermined amount of a third pharmaceutical composition. For example, the first pharmaceutical composition comprises CVC, or a salt, solvate, ester and/or prodrug thereof, and the second pharmaceutical composition comprises a FXR agonist; or alternatively, the first pharmaceutical composition comprises a FXR agonist, and the second pharmaceutical composition comprises CVC, or a salt, solvate, ester and/or prodrug thereof. The third pharmaceutical composition comprises a second additional therapeutic agent as described hereinabove. In one embodiment, the second additional therapeutic agent is another FXR agonist. In one embodiment, the second additional therapeutic agent is a PPAR-α agonist. In another example, the first pharmaceutical composition comprises CVC, or a salt, solvate, ester and/or prodrug thereof, and the second pharmaceutical composition comprises a PPAR-α agonist; or alternatively, the first pharmaceutical composition comprises a PPAR-α agonist, and the second pharmaceutical composition comprises CVC, or a salt, solvate, ester and/or prodrug thereof. The third pharmaceutical composition comprises a second additional therapeutic agent as described hereinabove. In one embodiment, the second additional therapeutic agent is another PPAR-α agonist. In one embodiment, the second additional therapeutic agent is a FXR agonist. In another example, the first pharmaceutical composition comprises CVC, or a salt, solvate, ester and/or prodrug thereof, and the second pharmaceutical composition comprises a chemokine antagonist; or alternatively, the first pharmaceutical composition comprises a chemokine antagonist, and the second pharmaceutical composition comprises CVC, or a salt, solvate, ester and/or prodrug thereof. The third pharmaceutical composition comprises a second additional therapeutic agent as described hereinabove.

The following Examples further illustrate the present invention in detail but are not to be construed to limit the scope thereof.

### EXAMPLES

### Example 1 - Cenicriviroc mesylate compositions

A series of cenicriviroc mesylate compositions that were identical except for the identity of the acid solubilizer were prepared by wet granulation in a Key 1L bowl granulator, followed by tray drying, sieving, mixing and compression into tablets on a Carver press. The composition of the formulations is shown in Table 2.

**Table 2**

| **Components** | **Unit Formula (mg/unit)** | | | |
|---|---|---|---|---|
| | **Ex.1a Citric Acid** | **Ex.1b Fumaric Acid** | **Ex. lc Maleic Acid** | **Ex.1d Sodium Bisulfate** |
| Cenicriviroc Mesylate | 28.45 | 28.45 | 28.45 | 28.45 |
| Mannitol | 7.88 | 7.88 | 7.88 | 7.88 |
| Hydroxypropyl Cellulose | 2.62 | 2.62 | 2.62 | 2.62 |
| Croscarmellose Sodium | 1.75 | 1.75 | 1.75 | 1.75 |
| Croscarmellose Sodium | 1.75 | 1.75 | 1.75 | 1.75 |
| Citric Acid | 43.75 | - | - | - |
| Fumaric Acid | - | 43.75 | - | - |
| Maleic Acid | - | - | 43.75 | - |
| Sodium Bisulfate | - | - | - | 43.75 |
| Silicon Dioxide | 0.43 | 0.43 | 0.43 | 0.43 |
| Magnesium Stearate | 0.88 | 0.88 | 0.88 | 0.88 |
| Total | 87.5 | 87.5 | 87.5 | 87.5 |

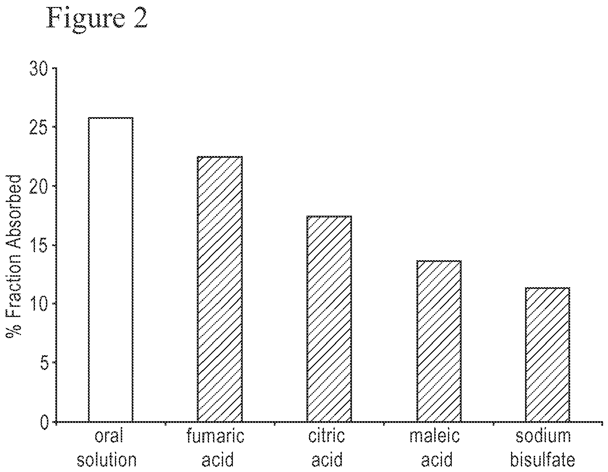

The tablets were administered to beagle dogs. An oral solution was also administered as a control. The absolute bioavailabilities of the formulations and of the oral solution were determined, and are shown in Figure 2. The result shows that the cenicriviroc mesylate with fumaric acid has a significantly higher bioavailability than any of the other solubilizers tested.

### Example 2: Cenicriviroc mesylate compositions

Cenicriviroc mesylate, fumaric acid, microcrystalline cellulose, cross-linked sodium carboxymethyl cellulose, and magnesium stearate were admixed, dry granulated, milled, blended with extragranular microcrystalline cellulose, cross-linked sodium carboxymethyl cellulose, and magnesium stearate and compressed into tablets having a hardness greater than 10 kP and friability less than 0.8% w/w. The resulting tablets had the composition shown in Table 3.

**Table 3.**

| **Components** | **Unit Formula (mg/unit)** | | | | |
|---|---|---|---|---|---|
| | **Ex. 2a** | **Ex. 2b** | **Ex. 2c** | **Ex. 2d** | **Ex. 2e** |
| Cenicriviroc Mesylate | 170.69^{a} | 170.69^{a} | 170.69^{a} | 170.69^{a} | 170.69^{a} |
| Fumaric Acid | 160.00 | 160.00 | 160.00^{b} | 160.00 | 80.00 |
| Microcrystalline Cellulose | 252.68 | 272.18 | 272.18 | 272.18 | 66.35 |
| Crospovidone | - | - | - | 19.50 | - |
| Croscarmellose Sodium | 58.50 | 39.00 | 39.00 | 19.50 | 20.70 |
| Magnesium Stearate | 8.13 | 8.13 | 8.13 | 8.13 | 2.55 |
| Total | 650.0 | 650.0 | 650.0 | 650.0 | 340.0 |

| | | | | | |
|---|---|---|---|---|---|
| a. Equivalent to 150 mg cenicriviroc freebase. b. Added in the extragranular portion of the powder blend. | | | | | |

By way of illustration, the concentration percentage and mass per tablet of the components in Example 2b (i.e., Ex. 2b)are given in Table 4.

**Table 4**

| **Component** | **Concentration (% w/w)** | **Mass (mg) per tablet** |
|---|---|---|
| Cenicriviroc mesylate | 26.26 | 170.69^{a} |
| Fumaric acid | 24.62 | 160.00 |
| Microcrystalline cellulose | 41.87 | 272.18 |
| Cross-linked sodium carboxymethyl cellulose | 6.00 | 39.00 |
| Magnesium stearate | 1.25 | 8.13 |
| Total | 100.0 | 650.0 |

| | | |
|---|---|---|
| ^{a} equivalent to 150 mg cenicriviroc free base | | |

### Example 3: Cenicriviroc mesylate compositions

Cenicriviroc mesylate, microcrystalline cellulose, cross-linked sodium carboxymethyl cellulose, and magnesium stearate were admixed, dry granulated, dried, milled, blended with extragranular microcrystalline cellulose, cross-linked sodium carboxymethyl cellulose, fumaric acid, colloidal silicon dioxide, and magnesium stearate and compressed into tablets having a hardness greater than 10 kP and friability less than 0.8% w/w. The resulting tablets had the composition shown in Table 5.

**Table 5**

| **Component** | **Concentration (% w/w)** | **Mass (mg) per tablet** |
|---|---|---|
| Cenicriviroc mesylate | 26.26 | 28.45^{a} |
| Fumaric acid | 24.62 | 26.67 |
| Microcrystalline cellulose | 41.87 | 45.36 |
| Cross-linked sodium carboxymethyl cellulose | 6.00 | 39.00 |
| Magnesium stearate | 1.25 | 1.35 |
| Total | 100.0 | 108.3 |

| | | |
|---|---|---|
| ^{a} equivalent to 25 mg cenicriviroc free base | | |

Notably, the formulation of Table 5 has the same ratio of components as that of Table 3b, and differs only in the total amount of the components that are used for each tablet. Thus, Table 4 shows tablets with 150 mg cenicriviroc (based on free base), whereas Table CC-1 shows tablets with 25 mg cenicriviroc (based on free base) with the same ratio of components as the 150 mg tablets of Example 2b, shown in Table 4.

### Example 4 - Reference

The citric acid based formulation of Table 6 was prepared as follows. Cenicriviroc, hydroxypropyl cellulose, mannitol, and cross-linked sodium carboxymethyl cellulose were admixed, wet granulated, dried, milled, and blended with microcrystalline cellulose, cross-linked sodium carboxymethyl cellulose, citric acid, colloidal silicon dioxide, talc, and magnesium stearate. The resulting blend was compressed into tablets having a hardness greater than 10 kP and friability less than 0.8% w/w. The tablets were coated with hydroxypropyl methylcellulose, polyethylene glycol 8000, titanium dioxide, and yellow iron oxide. The coated tablets thus produced were substantially identical to those disclosed in U.S. Patent Application Publication No. 2008/031942 (see, e.g., Table 3).

**Table 6**

| **Component** | **mg/tablet** | **%w/w** |
|---|---|---|
| Cenicriviroc mesylate | 28.91 | 4.68 |
| Mannitol | 341.09 | 56.85 |
| Microcrystalline cellulose | 80.00 | 12.94 |
| Colloidal silicon dioxide | 12.00 | 2.00 |
| Citric acid anhydrous | 75.00 | 12.14 |
| Hydroxypropyl cellulose | 12.00 | 1.94 |
| Cross-linked sodium carboxymethyl cellulose | 30.00 | 4.85 |
| Talc | 12.00 | 1.94 |
| Magnesium stearate | 9.00 | 1.46 |
| Hydroxypropyl methylcellulose | 11.71 | 1.89 |
| Polyethylene glycol 8000 | 2.69 | 0.44 |
| Titanium dioxide | 3.03 | 0.49 |
| Yellow iron oxide | 0.57 | 0.09 |

### Example 5- Reference

Cenicriviroc and hypromellose acetate succinate were dissolved in methanol and spray dried into a fine powder containing 25% cenicriviroc by weight (based on the weight of cenicriviroc free base). The powder was admixed with colloidal silicon dioxide, microcrystalline cellulose, mannitol, sodium lauryl sulfate, cross-linked sodium carboxymethyl cellulose, and magnesium stearate. The admixture was compressed into tablets having a hardness greater than 10 kP and friability less than 0.8% w/w. The final composition of the tablets is shown in Table 7.

**Table 7**

| **Component** | **Weight %** | **Mass (mg)** |
|---|---|---|
| Cenicriviroc (as mesylate salt) | 8.33 | 50.00 |
| Hypromellose acetate succinate | 25.00 | 150.00 |
| Sodium lauryl sulfate | 2.00 | 12.00 |
| Cross-linked sodium carboxymethyl cellulose | 6.00 | 36.00 |
| Microcrystalline cellulose | 27.83 | 167.00 |
| Mannitol | 27.83 | 167.00 |
| Colloidal silicon dioxide | 1.00 | 6.00 |
| Magnesium stearate | 2.00 | 12.00 |
| Total | 100.0 | 600.0 |

### Example 6: Bioavailibility of CVC formulation

The absolute bioavailability of the tablets of Example 3 in beagle dogs was compared to that of the tablets of Examples 4 and 5, as well as to both an oral solution of cenicriviroc mesylate and a gelatin capsule containing cenicriviroc mesylate powder. The results are shown in Table 8.

**Table 8**

| **Component** | **Absolute bioavailability(%)** |
|---|---|
| Oral Solution | 25.8 |
| Powder in capsule | 6.4 |
| Example 3 | 26.6 |
| Example 4 | 21.1 |
| Example 5 | 12.4 |

This example demonstrates that the bioavailability of cenicriviroc in dry granulated tablets with fumaric acid (Ex. 3) is substantially similar to that of an oral solution, and is significantly higher than the bioavailability of cenicriviroc in wet granulated tablets with fumaric (Ex. 1b) or citric acid (Ex. 4), and over double that of cenicriviroc in tablets with amorphous cenicriviroc in a spray dried dispersion with HPMC-AS (Ex. 5). These results are surprising, because there was no reason to suspect that dry granulation of crystalline API provides a significant increase in bioavailability over wet granulation and amorphous spray dried dispersions. This is especially so because amorphous spray dried dispersions are frequently used to increase the bioavailability of poorly water soluble drugs. These results are also surprising because fumaric acid has a slower dissolution time than citric acid and was used at a lower mass ratio of acid relative to CVC API (3:1 for citric acid: API versus 1.06:1 fumaric acid : API). Hence it was therefore surprising that fumaric acid proved to be a more effective solubilizer than citric acid for CVC.

### Example 7: Accelerated stability of CVC formulation

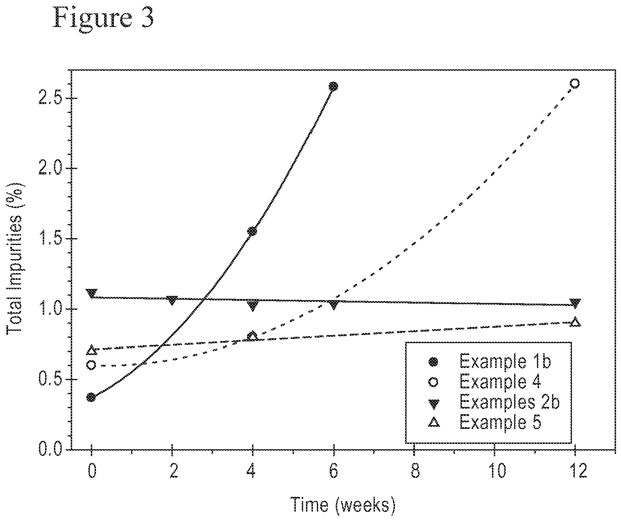

The accelerated stability of the tablets of Example 2b was compared to that of the tablets of Examples 1b, 4, and 5 via exposure to an environment of 75% relative humidity at 40° C. All tablets were packaged with a desiccant during the study. As shown in Figure 3, the tablets of Examples 2b are surprisingly much more stable than the other wet granulated tablets, and similarly stable as the spray dried dispersion tablets. This difference in stability between the tablets of Examples 2b and Example 4 is particularly surprising since the only significant difference between the two is the method of making the formulations (dry granulation vs. wet granulation). These results are also surprising, because it was not previously known that the method of granulation could have an effect on both cenicriviroc bioavailability and stability.

### Example 8: Stability of CVC formulation

The stability of the tablets of Examples 2 and 3 was tested by exposing the tablets to an environment of 75% relative humidity at 40° C for six weeks. All tablets were packaged with a desiccant during the study. The results are shown in Table 9, which shows that the tablets are very stable under these conditions.

**Table 9**

| **Time (Weeks)** | **Water content (%)** | **Strength (%)** | **Total Impurities (%)** |
|---|---|---|---|
| 0 | 1.5 | 99.1 | 1.2 |
| 2 | 1.4 | 99.2 | 1.1 |
| 4 | 1.4 | 98.0 | 1.0 |
| 6 | 1.4 | 98.6 | 1.0 |

### Example 9: Stability of CVC formulations

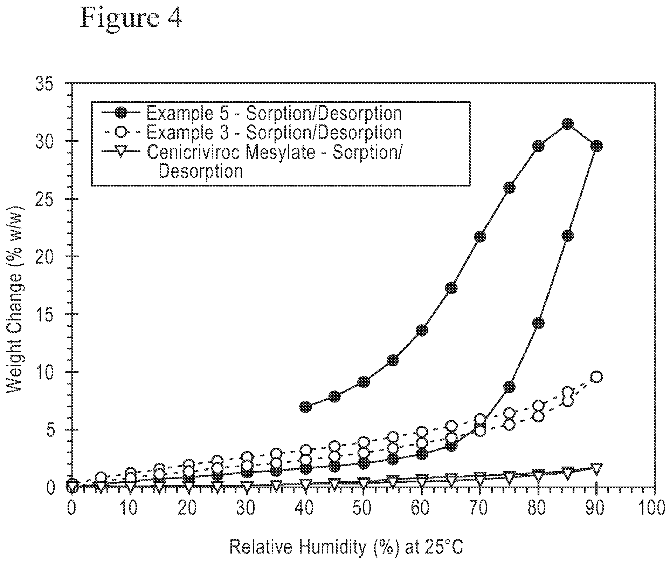

Dynamic vapor sorption isotherms at 25° C correlate to the stability of the tablets of Examples 3 and 4 with that of cenicriviroc mesylate. Sorption was performed from 0% relative humidity to 90% relative humidity at 5% intervals. At each interval, each sample was equilibrated for no less than 10 minutes and no longer than 30 minutes. Equilibration was stopped when the rate of mass increase was no more than 0.03% w/w per minute or after 30 minutes, whichever was shorter. The result, which appears in Figure 4, shows that tablets of Example 2b are significantly more stable than those of Example 4. This result is consistent with Example 3 being significantly less hygroscopic than Example 4. The increased hygroscopicity of Example 4, in comparison to Examples 2b, can be associated with a higher mobile water content which can in turn cause partial gelation and subsequent decreased stability of Example 4.

### Example 10: Anti-fibrotic And Anti-Inflammatory Activity Of The Dual CCR2 And CCR5 Antagonist Cenicriviroc In A Mouse Model Of NASH

**Background:** Non-alcoholic steatohepatitis (NASH) is characterized by fat accumulation, chronic inflammation (including pro-inflammatory monocytes and macrophages) and when fibrosis is present, it can lead to cirrhosis or hepatocellular carcinoma. There are currently no approved therapies for NASH. Evidence suggests that C-C chemokine receptor (CCR) type 2 and its main ligand, monocyte chemotactic protein-1, contribute to pro-inflammatory monocyte recruitment in the liver. Cenicriviroc (CVC) is an oral, potent, dual CCR2/CCR5 antagonist that showed favorable safety and tolerability in a 48-week Phase 2b study in 143 HIV-1-infected adults (NCT01338883). CVC was evaluated in a mouse model of diet-induced NASH that leads to hepatocellular carcinoma; data from the first, fibrotic stage of the model are presented.

**Methods:** NASH was induced in male mice by a single injection of 200µg streptozotocin 2 days after birth (causing impaired glucose control), followed by a high fat diet from 4 weeks of age. From 6 to 9 weeks of age, 3 groups of animals (n=6/group) were administered CVC doses of 0 (vehicle), 20 (low dose) or 100 (high dose) mg/kg/day, via twice daily oral gavage. Animals were sacrificed at 9 weeks of age, and biochemical, gene expression, and histologic evaluations of the liver were conducted.

**Results:** CVC treatment had no effect on body or liver weight, whole blood glucose, or liver triglycerides. Mean (±SD) alanine aminotransferase levels were significantly decreased in both CVC treatment groups compared to control (58±12, 51±13 and 133±80 U/L for low dose, high dose and vehicle, respectively; p<0.05) and liver hydroxyproline tended to decrease in treated groups. By real-time RT-PCR, collagen type 1 mRNA in whole liver lysates decreased by 27-37% with CVC treatment. The percentage of fibrosis area (by Sirius red staining) was significantly decreased by CVC treatment relative to control (p<0.01): 0.66% ± 0.16, 0.64% ± 0.19 and 1.10% ± 0.31 for 20 mg/kg/day, 100 mg/kg/day and control, respectively, when perivascular space was included; 0.29% ± 0.14, 0.20% ± 0.06, and 0.61%± 0.23, respectively, when perivascular space was subtracted. Importantly, the histologic non-alcoholic fatty liver disease activity score (score is 0 for untreated mice in this model) was significantly decreased with CVC treatment (4.0±0.6, 3.7±0.8 and 5.3±0.5 for low dose, high dose and vehicle, respectively; p<0.05), primarily due to reduced inflammation and ballooning scores. As previously shown in humans, a CVC dose-related compensatory increase in plasma monocyte chemotactic protein-1 levels was observed in mice (1.1- and 1.5-fold increase for low and high dose, respectively), consistent with antagonism of CCR2.

**Conclusions:** These data suggest that CVC, an investigational agent currently in human trials for HIV-1, has anti-fibrotic and anti-inflammatory activity in a mouse model of NASH, warranting clinical investigation. These findings provide further evidence that disrupting the CCR2/monocyte chemotactic protein-1 axis may be a novel treatment approach for NASH.

### Example 11: Significant Anti-fibrotic Activity Of Cenicriviroc, A Dual CCR2/CCR5 Antagonist, In A Rat Model Of Thioacetamide-Induced Liver Fibrosis And Cirrhosis

**Background:** C-C chemokine receptor (CCR) types 2 and 5 are expressed on pro-inflammatory monocytes and macrophages, Kupffer cells and hepatic stellate cells (HSCs), which contribute to inflammation and fibrogenesis in the liver. Cenicriviroc (CVC; novel, potent, oral, dual CCR2/CCR5 antagonist) had favorable safety/tolerability in a 48-week Phase 2b study in 143 HIV-1-infected adults (NCT01338883). This study evaluates the in vivo anti-fibrotic effect of CVC, and timing of treatment intervention relative to disease onset, in rats with emerging hepatic fibrosis due to thioacetamide (TAA)-induced injury.

**Methods:** Fibrosis was induced in male Sprague-Dawley rats by intraperitoneal administration of TAA 150 mg/kg 3 times/week for 8 weeks. Rats (n=4-8/group) received CVC 30 mg/kg/day (a), CVC 100 mg/kg/day (b) or vehicle control (c), concurrently with TAA for the first 8 weeks (Group 1; early intervention), during Weeks 4-8 (Group 2; emerging fibrosis) or during Weeks 8-12 following completion of TAA administration (Group 3; cirrhosis reversal). Biochemical, gene expression and histologic evaluations of the liver were conducted.

**Results:** When started concurrently with TAA (Group 1), CVC at 30 mg (Group 1a) and 100 mg (Group 1b) significantly reduced fibrosis (by 49% and 38%, respectively; p<0.001), as assessed by collagen morphometry. Protein levels for collagen type 1 were reduced by 30% and 12% for Groups 1a and 1b, respectively, while α-SMA was reduced by 17% and 22%, respectively. When treatment started 4 weeks after TAA-induced injury (Group 2), a statistically significant anti-fibrotic effect was observed for CVC 30 mg (Group 2a, 36% reduction in collagen; p<0.001), but not for CVC 100 mg (Group 2b).. When treatment was started at Week 8 (cirrhosis present) and continued for 4 weeks (Group 3), there was no significant effect of CVC on fibrogenic gene expression or fibrosis.

**Conclusions:** CVC is a potent anti-fibrotic agent in non-cirrhotic hepatic fibrosis due to TAA. The drug was effective in early intervention (Group 1) and in emerging fibrosis (Group 2a), but not when cirrhosis was already established (Group 3).

### Example 12: Anti-fibrotic Activity of Dual CCR5/CCR2 Antagonist Cenicriviroc in a Mouse Model of Renal Fibrosis

**Background:** Cenicriviroc (CVC) is a novel, oral, once-daily, dual CCR5/CCR2 antagonist that has completed Phase 2b HIV development (Study 202; NCT01338883). CVC has a favorable safety profile with 555 subjects having been treated with at least one dose, including 115 HIV-1-infected adults treated with CVC over a 48-week duration. Recently, CVC demonstrated significant anti-fibrotic activity in a mouse model of diet-induced, non-alcoholic steatohepatitis (NASH) and a rat model of thioacetamide-induced fibrosis. Here, we evaluated CVC in a well-established mouse model of renal fibrosis induced by unilateral ureter occlusion (UUO).

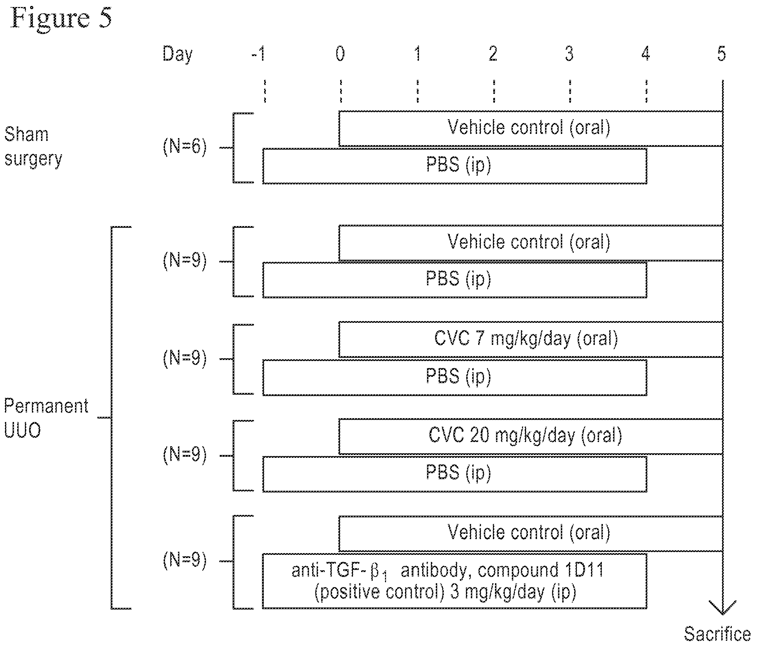

**Methodology:** Test animals were allocated to weight-matched treatment groups on the day prior to the surgical procedure (Day -1). Male CD-1 mice (N=51; age, 7-8 weeks) underwent either sham surgery or total ligation of the right ureter, i.e. UUO, via aseptic laparotomy (Figure 5). From Days 0 to 5: mice undergoing sham surgery received vehicle control (0.5% methylcellulose + 1% Tween-80) via twice-daily oral gavage; mice with permanent UUO received either vehicle control, CVC 7 mg/kg/day or CVC 20 mg/kg/day via twice-daily oral gavage. Another group received the anti-transforming growth factor TGF-β1 antibody, compound 1D11 (positive control) at 3 mg/kg/day from Days -1 to 4, injected intraperitoneally once daily, and vehicle control from Days 0 to 5. A CVC 100 mg/kg/day group (N=9) was initially included in the study but was terminated early due to moribundity (no analyses were conducted because no animal reached Day 5). CVC doses up to 2000 mg/kg/day were well tolerated in mouse toxicity studies that did not involve surgical procedures. On Day 5, animals were anaesthetised, blood and tissues were collected prior to sacrifice.

**Study endpoints:** Study endpoints included: a) body and kidney weights; b) fibrosis in obstructed kidney evaluated via histological quantitative image analysis of picrosirius red staining (ten images/depth/kidney obtained and assessed in a blinded fashion using light microscopy [at 200x] to enable sampling of 60-70% of the renal cortical area) and quantified by a composite Collagen Volume Fraction (CVF [% total area imaged]) score expressed as the average positive stain across three anatomically distinct (200-250 µM apart) tissue sections, or depths, from the obstructed kidney; c) hydroxyproline content of frozen renal cortical tissue biopsies as assessed by biochemical analyses; d) mRNA expression of profibrotic and inflammatory biomarkers (including MCP-1, Collagen 1a1, Collagen 3a1, TGF-β1, Fibronectin-1, α-smooth muscle actin (a-SMA) and connective tissue growth factor-1 (CTGF-1); assessed via Luminex® (Life Technologies™, Carlsbad, CA, USA) assay with relative expression normalised to HPRT (hypoxanthine phosphoribosyltransferase).

**Statistical analysis**: Data are expressed as mean ± standard error of mean (SEM). Statistical analyses were performed using GraphPad Prism® (GraphPad Software, Inc., San Diego, CA, USA). Treatment differences between sham-surgery+vehiclecontrol and UUO+vehicle-control groups, and between UUO+vehicle-control and UUO+compound-1D11 (positive control) groups, were analysed by unpaired t-Test. Treatment differences between UUO+vehicle-control and CVC-dose groups were analysed by one-way ANOVA (analysis of variance) with Dunnett's test (post-hoc).

**Methods**: CVC demonstrated significant antifibrotic effects, as defined by reductions in Collagen Volume Fraction or CVF (% area stained positively for collagen in histological obstructed-kidney sections), in a well-established mouse UUO model of renal fibrosis. Trends were observed for decreases in Collagen 1a1, Collagen 3a1, TGF-β1 and Fibronectin-1 mRNA expression in the obstructed kidney, but these did not achieve statistical significance. Taken together, CVC's mode of action, antifibrotic activity in animal models (kidney and liver), and extensive safety database support further evaluation in fibrotic diseases. A proof-of-concept study in non-HIV-infected patients with NASH and liver fibrosis is planned. Phase III trials in HIV-1-infected patients are also planned to evaluate a fixed-dose combination of CVC/lamivudine (3TC) as a novel 'backbone' versus tenofovir disoproxil fumarate/emtricitabine (TDF/FTC) when co-administered with guideline-preferred third agents.

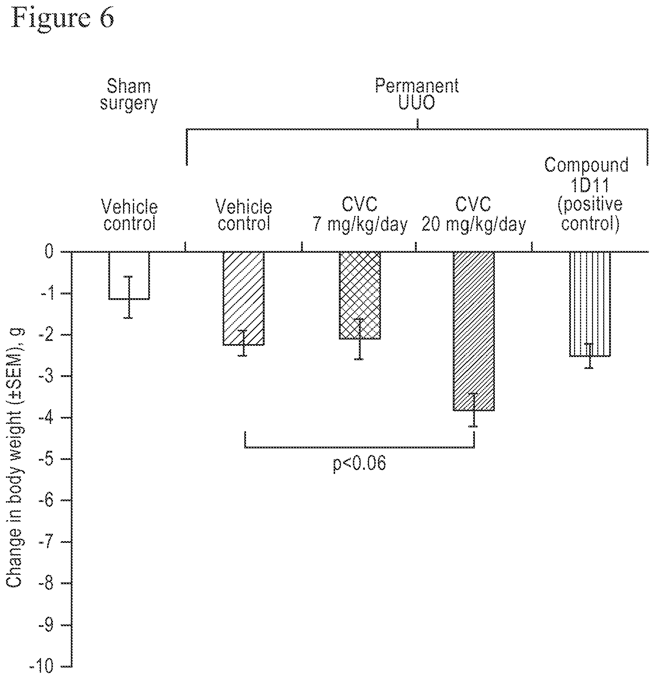

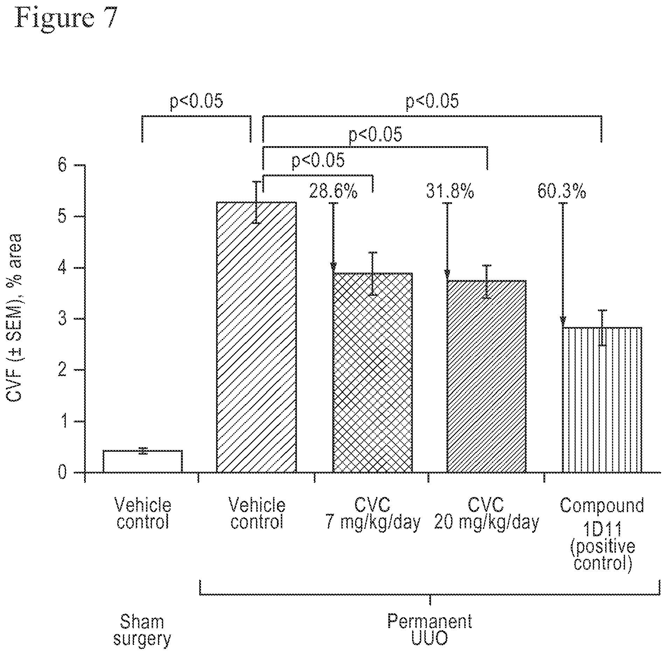

**Results**: Body weight and obstructed kidney weight: CVC 7 mg/kg/day and compound 1D11 (positive control) had no effect on body weight, whereas CVC 20 mg/kg/day led to a modest, but significant, decrease (5%) in body weight, relative to that of the UUO+vehicle-control group at Day 5 (p<0.05) (Figure 6; change in body weight shown in grams [g]). No significant treatment effects (CVC or compound 1D11 [positive control]) were observed on obstructed or contralateral kidney weight or kidney weight index versus the UUO+vehicle-control group (data not shown). Histology: The composite measure of CVF (% area averaged across three depths [+SEM]) was significantly higher in the UUO+vehicle-control group compared with that in the sham-surgery group (11.4+1.0-fold; p<0.05) (Figure 7). CVC 7 and 20 mg/kg/day and compound 1D11 (positive control) significantly attenuated UUO-induced increases in the composite measure of CVF (averaged across three depths [+SEM]) relative to that of the UUO+vehicle-control group (28.6±8.8%, 31.8±6.8% and 50.3±7.3% reduction, respectively; p<0.05).

**Hydroxyproline content:** Hydroxyproline content (% of protein) in obstructed kidneys from the UUO+vehicle-control group increased significantly relative to the sham-surgery group (0.72% vs 0.27%; p<0.05) (data not shown). Neither dose of CVC tested affected UUO-induced increases in obstructed kidney hydroxyproline content relative to the UUO+vehicle-control group; however, the compound 1D11 (positive control) group had significantly lower levels (0.55% vs 0.72%; p<0.05) (data not shown).

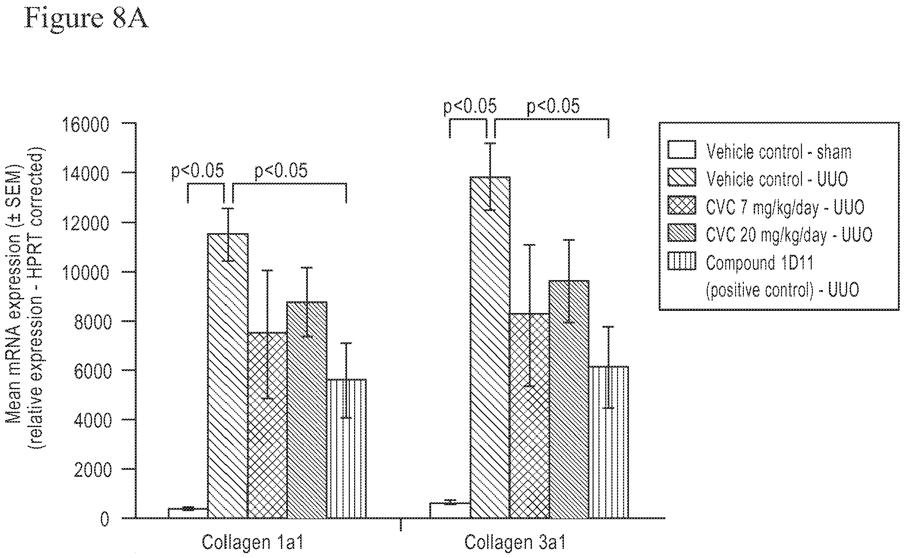

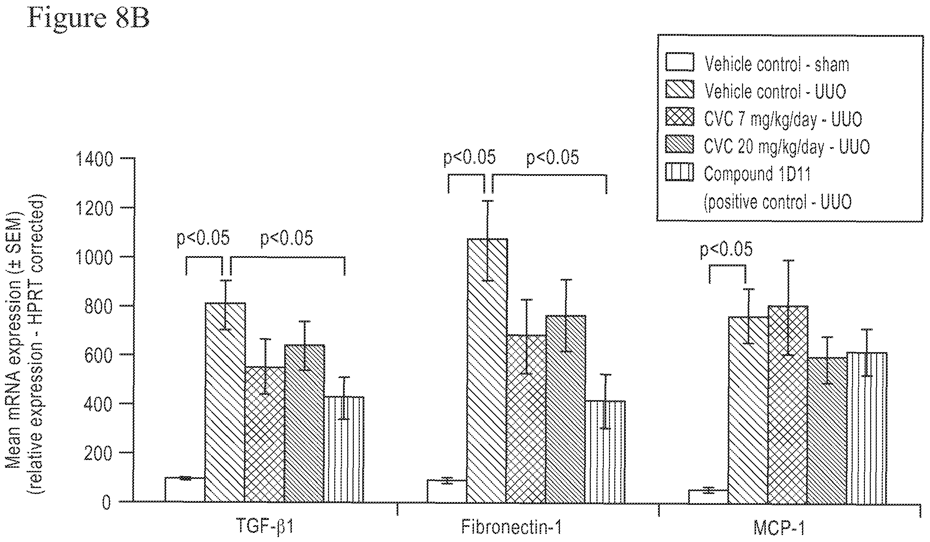

**Profibrotic and inflammatory biomarker mRNA expression:** For each of the biomarkers evaluated (MCP-1, Collagen 1a1, Collagen 3a1, TGF-β1, Fibronectin-1, α-SMA and CTGF-1), expression of mRNA in the UUO+vehicle-control group increased significantly compared with that in the shamsurgery group (p<0.05) (Figure 8). CVC 7 and 20 mg/kg/day attenuated UUO-induced increases in Collagen 1a1, Collagen 3a1, TGF-β1 and Fibronectin-1 mRNA expression. However, these reductions, compared with the UUO+vehicle-control group, did not reach statistical significance. Compound 1D11 (positive control) significantly reduced UUO-induced increases in mRNA expression of Collagen 1a1, Collagen 3a1, TGF-β1 and Fibronectin-1 relative to the UUO+vehicle-control group (p<0.05). CVC 7 and 20 mg/kg/day and compound 1D11 (positive control) did not have significant effects on UUO-induced increases in obstructed kidney cortical MCP-1, α-SMA and CTGF-1 mRNA expression, compared with the UUO+vehicle-control group (data not shown for α-SMA and CTGF-1 mRNA).

**Conclusions:** CVC demonstrated significant antifibrotic effects, as defined by reductions in Collagen Volume Fraction or CVF (% area stained positively for collagen in histological obstructed-kidney sections), in a well-established mouse UUO model of renal fibrosis. Trends were observed for decreases in Collagen 1a1, Collagen 3a1, TGF-β1 and Fibronectin-1 mRNA expression in the obstructed kidney, but these did not achieve statistical significance. Taken together, CVC's mode of action, antifibrotic activity in animal models (kidney and liver), and extensive safety database support further evaluation in fibrotic diseases. A proof-of-concept study in non-HIV-infected patients with NASH and liver fibrosis is planned. Phase III trials in HIV-1-infected patients are also planned to evaluate a fixed-dose combination of CVC/lamivudine (3TC) as a novel 'backbone' versus tenofovir disoproxil fumarate/emtricitabine (TDF/FTC) when co-administered with guideline-preferred third agents.

### Example 13: Improvements in APRI and FIB-4 fibrosis scores correlate with decreases in sCD14 in HIV-1 infected adults receiving cenicriviroc over 48 weeks

**Background and aims:** Cenicriviroc (CVC), a novel, oral, once-daily CCR2/CCR5 antagonist, has demonstrated favorable safety and anti-HIV activity in clinical trials. CVC demonstrated antifibrotic activity in two animal models of liver disease. Post-hoc analyses were conducted on APRI and FIB-4 scores in Study 202 (NCT01338883).

**Methods:** 143 adults with CCR5 tropic HIV-1, BMI≤35kg/m2 and no apparent liver disease (ie, ALT/AST Grade≤2, total bilirubin≤ULN, no HBV, HCV, active or chronic liver disease, or cirrhosis) were randomized 4:1 to CVC or efavirenz (EFV). APRI and FIB-4 scores were calculated. Change in score category from baseline (BL) to Weeks 24 and 48 was assessed in patients with non-missing data. Correlations between changes from BL in APRI and FIB-4 scores, and MCP-1 (CCR2 ligand) and sCD14 (inflammatory biomarker) levels were evaluated.

**Results:** At BL, more patients on CVC than EFV had APRI≥0.5 and FIB-4>1.45; proportion of CVC patients above these thresholds decreased at Weeks 24 and 48 (Table 10). Significant correlations were observed at Week 24 between changes in APRI score and MCP-1 levels (p=0.014), and between FIB-4 score and sCD14 levels (p=0.011), and at Week 48, between changes in APRI (p=0.028) and FIB-4 scores (p=0.007) and sCD14 levels. (Table 10).

**Table 10**

| | **Fibrosis index** | **CVC** | | | **EFV** | | |
|---|---|---|---|---|---|---|---|
| | | **Baseline (n=113)** | **Week 24 (n=92)** | **Week 48 (n=80)** | **Baseline (n=28)** | **Week 24 (n=20)** | **Week 48 (n=17)** |
| **APRI category** | <0.5 | 84% | 93% | 91% | 96% | 100% | 100% |
| | 0.5-1.5 | 14% | 7% | 8% | 4% | - | - |
| | >1.5 | 2% | - | 1% | - | - | - |
| | *Decreased 1 category from baseline* | N/A | 14% | 10% | N/A | 5% | 6% |
| **FIB-4 category** | <1.45 | 82% | 93% | 94% | 100% | 100% | 94% |
| | 1.45-3.25 | 17% | 7% | 5% | - | - | 6% |
| | >3.25 | 1% | - | 1% | - | - | - |
| | *Decreased 1 category* | N/A | 13% | 14% | N/A | - | - |
| | *from baseline* | | | | | | |

**Conclusions:** In this population with no apparent liver disease, CVC treatment was associated with improvements in APRI and FIB-4 scores, and correlations were observed between changes in APRI and FIB-4 scores and sCD14 levels at Week 48. Proven CCR2/CCR5 antagonism, antifibrotic effects in animal models and extensive clinical safety data all support clinical studies of CVC in liver fibrosis.

### Example 14: In Vivo Efficacy Study of Cenicriviroc in STAM Model of Non-alcoholic Steatohepatitis

This *in vivo* efficacy study was performed to examine the effects of Cenicriviroc in the STAM™ mouse model of Non-alcoholic Steatohepatitis.

### Materials and Methods

### Experimental Design and Treatment

### Study groups

Group 1-Vehicle: Eighteen NASH mice were orally administered vehicle at a volume of 10 mL/kg twice daily (9:00 and 19:00) from 6 weeks of age.
Group 2-Cenicriviroc 20 mg/kg (CVC-low): Eighteen NASH mice were orally administered vehicle supplemented with Cenicriviroc at a dose of 10 mg/kg twice daily (20 mg/kg/day) (9:00 and 19:00) from 6 weeks of age.
Group 3 - Cenicriviroc 100 mg/kg (CVC-high): Eighteen NASH mice were orally administered vehicle supplemented with Cenicriviroc at a dose of 50 mg/kg twice daily (100 mg/kg/day) (9:00 and 19:00) from 6 weeks of age.

Table 11 summarizes the treatment schedule:

**Table 11**

| Group | No. mice | Mice | Test substance | Dose (mg/kg) | Volume (mL/kg) | Regimen | Sacrifice (wks) |
|---|---|---|---|---|---|---|---|
| 1 | 18 | STAM | Vehicle | - | 10 | Oral, twice daily, 6-9 wks, 6 -18wks | 9 and 18 |
| 2 | 18 | STAM | CVC-low | 20 | 10 | Oral, twice daily,6-9 wks, 6 -18wks | 9 and 18 |
| 3 | 18 | STAM | CVC-high | 100 | 10 | Oral, twice daily,6-9 wks, 6 -18wks | 9 and 18 |

### Results

### Part 1: Study for Assessing the Anti-NASH/Fibrosis Effects of CVC

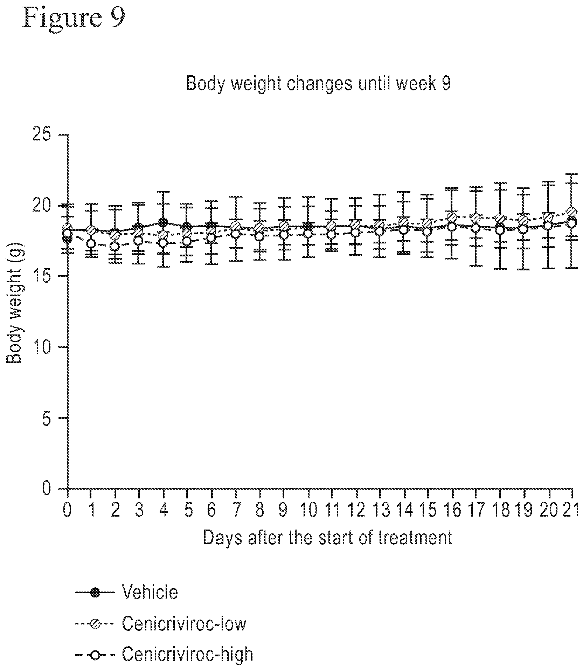

Body weight changes and general condition until Week 9 (Figure 9)

Body weight gradually increased during the treatment period. There were no significant differences in mean body weight between the Vehicle group and either the CVC-low or the CVC-high groups during the treatment period. None of the animals in the present study showed deterioration in general condition throughout the treatment period.

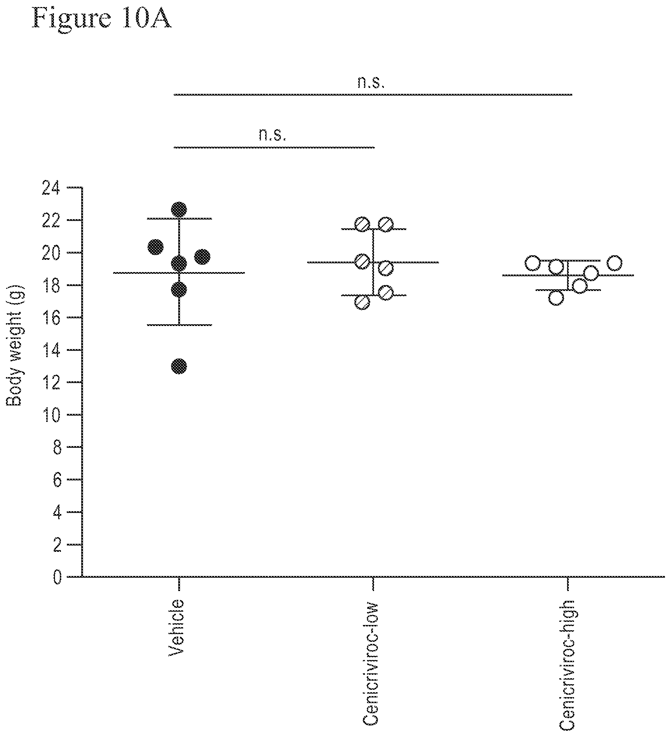

Body weight at the day of sacrifice at Week 9 (Figure 10A and Table 12)

There were no significant differences in mean body weight between the Vehicle group and either the CVC-low or the CVC-high groups (Vehicle: 18.9 ± 3.3 g, CVC-low: 19.5 ± 2.0 g, CVC-high: 18.7 ± 0.9 g).

**Table 12: Body Weight and Liver Weight at Week 9**

| **Parameter (Mean ± SD)** | **Vehicle (n=6)** | **Cenicriviroc-low (n=6)** | **Cenicriviroc-high (n=6)** |
|---|---|---|---|
| Body weight (g) | 18.9 ± 3.3 | 19.5 ± 2.0 | 18.7 ± 0.9 |
| Liver weight (mg) | 1270 ± 326 | 1334 ± 99 | 1307 ± 119 |
| Liver-to-body weight ratio (%) | 6.6 ± 0.8 | 6.9 ± 1.0 | 7.0 ± 0.8 |

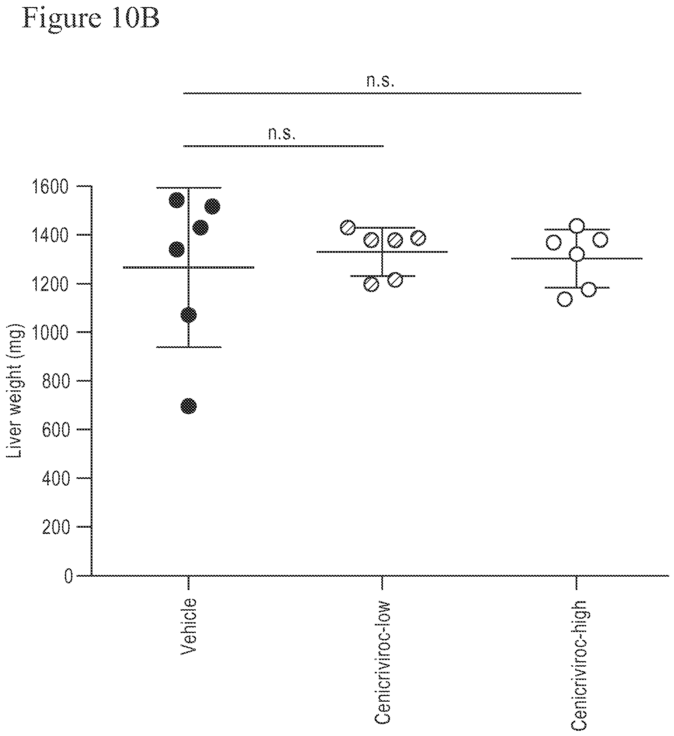

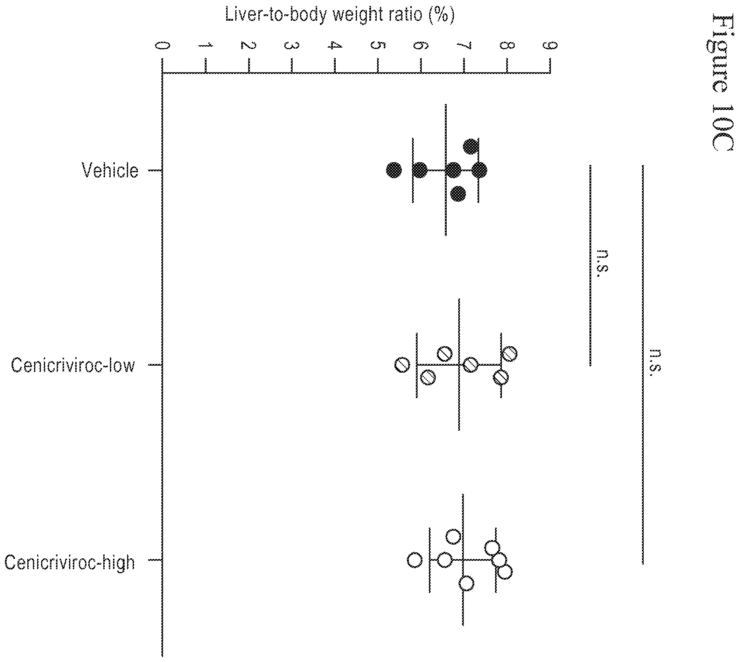

Liver weight and liver-to-body weight ratio at week 9 (Figures 10 B & C and Table 12)

There were no significant differences in mean liver weight between the Vehicle group and either the CVC-low or the CVC-high groups (Vehicle: 1270 ± 326 mg, CVC-low: 1334 ± 99 mg, CVC-high: 1307 ± 119 mg).

There were no significant differences in mean liver-to-body weight ratio between the Vehicle group and either the CVC-low or the CVC-high groups (Vehicle: 6.6 ± 0.8%, CVC-low: 6.9 ±1.0%, CVC-high: 7.0 ± 0.8%).

### Whole blood and biochemistry at week 9

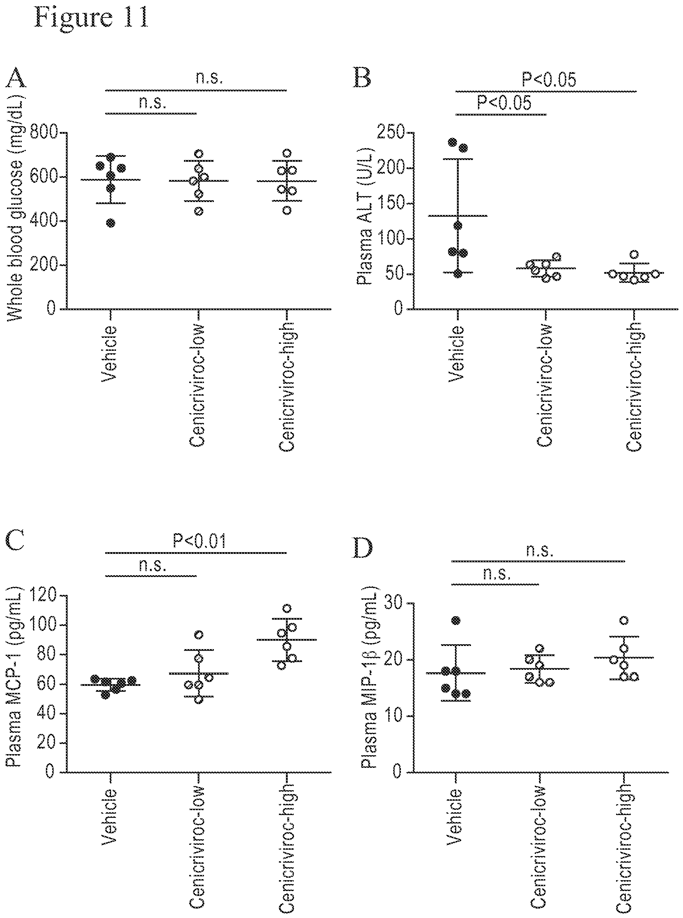

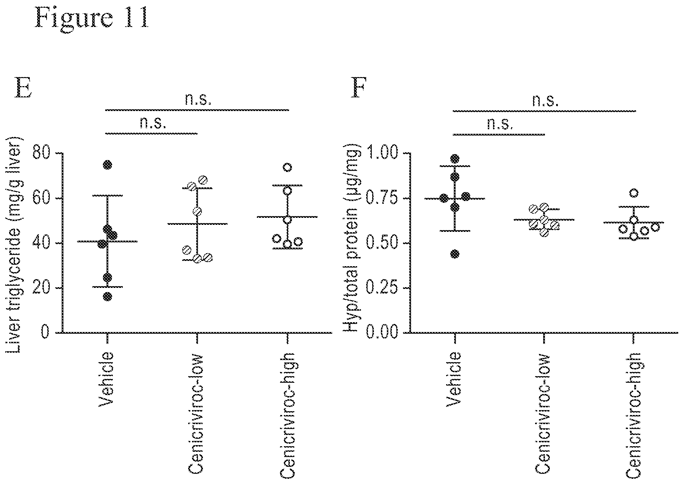

Whole blood glucose data are shown in Figures 11A-D and Table 13.

There were no significant differences in blood glucose levels between the Vehicle group and either the CVC-low or the CVC-high groups (Vehicle: 590 ± 108 mg/dL, CVC-low: 585 ± 91 mg/dL, CVC-high: 585 ± 91 mg/dL). 4.4.2. Plasma ALT (Figure 11B, Table 14). The CVC-low and the CVC-high groups showed significant decreased in plasma ALT levels compared with Vehicle group (Vehicle: 133 ± 80 U/L, CVC-low: 58 ± 12 U/L, CVC-high: 52 ± 13 U/L).

**Table 13: Blood and Liver Biochemistry at Week 9**

| **Parameter (Mean ± SD)** | **Vehicle (n=6)** | **Cenicriviroc-low (n=6)** | **Cenicriviroc-high (n=6)** |
|---|---|---|---|
| White blood glucose (mg/dL) | 590 ± 108 | 585 ± 91 | 585 ± 91 |
| Plasma ALT (U/L) | 133 ± 80 | 58 ± 12 | 52 ± 13 |
| Plasma MCP-1 (pg/mL) | 60 ± 4 | 68 ± 16 | 91 ± 14 |
| Plasma MIP-1β (pg/mL) | 18 ± 5 | 18 ± 2 | 20 ± 4 |
| Liver triglyceride (mg/g liver) | 40.8 ± 20.4 | 48.5 ± 16.1 | 51.7 ± 14.1 |
| Liver hydroxyproline (µg/mg total protein) | 0.75 ± 0.18 | 0.63 ± 0.05 | 0.62 ± 0.09 |

Plasma MCP-1 data are shown in Figure 11C and Table 13. The CVC-high group showed a significant increase in plasma MCP-1 levels compared with the Vehicle group. There were no significant differences in plasma MCP-1 levels between the Vehicle group and the CVC-low group (Vehicle: 60 ± 4 pg/mL, CVC-low: 68 ± 16 pg/mL, CVC-high: 91 ± 14 pg/mL).

Plasma MIP-1β data are shown in Figure 11D, Table 13. There were no significant differences in plasma MIP-1β levels between the Vehicle group and either the CVC-low or the CVC-high groups (Vehicle: 18 ± 5 pg/mL, CVC-low: 18 ± 2 pg/mL, CVC-high: 20 ± 4 pg/mL). Liver Biochemistry at Week 9

Liver triglyceride content data are shown in Figure 11D and Table 13. There were no significant differences in liver triglyceride content between the Vehicle group and either the CVC-low or the CVC-high groups (Vehicle: 40.8 ± 20.4 mg/g liver, CVC-low: 48.5 ± 16.1 mg/g liver, CVC-high: 51.7 ± 14.1 mg/g liver).

Liver hydroxyproline content data are shown in Figure 11E and Table 13. The liver hydroxyproline content tended to decease in the CVC-low and the CVC-high groups compared with the Vehicle group (Vehicle: 0.75 ± 0.18 µg/mg, CVC-low: 0.63 ± 0.05 µg/mg, CVC-high: 0.62 ± 0.09 µg/mg).

### Histological Analyses at Week 9

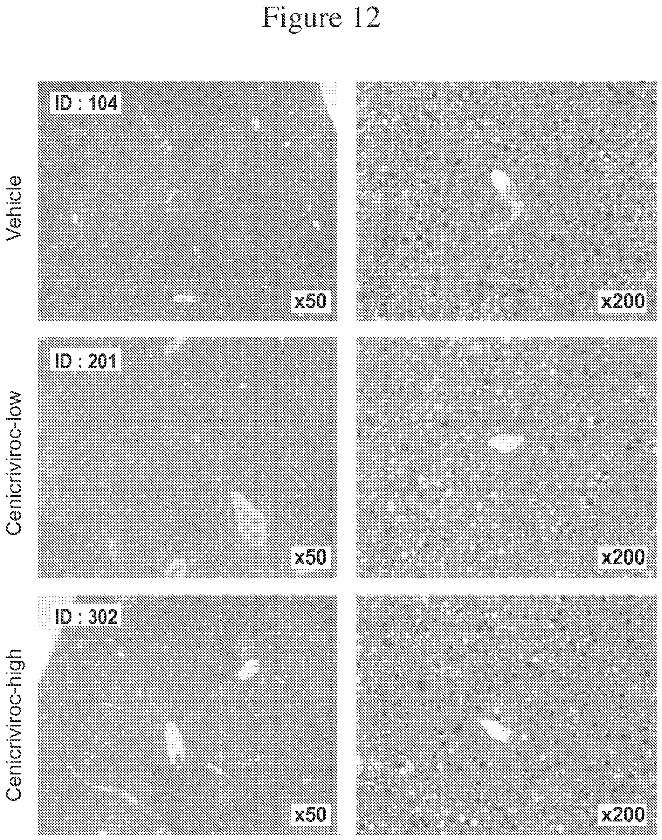

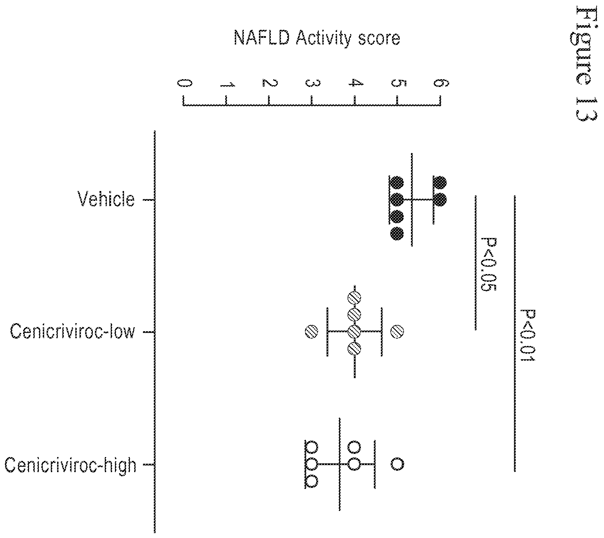

HE staining and NAFLD Activity score data are shown in Figures 12 and 13, and Table 15. Liver sections from the Vehicle group exhibited severe micro- and macrovesicular fat deposition, hepatocellular ballooning and inflammatory cell infiltration. The CVC-low and the CVC-high groups showed moderate improvements in inflammatory cell infiltration and hepatocellular ballooning, with a significant reduction in NAS compared with the Vehicle group (Vehicle: 5.3 ± 0.5, CVC-low: 4.0 ± 0.6, CVC-high: 3.7 ± 0.8). Representative photomicrographs of the HE-stained sections are shown in Figure 12.

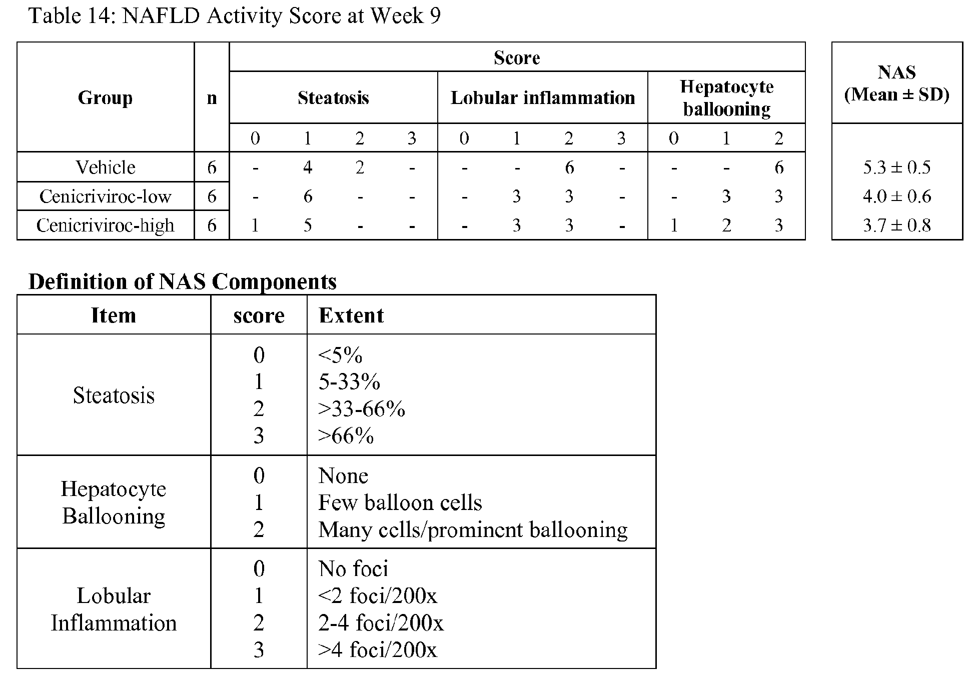

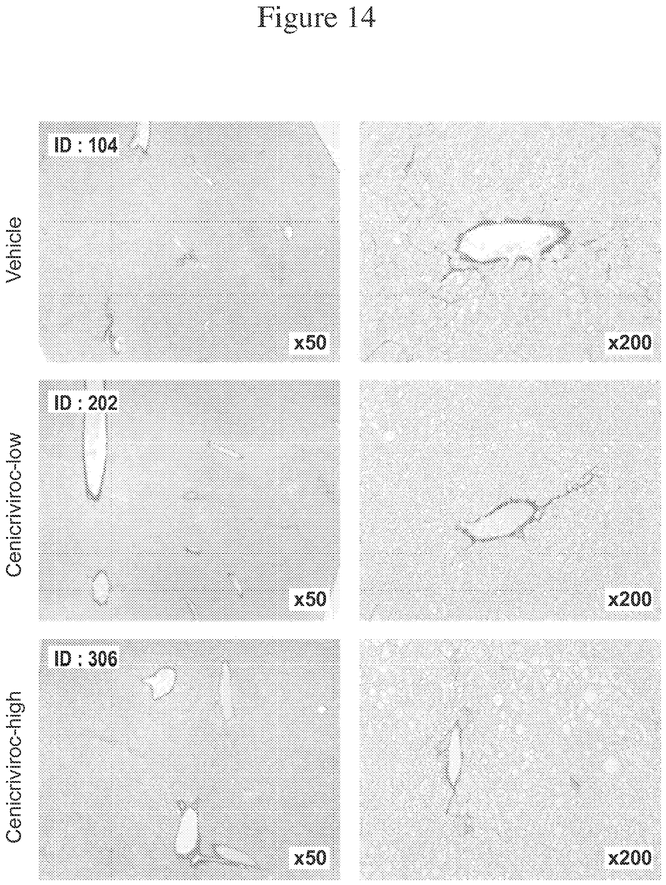

Sirius red staining data are shown in Figures 14, 15, and 16, and Table 15. Liver sections from the Vehicle group showed collagen deposition in the pericentral region of the liver lobule. Compared with the Vehicle group, collagen deposition in the pericentral region was markedly reduced in the CVC-low and the CVC-high groups. The fibrosis area (Sirius red-positive area) significantly decreased in the CVC-low and the CVC-high groups compared with the Vehicle group (Vehicle: 1.10 ± 0.31%, CVC-low: 0.66 ± 0.16%, CVC-high: 0.64 ± 0.19%). The modified fibrosis areas were also significantly reduced in the CVC-low and the CVC-high groups compared with the Vehicle group (Vehicle: 0.61 ± 0.23%, CVC-low: 0.29 ± 0.14%, CVC-high: 0.20 ±0.06%).

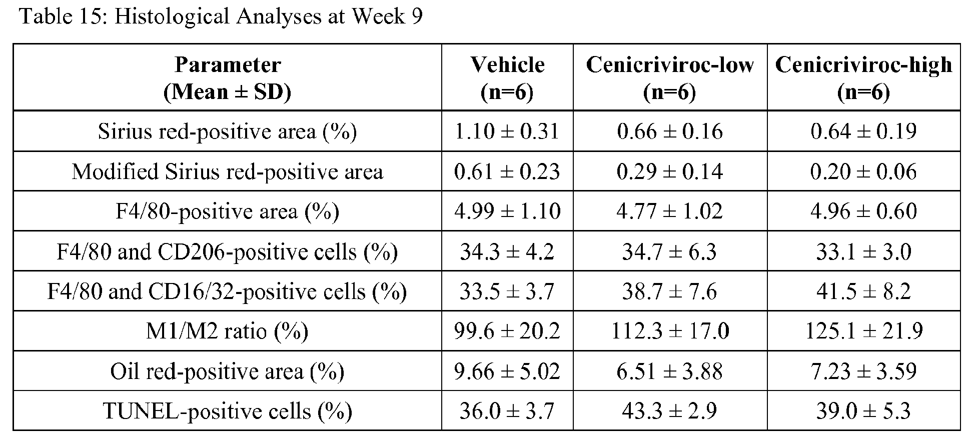

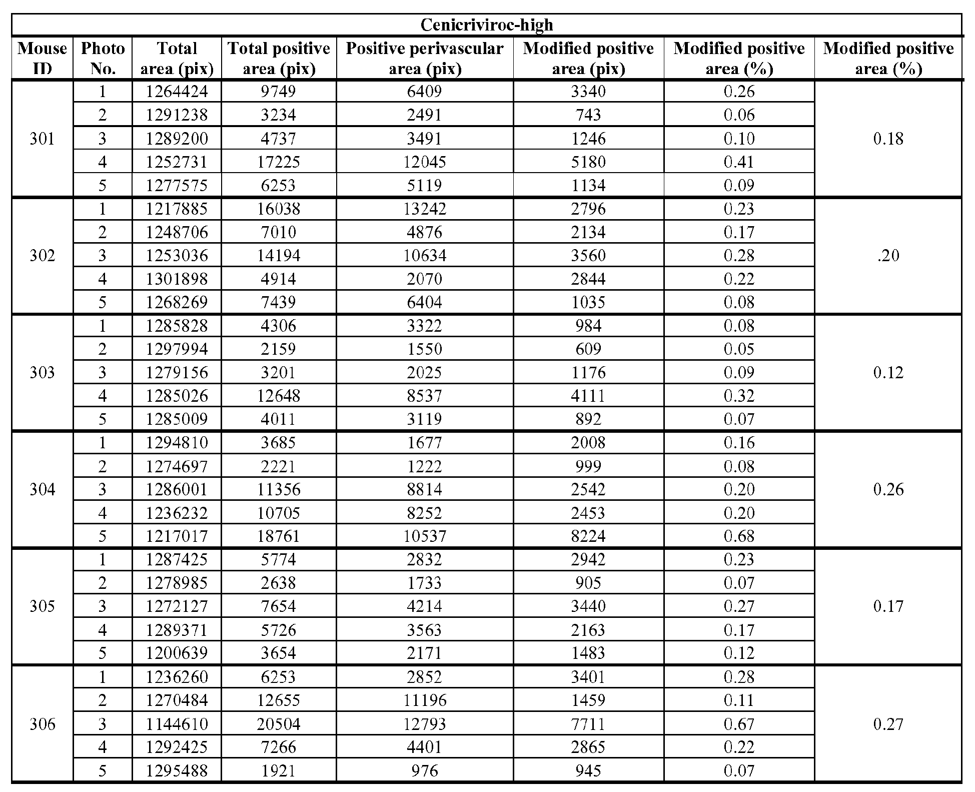

Representative photomicrographs of Sirius red-stained sections of livers are shown in Figure 14.

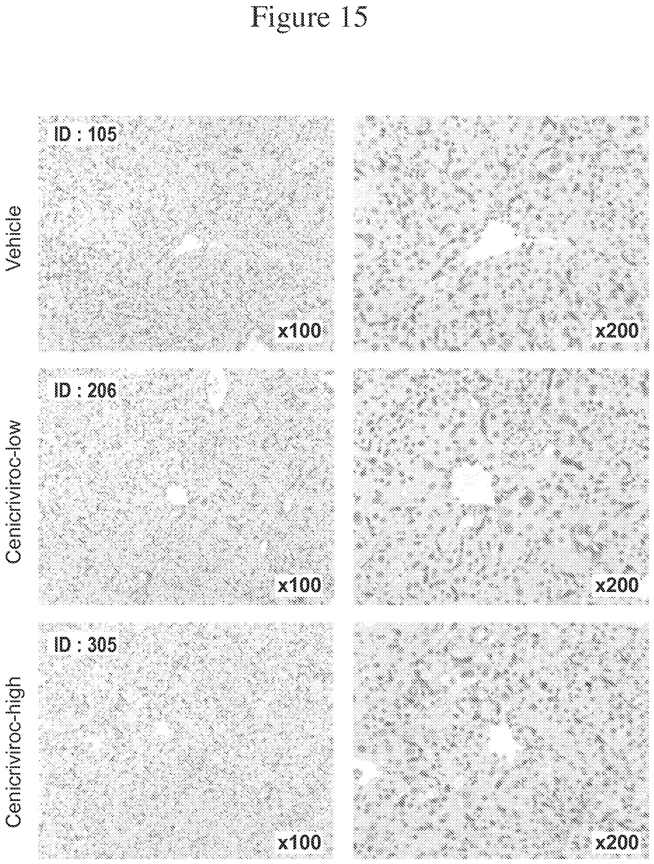

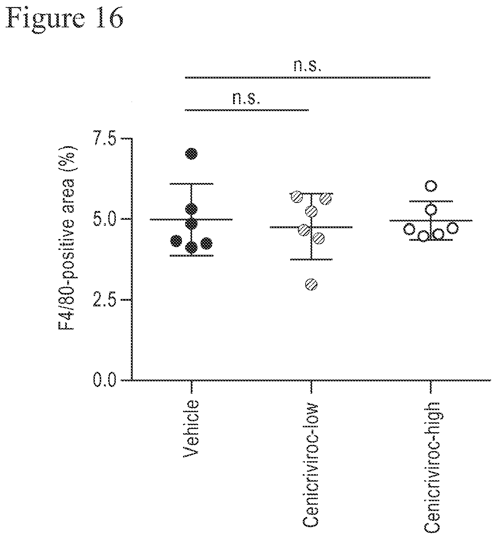

F4/80 immunohistochemistry data are shown Figures 15 and 16, and Table 15. F4/80 immunostaining of liver sections form the Vehicle group demonstrated accumulation of F4/80+ cells in the liver lobule. There were no significant differences in the number and size of F4/80+ cells between the Vehicle group and either the CVC-low or the CVC-high groups, as well as in the percentage of inflammation area (F4/80-positive area) (Vehicle: 4.99 ± 1.10%, CVC-low: 4.77 ± 1.02%, CVC-high: 4.96 ± 0.60%).

Representative photomicrographs of the F4/80-immunostained sections are shown in Figure 15.

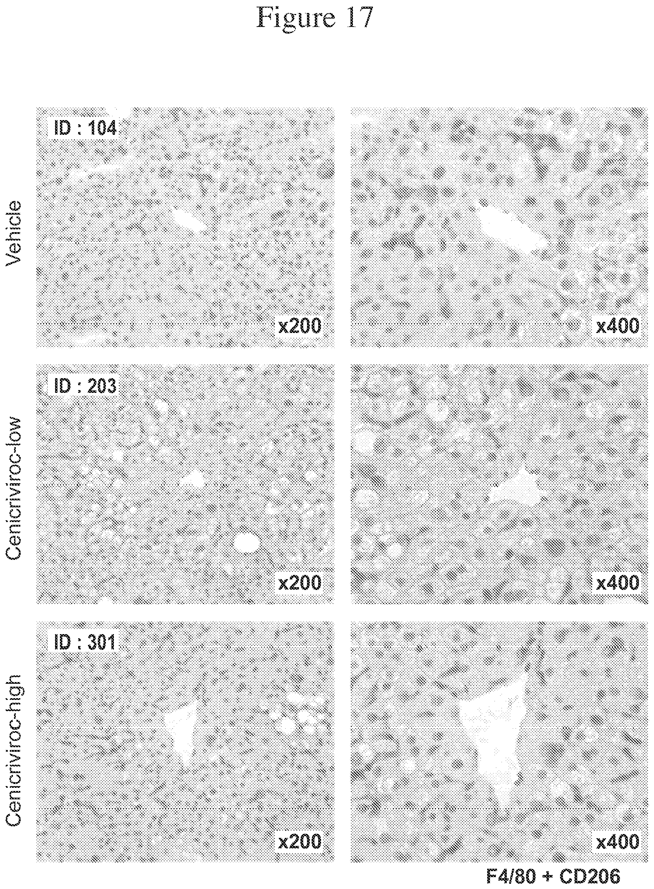

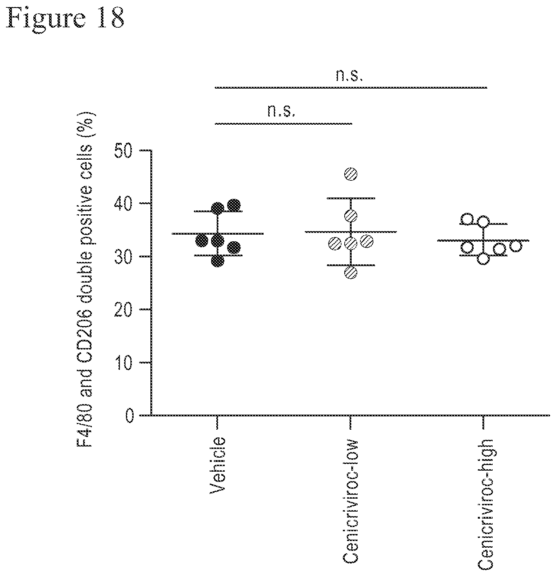

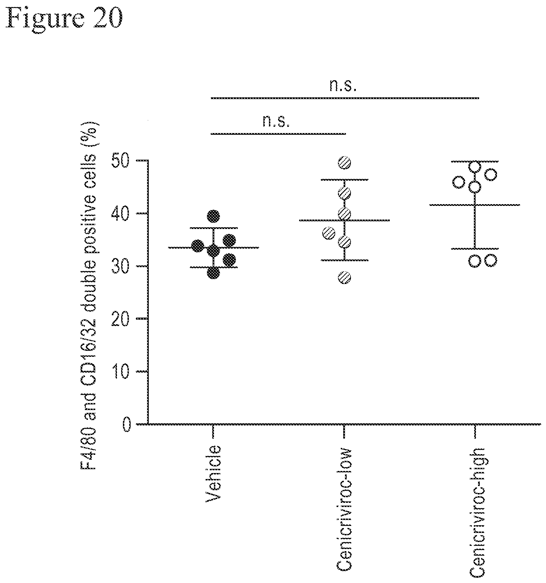

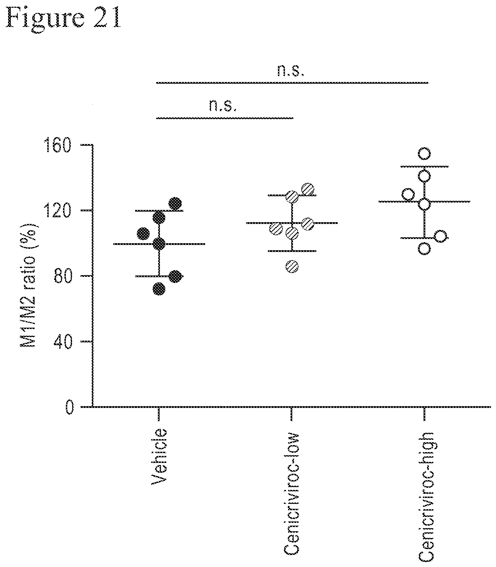

F4/80+CD206+ and F4/80+CD16/32+ immunohistochemistry data are shown in Figures 17-21, and Table 15). There were no significant differences in the percentages of F4/80+CD206+ cells in macrophages between the Vehicle group and either the CVC-low or the CVC-high groups (Vehicle: 34.3 ± 4.2%, CVC-low: 34.7 ± 6.3%, CVC-high: 33.1 ± 3.0%). There was no significant difference in the percentages of F4/80+CD16/32+ cells in macrophages between the Vehicle group and the CVC-low group. The percentages of F4/80+CD16/32+ cells tended to increase in the CVC-high group compared with the Vehicle (Vehicle: 33.5 ± 3.7%, CVC-low: 38.7 ± 7.6%, CVC-high: 41.5 ± 8.2%). There was no significant difference in the M1/M2 ratio between the Vehicle group and the CVC-low group. In the CVC-high group, the M1/M2 ratio tended to increase compared with the Vehicle (Vehicle: 99.6 ± 20.2%, CVC-low: 112.3 ± 17.0%, CVC-high: 125.1 ± 21.9%).

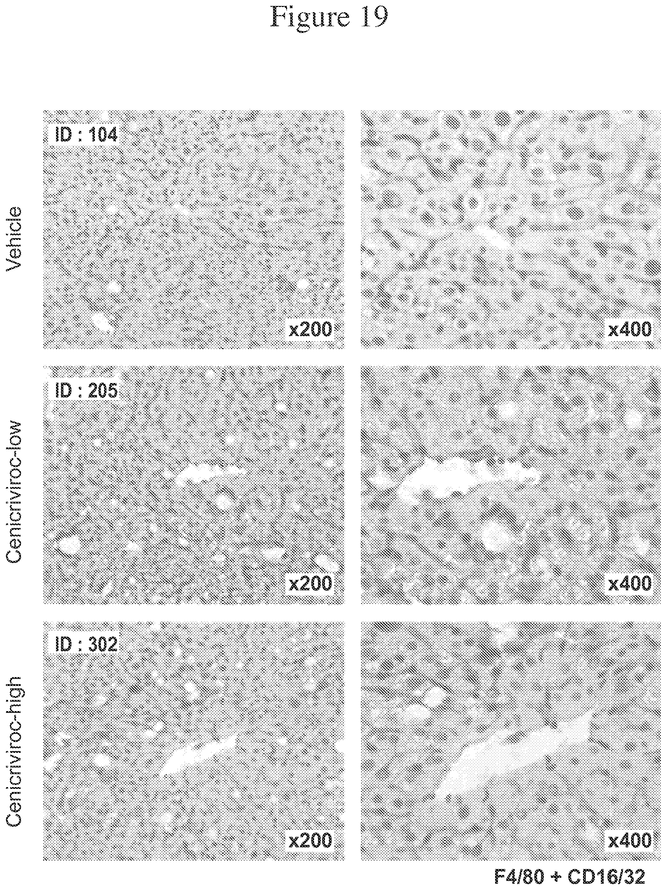

Representative photomicrographs of the F4/80 and CD206, F4/80 and CD16/32 double-immunostained sections are shown in Figures 17 and 19.

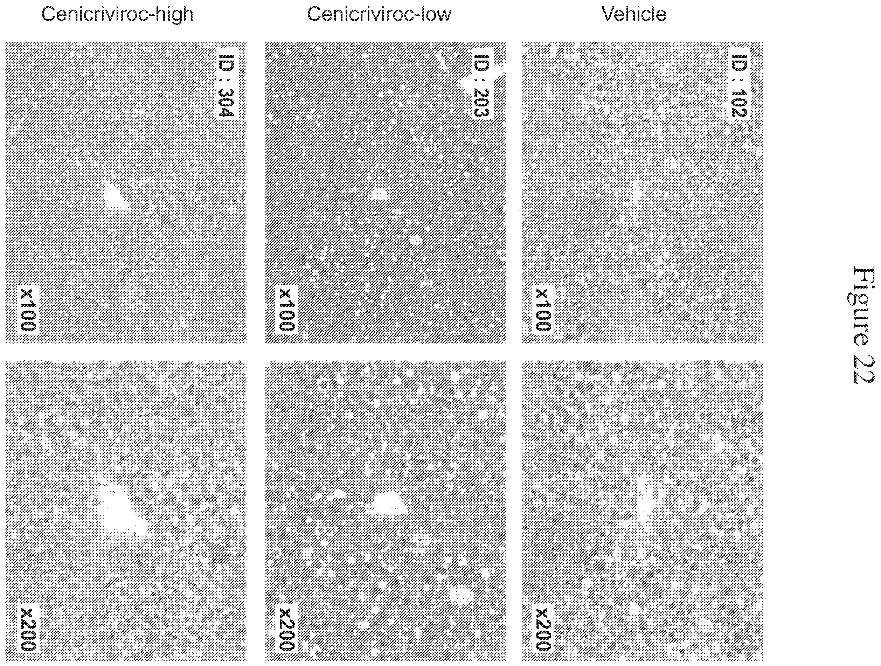

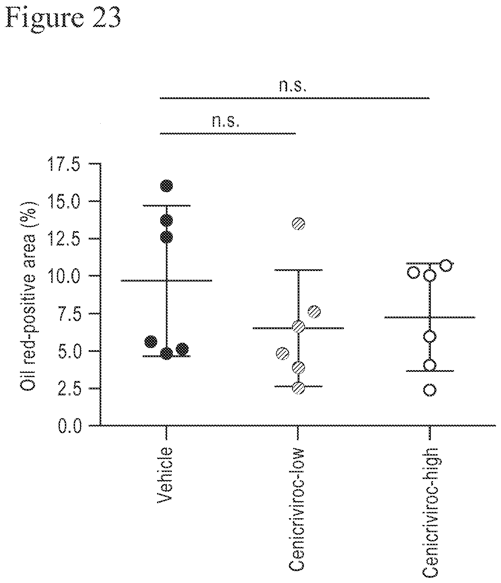

Oil red staining data are shown in Figures 22, 23, and Table 15. There were no significant differences in the fat deposition between the Vehicle group and either the CVC-low or the CVC-high groups, as well as in the percentage of fat deposition area (oil-positive area) (Vehicle: 9.66 + 5.02%, CVC-low: 6.51 + 3.88%, CVC-high: 7.23 + 3.59%).

Representative photomicrographs of the oil red-stained sections are shown in Figure 22.

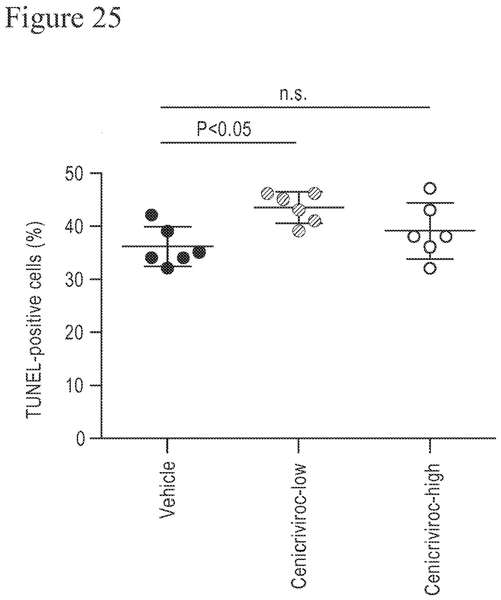

TUNEL staining data are shown in Figures 23, 25 and Table 15. The percentages of TUNEL-positive cells significantly increased in the CVC-low group compared with the Vehicle group. There was no significant difference in percentages of TUNEL-positive cells between the Vehicle group and the CVC-high group (Vehicle: 36.0 + 3.7%, CVC-low: 43.3 + 2.9%, CVC-high: 39.0 ± 5.3%).

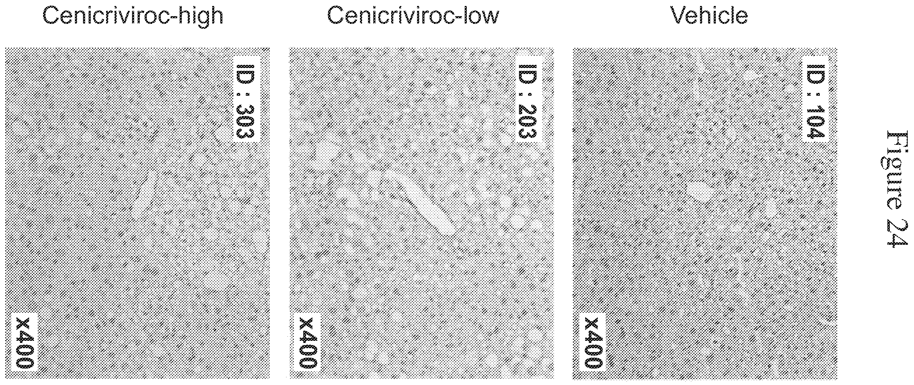

Representative photomicrographs of TUNEL-positive cells in livers are shown in Figure 24.

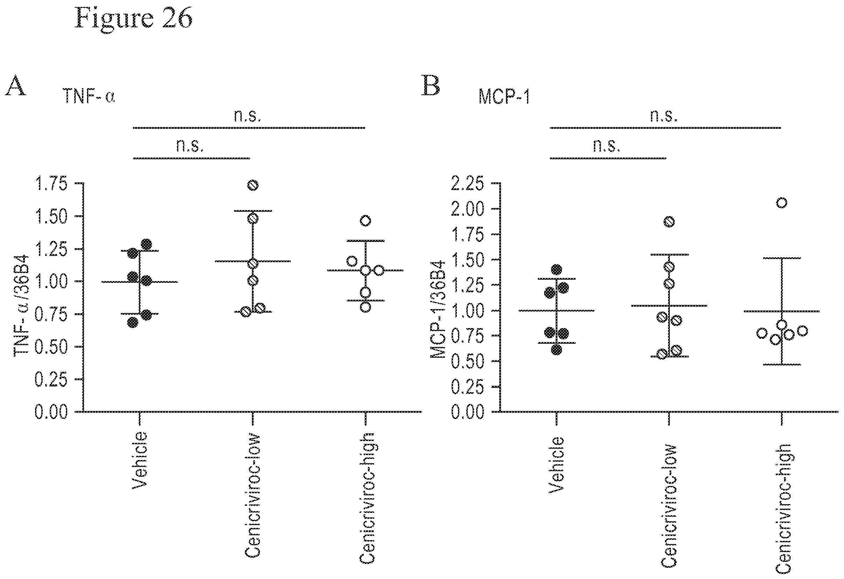

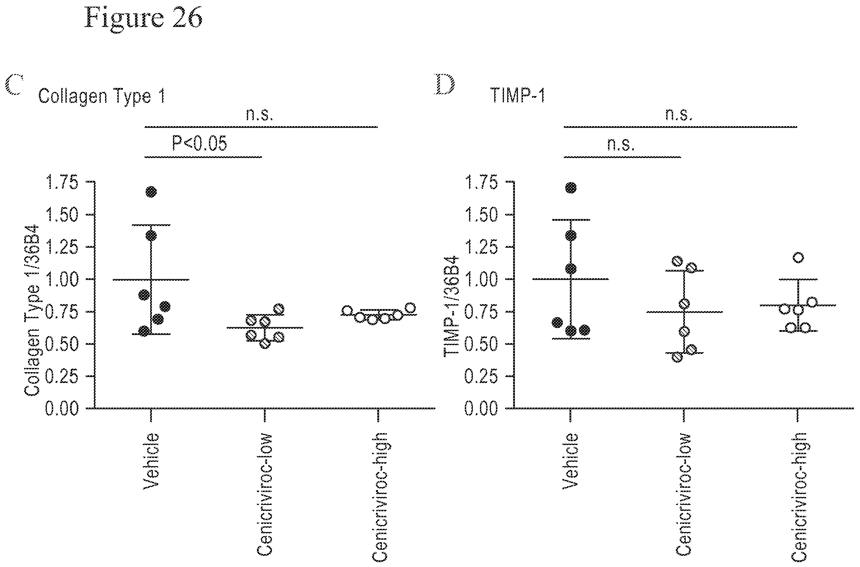

Gene Expression Analysis at Week 9 data are shown in Figure 26 and Tables 16-17.

**Table 16: Gene Expression Analysis at Week 9**

| **Parameter (Mean ± SD)** | **Vehicle (n=6)** | **Cenicriviroc-low (n=6)** | **Cenicriviroc-high (n=6)** |
|---|---|---|---|
| TNF-α | 1.00 ± 0.24 | 1.16 ± 0.39 | 1.09 ± 0.23 |
| MCP-1 | 1.00 ± 0.31 | 1.05 ± 0.50 | 1.00 ± 0.53 |
| Collagen Type 1 | 1.00 ± 0.42 | 0.63 ± 0.10 | 0.73 ± 0.04 |
| TIMP-1 | 1.00 ± 0.46 | 0.75 ± 0.32 | 0.80 ± 0.20 |

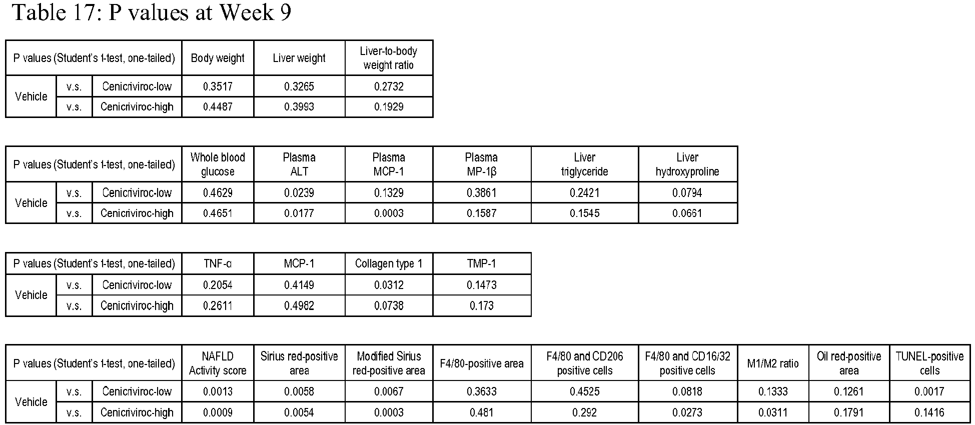

### TNFα

There were no significant differences in TNFα mRNA expression levels between the Vehicle group and either the CVC-low or the CVC-high groups (Vehicle: 1.00 ± 0.24, CVC-low: 1.16 ± 0.39, CVC-high: 1.09 ± 0.23).

### MCP-1

There were no significant differences in MCP-1 mRNA between the Vehicle group and either the CVC-low or the CVC-high groups (Vehicle: 1.00 ± 0.31, CVC-low: 1.05 ± 0.50, CVC-high: 1.00 ± 0.53).

### Collagen Type 1

Collagen Type 1 mRNA expression levels were significantly down-regulated in the CVC-low group compared with the Vehicle group. Collagen Type 1 mRNA expression levels tended to be down-regulated in the CVC-high group compared with the Vehicle group. (Vehicle: 1.00 ± 0.42, CVC-low: 0.63 ± 0.10, CVC-high: 0.73 ± 0.04).

### TIMP-1

There were no significant differences in TIMP-1 mRNA expression levels between the Vehicle group and either the CVC-low and the CVC-high groups (Vehicle: 1.00 ± 0.46, CVC-low: 0.75 ± 0.32, CVC-high: 0.80 ± 0.20).

### Part 2: study for assessing the anti-HCC effects of CVC

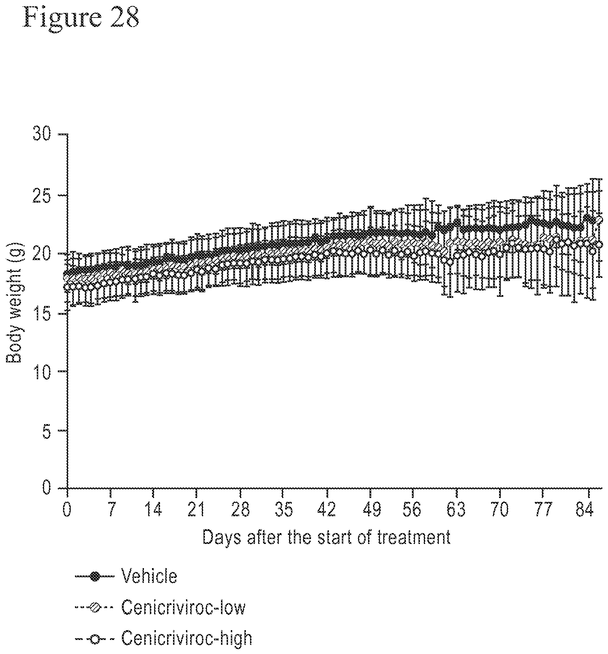

### Body weight changes until week 18 (Figure 28)

Body weight gradually increased during the treatment period. There were no significant differences in mean body weight between the Vehicle group and either the CVC-low or the CVC-high groups during the treatment period.

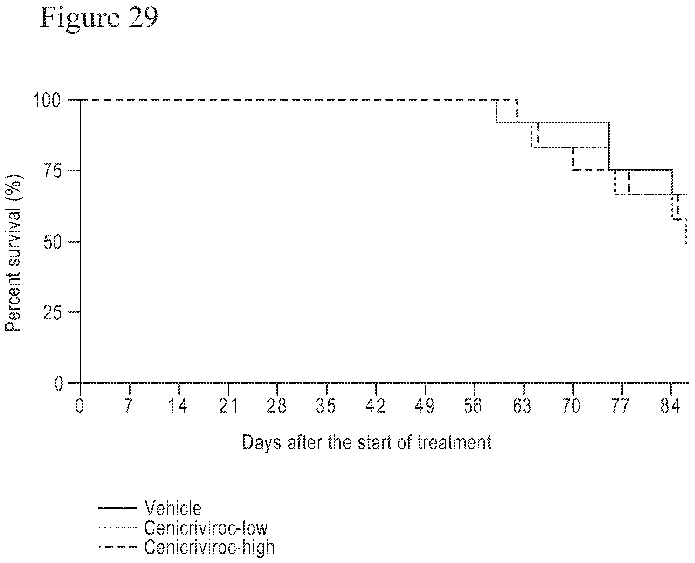

Survival analysis data are shown in Figure 29. Four out of twelve mice died at day 59 (ID112), day 75 (ID113, 115) and day 84 (ID116) in the Vehicle group (The first day of administration was designed as day 0). Six out of twelve mice died at day 62 (ID209), day 64 (ID217), day 75 (ID212), day 76 (ID213), day 84 (ID215) and day 86 (ID208) in the CVC-low group. Five out of twelve mice died at day 62 (ID317), day 65 (ID312), day 70 (ID316), day 78 (ID314) and day 85 (ID309) in the CVC-high group. There were no abnormal necropsy findings in the dead animals except for the typical hepatic lesions of NASH. There were no significant differences in survival rate between the Vehicle group and either the CVC-low or the CVC-high groups. By consigner instruction, the rest of the animals were sacrificed earlier than scheduled at 18 weeks of age (scheduled sacrificed at 20 weeks of age).

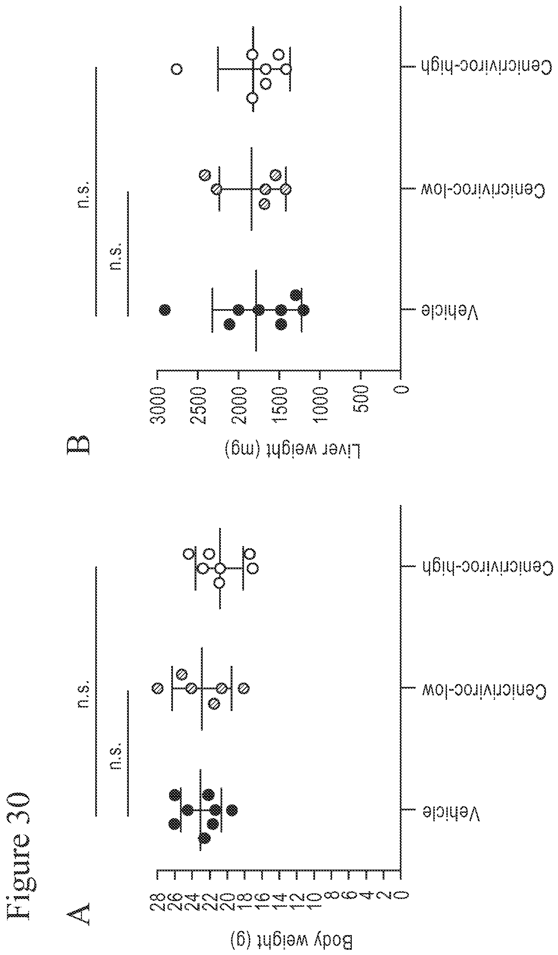

Body Weight at the Day of Sacrifice at Week 18 data are shown in Figure 30A and Table 18. The body weight tended to decrease in the CVC-high group compared with the Vehicle group. There was no significant difference in mean body weight between the Vehicle group and the CVC-low group (Vehicle: 23.0 ± 2.3 g, CVC-low: 22.9 ± 3.5 g, CVC-high: 20.8 ± 2.7 g).

**Table 18: Body Weight and Liver Weight at Week 18**

| **Parameter (Mean ± SD)** | **Vehicle (n=8)** | **Cenicriviroc-low (n=6)** | **Cenicriviroc-high (n=7)** |
|---|---|---|---|
| Body weight (g) | 23.0 ± 2.3 | 22.9 ± 3.5 | 20.8 ± 2.7 |
| Liver weight (mg) | 1782 ± 558 | 1837 ± 410 | 1817 ± 446 |
| Liver-to-body weight ratio (%) | 7.7 ± 2.2 | 8.3 ± 2.8 | 8.8 ± 2.3 |

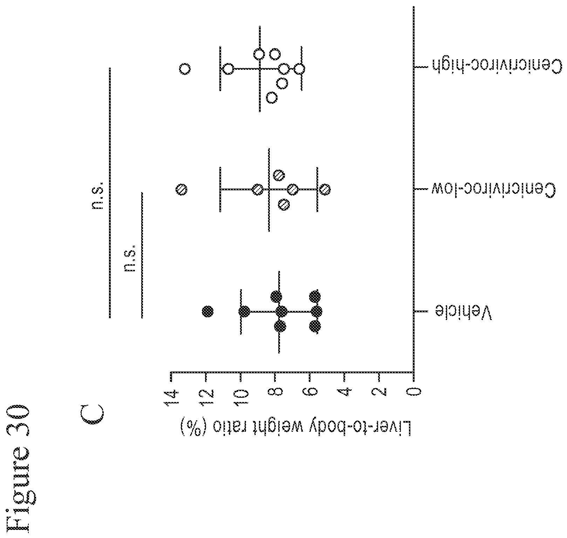

Liver Weight and Liver-to-Body Weight Ratio at Week 18 data are shown in Figures 30B & C and Table 18. There were no significant differences in mean liver weight between the Vehicle group and either the CVC-low or the CVC-high groups (Vehicle: 1782 ± 558 mg, CVC-low: 1837 ± 410 mg, CVC-high: 1817 ± 446 mg). There were no significant differences in mean liver-to-body weight ratio between the Vehicle group and either the CVC-low or the CVC-high groups (Vehicle: 7.7 ± 2.2%, CVC-low: 8.3 ± 2.8%, CVC-high: 8.8 ± 2.3%).

### Macroscopic Analyses of Liver at Week 18

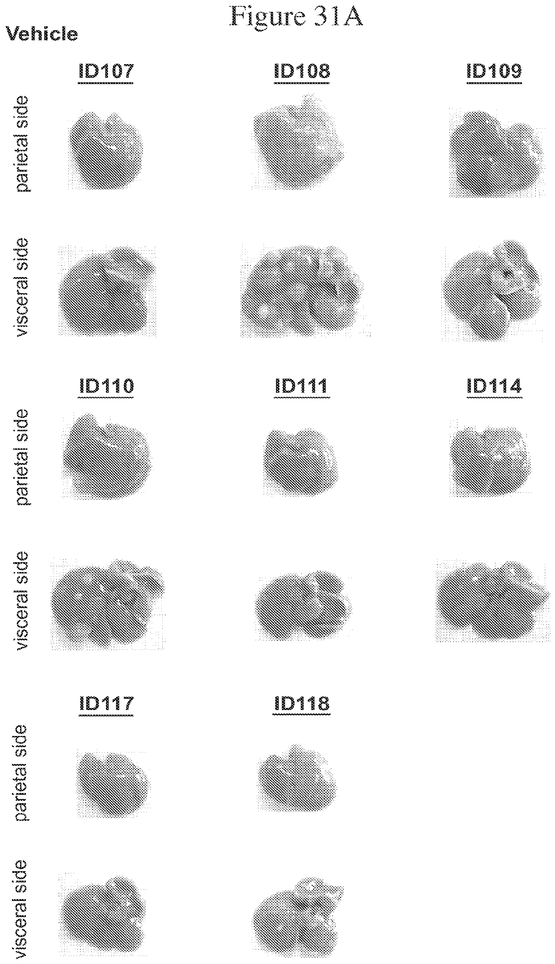

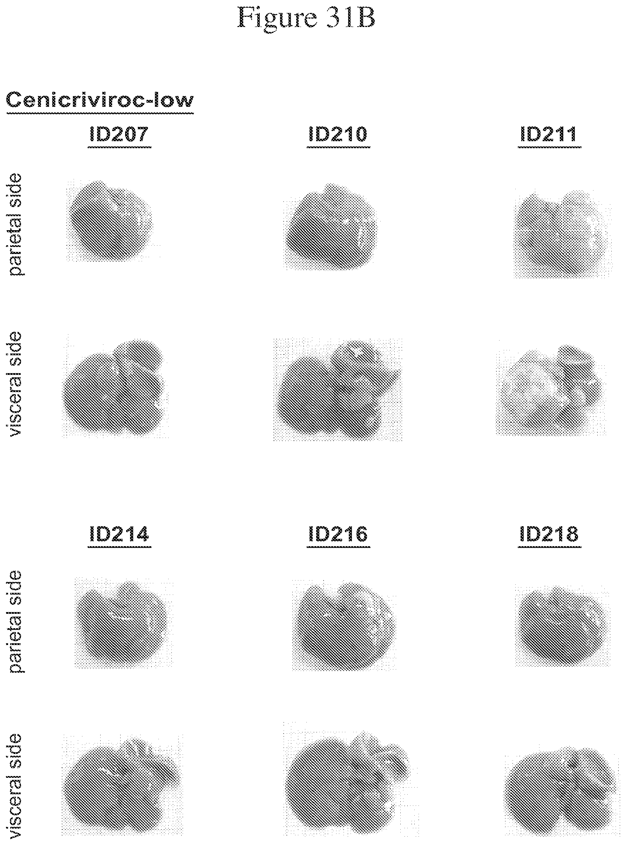

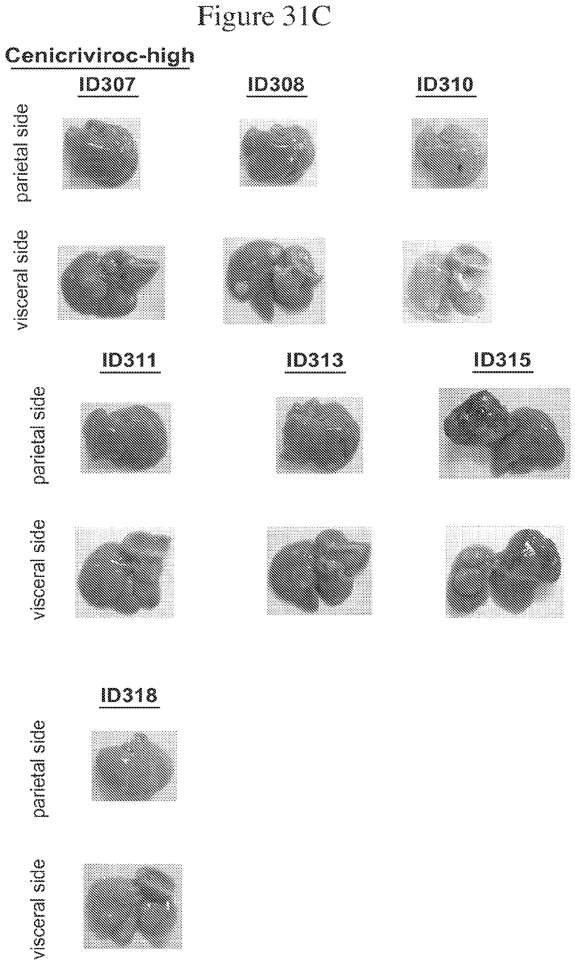

Macroscopic appearance of livers is shown in Figures 31A-C.

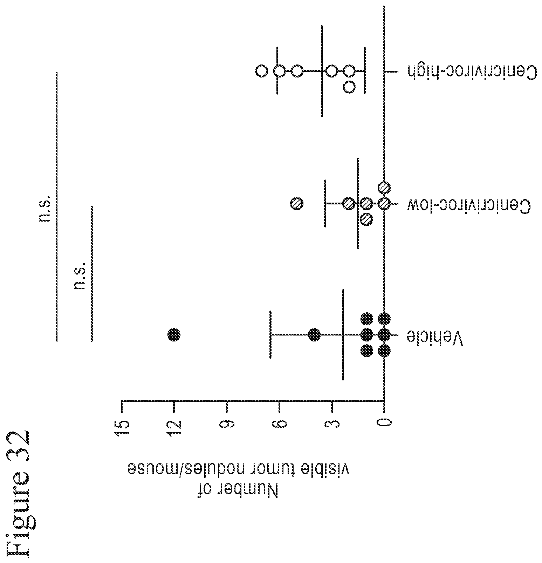

Number of visible tumor nodules formed on liver surface are shown in Figure 32 and Table 29. There were no significant differences in the number of hepatic tumor nodules per individual mouse between the Vehicle group and either the CVC-low or the CVC-high groups (Vehicle: 2.4 ± 4.1, CVC-low: 1.5 ± 1.9, CVC-high: 3.6 ± 2.5).

**Table 19: Macroscopic Analyses of Liver at Week 18**

| **Parameter (Mean ± SD)** | **Vehicle (n=8)** | **Cenicriviroc-low (n=6)** | **Cenicriviroc-high (n=7)** |
|---|---|---|---|
| Number of visible tumor nodules | 2.4 ± 4.1 | 1.5 ± 1.9 | 3.6 ± 2.5 |
| Maximum diameter of visible tumor nodules (mm) | 4.0 ± 14.7 | 4.8 ± 5.4 | 5.3 ± 5.1 |

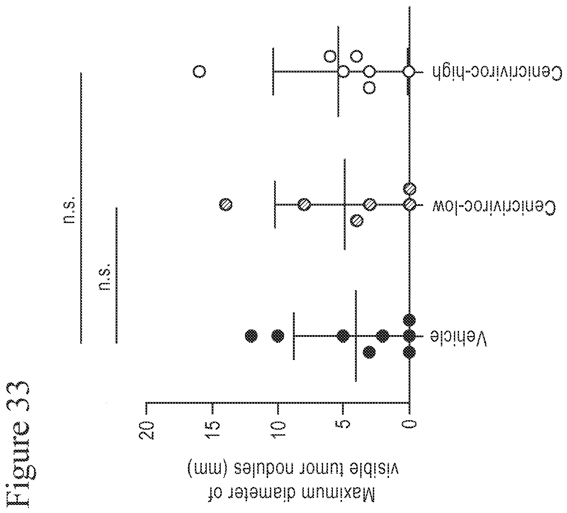

Maximum diameters of visible tumor nodules formed on liver surface are shown in Figure 33 and Table 19. There were no significant differences in maximum diameter of tumor between the Vehicle group and either the CVC-low or the CVC-high groups (Vehicle: 4.0 ± 4.7 mm, CVC-low: 4.8 ± 5.4 mm, CVC-high: 5.3 ± 5.1 mm).

### Histological Analyses at Week 18

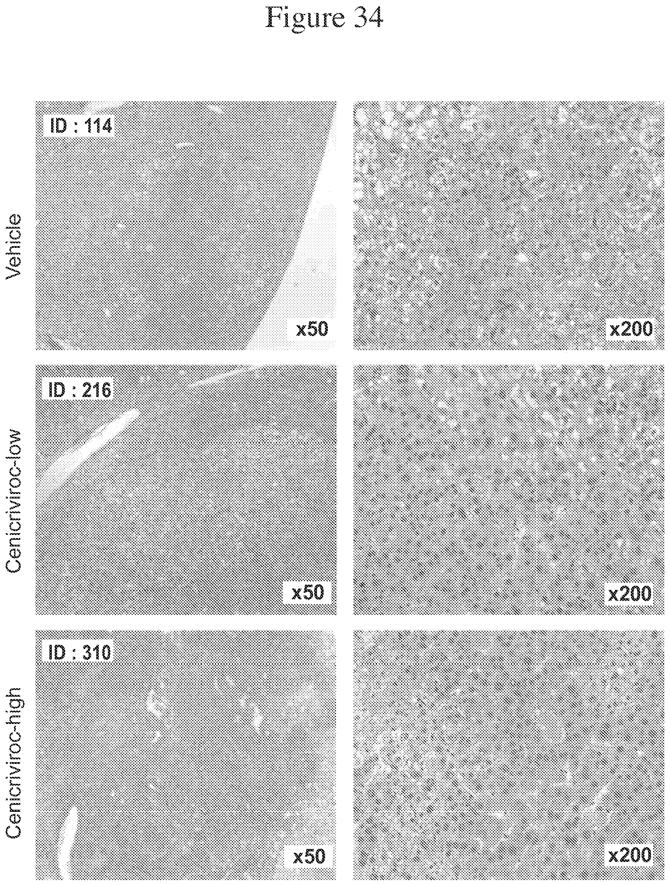

HE staining data are shown in Figure 34. HE staining revealed infiltration of inflammatory cells, macro- and microvesicular fat deposition, hepatocellular ballooning, altered foci and nodular lesions in the Vehicle group. Six out of eight mice in the Vehicle group exhibited HCC lesions. HCC lesions were detected in five out of six mice in the CVC-low group and six out of seven mice in the CVC-high group. No obvious differences were found between the Vehicle group and either the CVC-low or the CVC-high groups.

Representative photomicrographs of the HE-stained sections are shown in Figure 34.

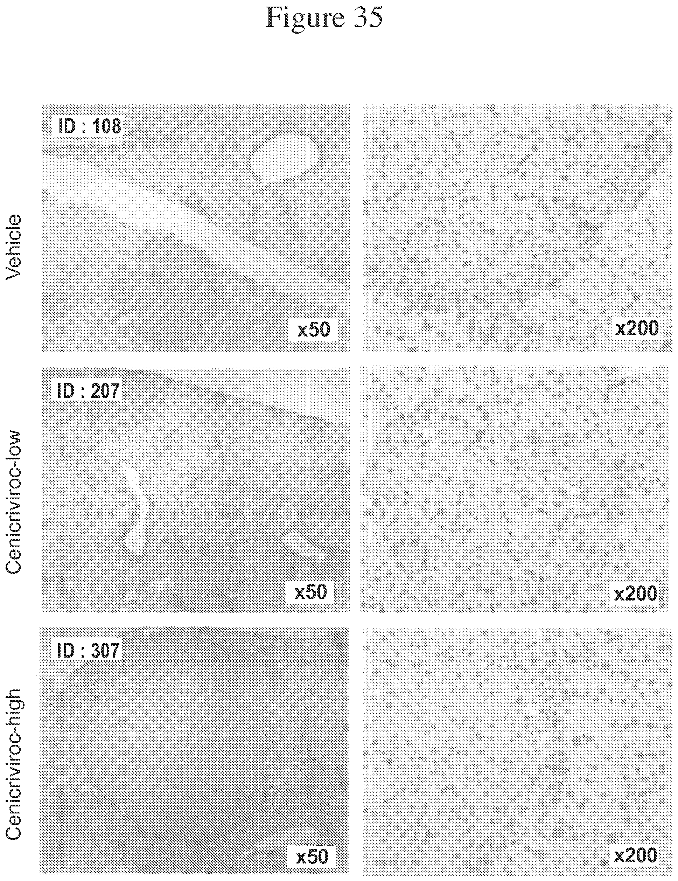

GS immunohistochemistry data are shown in Figure 35. GS-positive nodules in the sections were detected in six out of eight mice in the Vehicle group, five out of six mice in the CVC-low group and seven out of seven mice in the CVC-high group, respectively.

Representative photomicrographs of the GS-stained sections are shown in Figure 35.

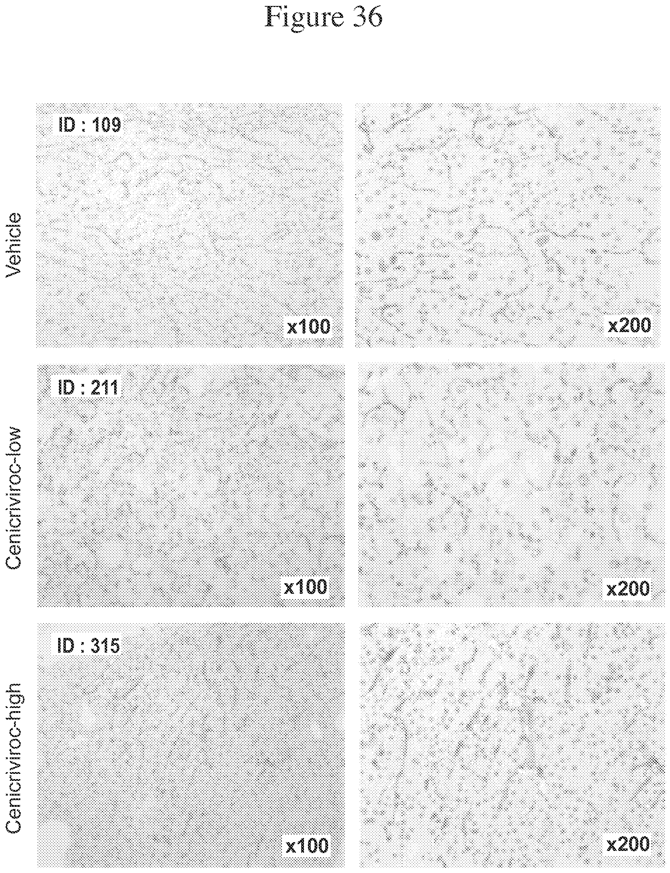

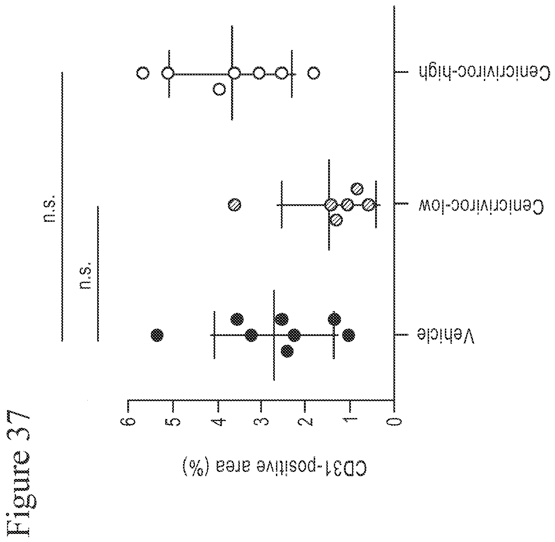

CD31 immunohistochemistry data are shown in Figures 36 and 37 and Table 20. The CD31-positive area tended to decrease in the CVC-low group compared with the Vehicle group. The CD31-positive area tended to increase in the CVC-high group compared with the Vehicle group (Vehicle: 2.71 ± 1.36%, CVC-low: 1.47 ± 1.10%, CVC-high: 3.68 ± 1.37%).

Representative photomicrographs of the CD31-stained sections are shown in Figure 36.

**Table 20: Histological Analyses at Week 18**

| Parameter (Mean + SD) | Vehicle (n=8) | Cenicriviroc-low (n=6) | Cenicriviroc-high (n=7) |
|---|---|---|---|
| CD31-positive area (%) | 2.71 ± 1.36 | 1.47 ± 1.10 | 3.68 ± 1.37 |

**Table 21: P Values at Week 18**

| P value (Student's t-test, one-tailed) | Body Weig ht | Liver Weig ht | Liver-to-body weight ratio | The number of visible tumor nodules | Maximum diameter of visible tumor nodules | CD31-positive area |
|---|---|---|---|---|---|---|
| Vehicle vs Cenicriviroc-low | 0.475 8 | 0.421 5 | 0.341 | 0.3191 | 0.3812 | 0.0456 |
| Vehicle vs Cenicriviroc-high | 0.057 4 | 0.447 6 | 0.184 | 0.2578 | 0.3096 | 0.0972 |
| | | | | | | |
| P values (Logrank-test) | Survival Curve | | | | | |
| Vehicle vs Cenicriviroc-low | 0.7513 | | | | | |
| Vehicle vs Cenicriviroc-high | 0.5701 | | | | | |

### SUMMARY AND DISCUSSION

In the analyses at week 9, treatment with low and high dose of CVC significantly reduced fibrosis area in a dose dependent manner, demonstrating anti-fibrotic effect of CVC in the present study. Treatment with low and high dose of CVC also reduced the mRNA expression levels of Collagen Type 1 and liver hydroxyproline content, supporting its anti-fibrotic property. CVC treatment groups significantly decreased plasma ALT levels and NAS compared with the Vehicle group in a dose dependent manner. The improvement in NAS was attributable to the reduction in lobular inflammation and hepatocyte ballooning. Since hepatocyte ballooning is derived from oxidative stress-induced hepatocellular damage and is associated with disease progression of NASH [26; 27], it is strongly suggested that CVC improved NASH pathology by inhibiting hepatocyte damage and ballooning. Together, CVC have potential anti-NASH and hepatoprotective effects in this study.

As shown in humans, plasma MCP-1 levels increased by the treatment with CVC in the present study, indicating dose-dependent antagonism of CCR2 by CVC, but plasma MIP-1β levels did not show any significant changes by the treatment. To investigate the mechanism of action of CVC, we evaluated the effect of CVC on population of the macrophages. Preliminary results demonstrated that CVC showed the tendency of high M1/M2 ratio compared with Vehicle group, suggesting that CVC might inhibit the fibrogenesis by regulating the balance of macrophage subpopulation in the inflamed liver. This will be further investigated in the future.

In the analyses at week 18, the effect on NASH-derived HCC was not observed in the CVC treatment groups. In conclusion, CVC showed anti-NASH, hepatoprotective and anti-fibrotic effects in the present study.

### Example 15: Long-Term Efficacy Data in HIV-1 Infected Adult Subjects

### Efficacy Results of Study 202

### Study Design and Objectives

As described in US Application Nos. 61/968,829 and 62/024,713 (both herein indorporated by reference in their entireties, for all purposes) analysis of randomized, double-blind, double-dummy, 48-week comparative study evaluating efficacy and safety of CVC 100 mg and CVC 200 mg compared to approved antiretroviral agent efavirenz (EFV, Sustiva®) (Study 202) showed CVC administration has an anti-fibrotic effect.

### Biomarkers of Inflammation

As an exploratory analysis, levels of inflammation biomarkers MCP-1, sCD14, high sensitivity C-reactive protein [hs-CRP], interleukin-6 [IL-6], D-dimer, and fibrinogen) were measured. Baseline values and changes from baseline at Week 24 and Week 48 of MCP-1, sCD14, hs-CRP, IL-6, D-dimer, and fibrinogen are summarized in Table 22.

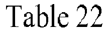

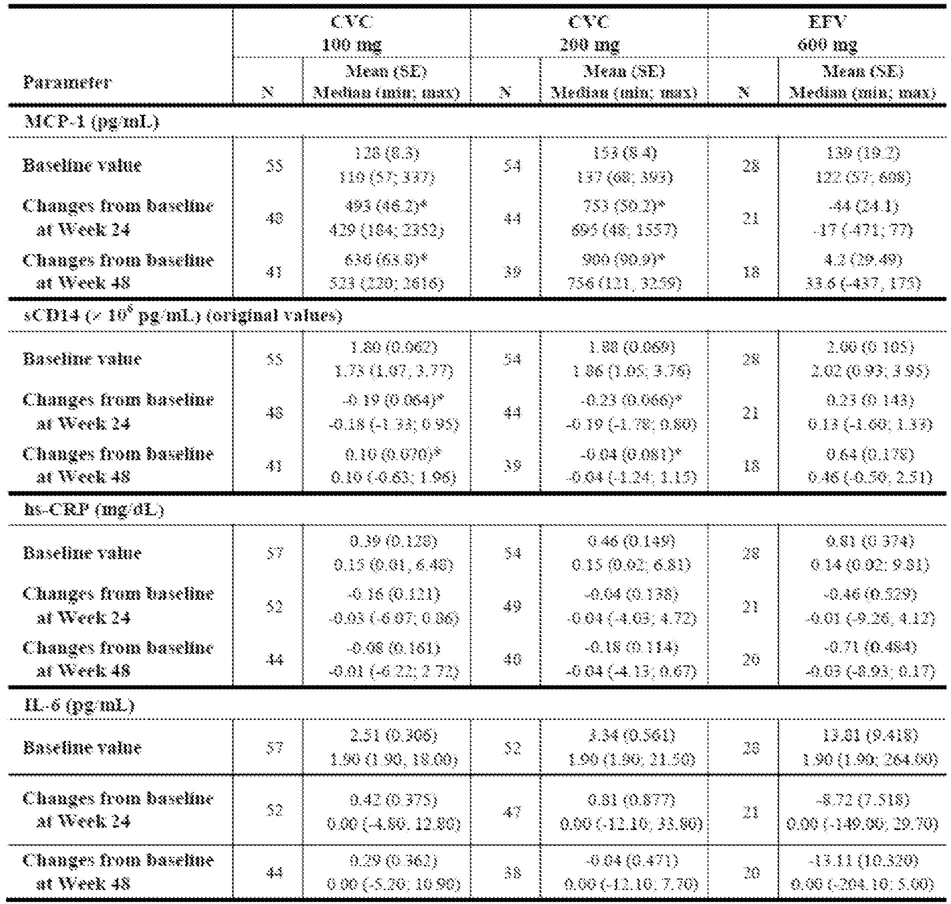

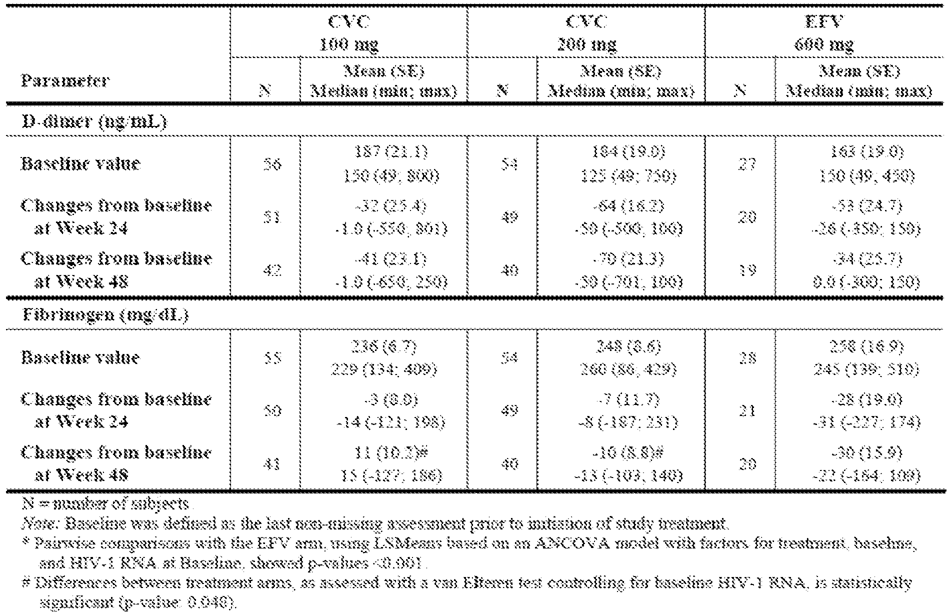

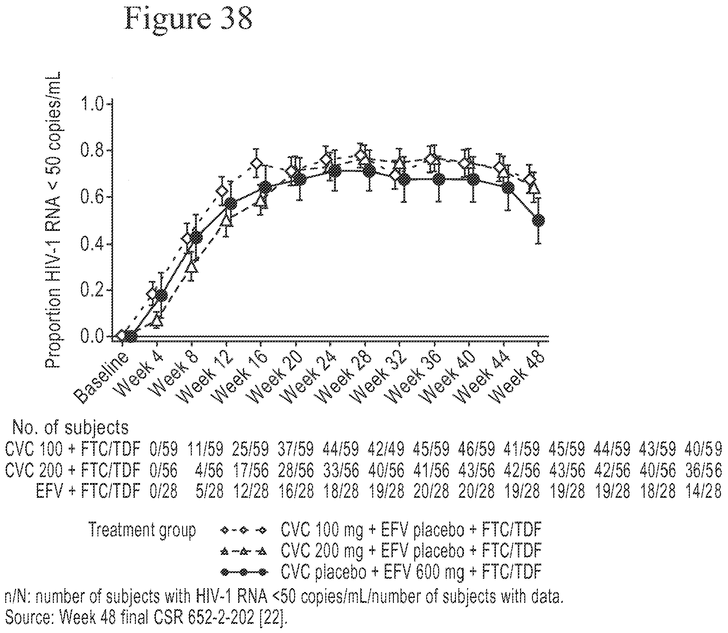

A dose-response was observed with CVC in increases over time of MCP-1, a ligand of CCR2, while MCP-1 remained at baseline values in the EFV arm (see Figure 38). The differences in changes from baseline of plasma MCP-1 between the EFV and CVC 100 mg and CVC 200 mg treatment arms were statistically significant (p<0.001) at Week 24 and Week 48 (see Table 22).

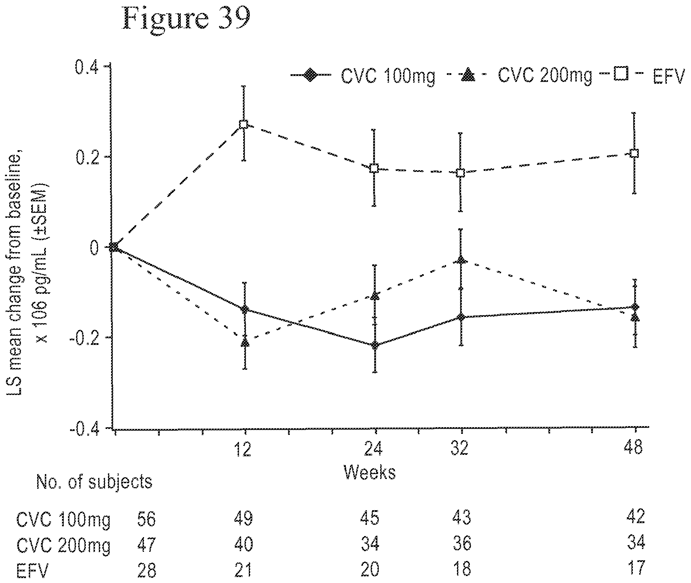

In addition, a decrease over 48 weeks of treatment was observed for sCD14 (linear mixed-model analysis of repeat sCD14 analysis, see below) in both CVC treatment arms, while an increase was observed for sCD14 in the EFV arm during the same observation period (see Figure 39). Soluble CD14 is a biomarker of monocyte activation and has been independently associated with morbidity and mortality in large, long-term cohort studies in HIV-infected patients and with worse clinical outcomes in patients with chronic viral hepatitis and patients with severe hepatic fibrosis.

The sCD14 samples were originally analyzed in 2 separate batches: Batch 1 included samples leading up to the Week 24 primary analysis and Batch 2 included Week 32 and Week 48 (end of study) samples. Results for changes in sCD14 from baseline from the 2-batch analysis are presented in Table 22. A repeat analysis of archived samples all analyzed in one batch was conducted for consistency in analysis across time points. To control for the effects of covariates, a linear mixed-model repeated-measures analysis was conducted on the changes from baseline in sCD14 (analysis dated September 2013). With the exception of changes from baseline to Week 32 in the CVC 200 mg arm, reductions in sCD14 levels observed with CVC at both doses (100 and 200 mg) over 48 weeks of treatment (LS means) were statistically significant compared to increases observed with EFV (p<0.05) (see Table 23 and Figure 39).

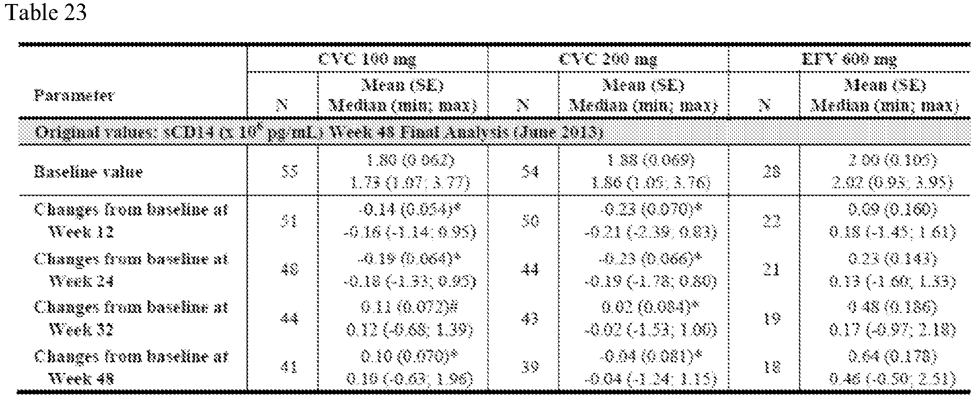

Changes in other biomarkers of inflammation (hs-CRP, IL-6, D-dimer) were similar in the CVC and EFV treatment groups.

### APRI and FIB-4 Scores

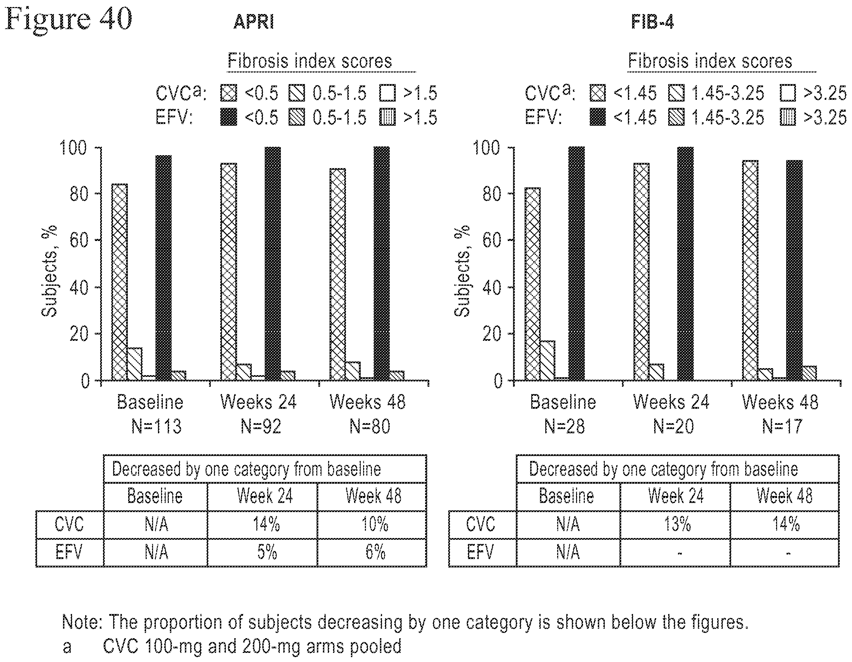

Furthermore, in post-hoc analyses of data from this study that enrolled subjects with no apparent liver disease according to stringent eligibility criteria (HIV-1 infection and without ALT/AST Grade ≥ 2, total bilirubin > ULN, HBV and/or HCV, active or chronic liver disease, cirrhosis or BMI > 35 kg/m2), improvements in AST-to-platelet ratio index (APRI) and noninvasive hepatic fibrosis index score combining standard biochemical values, platelets, ALT, AST, and age (FIB-4) scores were observed over time in ≥ 10% of all CVC-treated subjects (pooled data for CVC 100 mg and 200 mg) (Figure 40). In the EFV arm, 5% of subjects at Week 24 and 6% of subjects at Week 48 had a decrease in APRI score by one category from baseline; no subject treated with EFV decreased in FIB-4 score by one category where all subjects had scores < 1.45 at baseline.

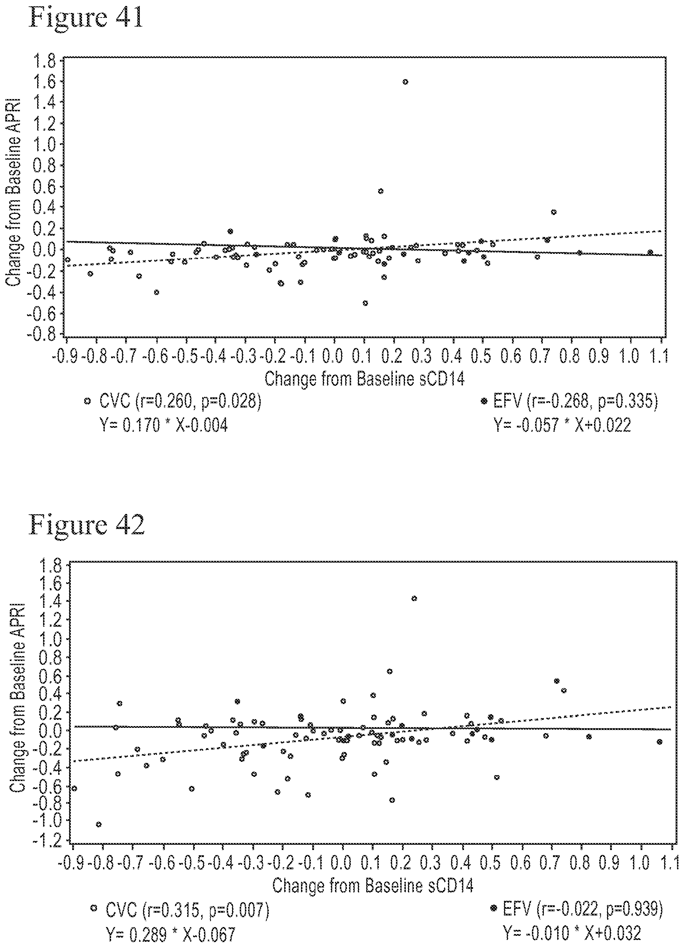

As mentioned above, in this study, CVC also had a significant effect on sCD14, an important marker of monocyte activation. In the same post-hoc analyses described above, statistically significant correlations were observed between changes in FIB-4 score and sCD14 levels in CVC-treated subjects at Week 24, and between changes in APRI and FIB-4 scores and sCD14 levels at Week 48. The Week 48 results are shown in Figure 41 and Figure 42.

No indication of inflammation was seen in clinical pathology parameters or in any tissue, including the liver, by microscopic evaluation at the high dose of 1000 mg/kg/day where plasma MCP-1 levels in the chronic (3- and 9-month) monkey toxicity studies were ∼ 5-fold over controls.

In fact, anti-fibrotic effects of CVC at the 100 mg/kg/day dose observed in the mouse model of NASH were seen in conjunction with significantly increased plasma MCP-1 levels. In addition, improvements in APRI and FIB-4 fibrosis index scores observed in CVC-treated subjects over 48 weeks occurred despite significant and sustained MCP-1 elevations. Also in this study, CVC was generally well tolerated in 115 subjects treated with CVC 100 mg and 200 mg for up to 48 weeks.

Changes in NAS and in hepatic fibrosis stage (NASH CRN system and Ishak) at Year 1 and 2 will be assessed by histology. Changes in morphometric quantitative assessment of collagen on liver biopsy will also be assessed. Correlations between efficacy endpoints and MCP-1 plasma levels will be evaluated to determine whether or not prolonged MCP-1 increases observed with CVC treatment pose a potential risk in subjects with liver fibrosis due to NASH.

### Example 16: Biomarkers of Inflammation and Immune Function

A dose-response was observed with CVC in increases over time of MCP-1, the ligand of CCR2, which is a chemokine receptor found on monocytes, while MCP-1 remained at baseline values in the EFV arm. The differences in changes from baseline of plasma MCP-1 between the EFV and CVC 100 mg and CVC 200 mg treatment arms were statistically significant (p<0.001) at Week 24 and Week 48, suggesting potent and dose-dependent CCR2 blockade by CVC. Furthermore, a decrease over the first 24 weeks was observed for sCD14, a biomarker of monocyte activation and an independent predictor of mortality in HIV infection, in both CVC treatment arms, while an increase was observed for sCD14 in the EFV arm during the same observation period. Between Weeks 24 and 48, sCD14 levels returned to baseline values in CVC-treated subjects whereas they continued to rise in EFV-treated subjects. The differences in changes from baseline between the CVC arms and the EFV arm were statistically significant (p<0.001) at Week 24 and Week 48 and also at Week 48 in a repeat analysis. These results indicate a potential effect of CVC on decreasing monocyte activation.

No meaningful differences between the treatment arms were observed in changes from Baseline in other inflammation biomarkers (hs-CRP, fibrinogen, IL-6, and D-dimer) and biomarkers of immune function (total CD38+ expression and total HLA DR+ expression on CD4+ T cells or on CD8+ T cells).

### Example 17: Study of CVC to Evaluate Hepatic Histological Improvement in NASH

Based on the nonclinical and clinical data indicating that CVC has anti-inflammatory and anti-fibrotic activity and is generally well tolerated, Tobira plans to investigate CVC in a Phase 2 study in subjects with hepatic fibrosis due to NASH. This Phase 2 study will evaluate the efficacy of CVC for the treatment of NASH in adult subjects with liver fibrosis who are at risk of disease progression due to the presence of at least one contributing factor, including type 2 diabetes mellitus (T2DM), high body mass index (BMI) (> 25 kg/m2) with at least 1 criterion of the metabolic syndrome (MS) as defined by the National Cholesterol Education Program (NCEP), bridging fibrosis, and/or definite NASH (NAS ≥ 5).

The Phase 2 study is designed to evaluate the potential of CVC to treat this serious condition and to address the significant unmet medical need of patients with hepatic fibrosis due to NASH. This study is a randomized, double-blind, placebo-controlled study designed to evaluate the efficacy and safety of CVC 150 mg when compared to placebo in subjects with hepatic fibrosis due to NASH. The study population consists of subjects with liver fibrosis (NASH Clinical Research Network [CRN] Stage 1-3) due to NASH (NAS ≥ 4) at risk of disease progression.

A dose of CVC 150 mg (DP7 formulation) will be evaluated for the treatment of NASH in subjects with liver fibrosis in Study 652-2-203 based on the following considerations:

CVC is expected to provide both anti-inflammatory and anti-fibrotic activity, primarily due to its antagonism of CCR2 and CCR5 co-receptors and the resulting effects on recruitment, migration and infiltration of pro-inflammatory monocytes to the site of liver injury. Therefore, a primary consideration for selecting a dose for use in this study is to ensure that CVC plasma exposures are sufficient to provide near maximal antagonism of CCR2 and CCR5.

CCR2 and CCR5 antagonism by CVC have been evaluated in in vitro and ex vivo studies and in 2 clinical studies of CVC in the treatment of HIV-1 infection (Phase 2a Study 652-2-201 and Phase 2b Study 652-2-202). In each case, potent and concentration-dependent antagonism of CCR2 and CCR5 was observed. Clinical evidence of CCR2 and CCR5 antagonism was established by measuring changes from baseline in plasma MCP-1 (a ligand of CCR2) concentrations and changes in plasma HIV-RNA (CCR5 co-receptor required for HIV entry), respectively, in these 2 Phase 2 Studies.

In Study 652-2-202, doses of CVC 100 mg and CVC 200 mg (DP6 formulation) were evaluated in 115 HIV-1 infected subjects for up to 48 weeks (mean [SE] duration of CVC intake: 41.1 [1.33] weeks) and were found to be effective and well tolerated in the treatment of HIV infection. Based on exposure-response analyses, which showed that increasing CVC plasma concentrations correlated with an improved virologic outcome, CVC 200 mg was considered an appropriate dose for further evaluation of CVC as an antiviral agent for the treatment of HIV infection in Phase 3 studies.

CVC plasma exposures, however, appear to be higher in non-HIV infected healthy volunteer subjects as compared to HIV-infected subjects when CVC is administered under the same dosing conditions (Studies 652-1-111, 652-1-110, 652-2-202). A dose of CVC 150 mg will be evaluated for the treatment of NASH in subjects with liver fibrosis in Study 652 2 203. Based on the referenced available data, this dose is considered to be in a therapeutically relevant range and is expected to provide exposures in subjects with NASH and liver fibrosis that are comparable to those of CVC 200 mg, which was evaluated in Study 652-2-202 and found to result in potent CCR2 and CCR5 antagonism.

A total of 250 subjects (125 subjects per treatment arm) are planned, and total study treatment duration will be 2 years. The study population will include subjects with NASH (NAS ≥ 4) and liver fibrosis (Stages 1 to 3 [NASH CRN system]) who are at increased risk of disease progression due to the presence of ≥ 1 contributing factor(s):

### Documented evidence of type 2 diabetes mellitus

High BMI (> 25 kg/m2) with at least 1 of the following criteria of the metabolic syndrome, as defined by the NCEP:
Central obesity: waist circumference ≥ 102 cm or 40 inches (male), ≥ 88 cm or 35 inches (female)
Dyslipidemia: TG ≥ 1.7 mmol/L (150 mg/dL)
Dyslipidemia: HDL-cholesterol < 40 mg/dL (male), < 50 mg/dL (female)
Blood pressure ≥ 130/85 mmHg (or treated for hypertension)
Fasting plasma glucose ≥ 6.1 mmol/L (110 mg/dL); or
Bridging fibrosis (NASH CRN Stage 3) and/or definite NASH (NAS ≥ 5).

There will be 2 treatment periods. Treatment Period 1 will consist of double-blind randomized treatment (CVC 150 mg or matching placebo) for 1 year. Subjects and investigators will remain blinded to treatment assignment during Period 1. During Treatment Period 2, subjects originally randomized to CVC 150 mg will continue to receive that treatment for an additional year, and subjects originally randomized to placebo will cross over from placebo to CVC 150 mg.

Subjects will receive study drug, once daily (QD), for 2 years. The study will comprise 2 treatment periods: Treatment Period 1 (first year) and Treatment Period 2 (second year). Eligible subjects will be assigned to receive CVC (n=126) or matching placebo (n=126) during the first year of treatment (Treatment Period 1). For Treatment Period 2, half of the placebo-treated subjects (randomized at Baseline) will cross-over to CVC and the other half will remain on placebo for the second year of treatment. At Baseline (Day 1), following Screening evaluations, eligible subjects will be assigned to the treatment arms using permuted block randomization stratified by NAS at Screening (4 or ≥ 5) and fibrosis stage (≤ 2 or > 2). Eligible subjects will be randomized in a 2:1:1 ratio to one of the following 3 treatment arms as shown in Figure 24.

**Table 24**

| **Arm** | **N** | **Treatment Period 1** | **Treatment Period 2** |
|---|---|---|---|
| A | 126 | CVC 150 mg, QD | CVC 150 mg, QD |
| B | 63 | Matching placebo, QD | CVC 150 mg, QD |
| C | 63 | Matching placebo, QD | Matching placebo, QD |

CVC and matching placebo will be administered as double-blinded study drug. Study drug (CVC/matching placebo) should be taken every morning with food.

The primary endpoint (Year 1) biopsy must be performed within 1 month prior to the end of Treatment Period 1 before starting Treatment Period 2. The final (Year 2) biopsy must be performed within 1 month prior to end of treatment with study drug.

Enrollment will be initiated at a limited number of sites until up to 20 subjects have been randomized and treated and safety data have been reviewed by the Data Monitoring Committee (DMC). The first DMC review will occur within 3 months of the first subject enrolled or, when up to 20 subjects have been randomized and at least 10 subjects have been treated for 1 month, whichever comes first. Subsequent enrollment of the remainder of study subjects will occur once the DMC has evaluated the safety data for these first 10-20 subjects and has determined that the study may continue.

During Treatment Period 1, all subjects will undergo safety assessments at Weeks 2 and 4 of Month 1. In addition, the first 20 subjects will undergo safety assessments at Weeks 1 and 3 of Month 1. All subjects will undergo study visit assessments every 2 weeks during Month 2, monthly visits during Months 3 to 6, and at Months 8, 10, and 12. During Treatment Period 2, subjects will undergo monthly visits during Months 13 to 15, and at Months 18, 21 and 24.

### Key Assessments

### During the study:

Liver biopsies will be taken at Screening, at the primary endpoint (Year 1: within 1 month prior to end of Treatment Period 1 and before starting Treatment Period 2), and at Year 2 (within 1 month prior to end of treatment)

Pro-inflammatory cytokines, biomarkers of inflammation, biomarkers of hepatocyte apoptosis, biomarkers of bacterial translocation, fasting metabolic parameters, renal parameters, and eGFR will be measured at Baseline and Months 3, 6, 12, 15, 18, and 24.

At sites where available, assessment of non invasive liver imaging (e.g., ultrasound transient elastography [TE], two-dimensional magnetic resonance elastography [MRE], acoustic radiation force impulse [ARFI]) will be performed at Baseline and at Months 6, 12, 18, and 24.

Pharmacokinetic samples for CVC will be collected at Baseline (pre-dose sample just before starting treatment), at Months 0.5, 3 and 15 (pre-dose and at least 1 hour post-dose), and at Months 6, 12, 18 and 24 (pre-dose).

Weight, waist circumference, hip circumference, arm circumference, and tricep skinfold will be performed at Baseline and at Months 3, 6, 12, 15, 18, and 24. Height will be performed at Screening and Month 12.

Physical examinations and laboratory analyses will be performed at each visit. ECGs will be performed at Baseline and at Months 3, 6, 12, 15, 18, and 24.

Adverse events and concomitant medications will be assessed at each visit.

The informed consent and patient education materials about NASH, liver fibrosis, and liver biopsy procedures will be reviewed at the screening visit.

Study drug diaries will be provided to each subject at the same time that study drug is dispensed. The diary will be reviewed at all On-treatment Visits and the Early Discontinuation Visit.

Subjects will return to the clinic 1 month after receiving their last treatment for an end of study follow-up evaluation.

The primary efficacy objective of the study will be to evaluate hepatic histological improvement in nonalcoholic fatty liver disease (NAFLD) activity score (NAS) at Year 1 relative to screening biopsy, defined by a minimum 2-point improvement in NAS with at least a 1-point improvement in more than 1 category and no concurrent worsening of fibrosis stage (with worsening defined as progression to bridging fibrosis or cirrhosis).

Secondary efficacy objectives include evaluation of the resolution of NASH with no concurrent worsening of fibrosis stage (worsening defined as progression to bridging fibrosis or cirrhosis) at Year 2; the resolution of NASH with no concurrent worsening of fibrosis stage (worsening defined as progression to bridging fibrosis or cirrhosis) at Year 1; the safety and tolerability of CVC over 1 and 2 years of treatment of NASH in adult subjects with liver fibrosis; characterization of the plasma PK of CVC in a population PK analysis; evaluation of the hepatic histological improvement in NAS at Year 2, defined by a minimum 2-point improvement in NAS with at least a 1-point improvement in more than 1 category and with no concurrent worsening of fibrosis stage (worsening defined as progression to bridging fibrosis or cirrhosis); evaluation of the efficacy of CVC versus placebo in adult subjects with liver fibrosis as determined by change in morphometric quantitative collagen on liver biopsy at Years 1 and 2; evaluation of the change in histologic fibrosis stage (nonalcoholic steatohepatitis clinical research network [NASH CRN] system and Ishak) at Years 1 and 2; evaluation of the change from in hepatic tissue fibrogenic protein (alpha-smooth muscle actin [a-SMA]) at Years 1 and 2; evaluation of the change from Baseline in noninvasive hepatic fibrosis markers (APRI, FIB-4, hyaluronic acid, FibroTest (FibroSure), NAFLD fibrosis score [NFS] and enhanced liver fibrosis test [ELF]) at Months 3, 6, 12, 15, 18, and 24; evaluation of the change from Baseline in biomarkers of hepatocyte apoptosis at Years 1 and 2; evaluation of the change from Baseline in liver parameters and fasting metabolic parameters at Months 3, 6, 12, 15, 18, and 24; evaluation of the change from Baseline in weight, BMI, waist circumference, waist-hip ratio, arm circumference, and tricep skinfold at Months 3, 6, 12, 15, 18, and 24.

Tertiary Objectives include evaluation of the change from Baseline in non-invasive liver imaging method (e.g., ultrasound transient elastography [TE], 2-dimensional magnetic resonance elastography [MRE], acoustic radiation force impulse [ARFI]) at Months 6, 12, 18, and 24 (at sites where available); the change from Baseline in pro-inflammatory cytokines and biomarkers of inflammation at Months 3, 6, 12, 15, 18, and 24; the change from Baseline in estimated glomerular filtration rate (eGFR) and in renal parameters at Months 3, 6, 12, 15, 18, and 24; and the change from Baseline in biomarkers associated with bacterial translocation at Months 3, 6, 12, 15, 18, and 24.

### Example 18: Evaluation of CVC combination therapy in the treatment of fibrosis

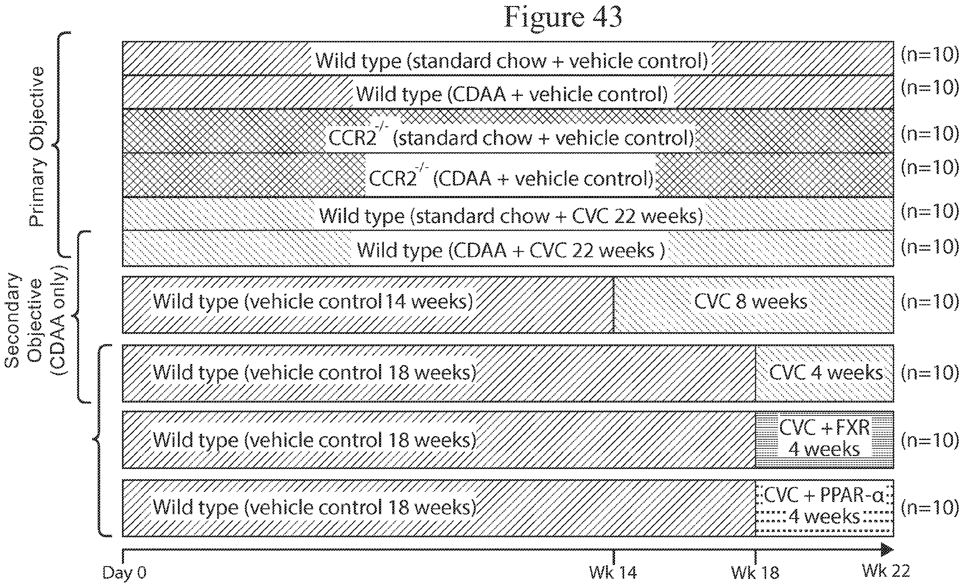

This non-clinical study aims to evaluate treatment with CVC alone or in combination with an FXR agonist or a PPAR-α and -δ agonist in the treatment of fibrosis. Briefly, CVC will be administered either alone (22 weeks, 8 weeks, and 4 weeks) or in combination either the FXR agonist or PPAR-α agonist simultaneously for four weeks. This study will be performed in a CDAA mouse model of NASH. Figure 43 shows the different treatment groups that will be used in this study.

The primary objective is to compare the treatment of wild type mice with vehicle control or CVC vs. CCR2-/- mice (standard chow vs. CDAA diet, administered over 22 weeks).

The Secondary objectives will be studied in the mice receiving CDAA diet only. We will compare 22 weeks of treatment of CVC with 8-week treatment (Weeks 14 to 22) and 4-week treatment (Weeks 18-22). Further, we will compare 4 weeks of treatment with (Week 18 to 22) of treatment with CVC alone vs. FXR agonist alone vs. PPAR-α and -δ alone vs. CVC and a FXR agonist (GW 4064) vs. CVC and a PPAR-α agonist (GW7647).

### Example 19: Cenicriviroc and Pioglitazone Co-administration Shows Favorable Pharmacokinetics and Safety in Healthy Subjects

**Background:** Cenicriviroc (CVC) is a potent, oral, once-daily dual CCR2/CCR5 antagonist evaluated in a Phase 2b study in adults with non-alcoholic steatohepatitis (NASH) and liver fibrosis. Pioglitazone (PGZ) is a PPARγ agonist used in patients with type 2 diabetes and has shown benefit in subjects with NASH. Both drugs are metabolized by CYP2C8 and CYP3A4, and CVC may be administered with PGZ or other antidiabetic agents.

**Methods:** This Phase 1, multiple-dose, open-label, 3-period fixed-sequence crossover study evaluated the PK and safety/tolerability of CVC and PGZ, used alone or in combination in healthy subjects. Treatment periods of 10 days consisted of: A: CVC 150mg QD; B: PGX 45 mg QD; C: CVC 150mg and PGZ 45mg QD. Subjects (n=20) were randomized to treatment sequence A/B/C (n=10) or B/A/C (n=10), with a 10-day washout between A and B. Steady-state PKs of CVC, PGZ and its active metabolites M-III and M-IV were evaluated. Plasma samples were collected prior to each dose and over 24 hours at the end of each treatment period. Cₘₐₓ, Cₘᵢₙ, tₘₐₓ, and AUC₀₋ₜₐᵤ were determined using non-compartmental methods. Geometric means, GMRs and CIs were calculated.

**Results:** CVC and PGZ exposures were slightly lower when both drugs were co-administered, with a modest decrease in steady-state Cₘₐₓ and AUC₀₋ₜₐᵤ for both drugs, whereas Cₘᵢₙ remained unchanged; GMRs for both drugs were ≥0.80. Effects of co-administration on PGS M-II and M-IV metabolites were less pronounced, with 90% CI for systemic exposure ratios within the 0.80-1.25 "no effect" range for all three PK parameters (data not shown). Combination treatment was well tolerated, with no serious AEs or AEs leading to discontinuation. All AEs were of mild severity, and the two most commonly reported were headache and fatigue.

**Conclusions:** Co-administration of CVC and PGZ was well tolerated and resulted in a modest interaction that was not considered clinically significant, which suggest that dose adjustment is not required when both drugs are used in combination.

The detailed description herein describes various aspects and embodiments of the invention, however, unless otherwise specified, none of those are intended to be limiting. Indeed, a person of skill in the art, having read this disclosure, will envision variations, alterations, and adjustments that can be made without departing from the scope and spirit of the invention, all of which should be considered to be part of the invention unless otherwise specified. Applicants thus envision that the invention described herein will be limited only by the appended claims.

References:
1. Saiman Y, Friedman SL. The role of chemokines in acute liver injury. 2012;3:213.
2. Zimmermann HW, Tacke F. Modification of chemokine pathways and immune cell infiltration as a novel therapeutic approach in liver inflammation and fibrosis. Inflamm Allergy Drug Targets. 2011;10:509-536.
3. Seki E, De Minicis S, Gwak GY, Kluwe J, Inokuchi S, Bursill CA, Llovet JM, Brenner DA, Schwabe RF. CCR1 and CCR5 promote hepatic fibrosis in mice. J Clin Invest. 2009;119:1858-1870.
4. Seki E, de Minicis S, Inokuchi S, Taura K, Miyai K, van Rooijen N, Schwabe RF, Brenner DA. CCR2 promotes hepatic fibrosis in mice. Hepatology. 2009;50:185-197.
5. Miura K, Yang L, van Rooijen N, Ohnishi H, Seki E. Hepatic recruitment of macrophages promotes nonalcoholic steatohepatitis through CCR2. Am J Physiol Gastrointest Liver Physiol. 2012;302:G1310-1331.
6. Mitchell C, Couton D, Couty JP, Anson M, Crain AM, Bizet V, Rénia L, Pol S, Mallet V, Gilgenkrantz H. Dual role of CCR2 in the constitution and the resolution of liver fibrosis in mice. Am J Pathol. 2009;174:1766-1775.
7. Xia Y, Entman ML, Wang Y. CCR2 regulates the uptake of bone marrow-derived fibroblasts in renal fibrosis. PLoS ONE 2013; 8(10): e77493. doi:10.1371/journal.pone.0077493
8. Karlmark KR, Wasmuth HE, Trautwein C, Tacke F. Chemokine-directed immune cell infiltration in acute and chronic liver disease. Expert Review Gastroenterology Hepatology. 2008; 2: 233-242
9. Vielhauer V, Anders H-J, Mack M, Cihak J, Strutz F, Stangassinger M, Luckow B, Gröne H-J, Schlöndorff D. Obstructive nephropathy in the mouse: Progressive fibrosis correlates with tubulointerstitial chemokine expression and accumulation of CC chemokine receptor 2- and 5-positive leukocytes. J Am Soc Nephrol 2001;12:1173-1187.
10. Segerer S, Mack M, Regele H, Kerjaschki D, Schlöndorff D. Expression of the C-C chemokine receptor 5 in human kidney disease. Kidney Int 1999; 56:52-64.
11. Wai C-T, Greenson J, Fontana R, Kalbfleisch J, Marrero J, Conjeevaram H, Lok A. A simple noninvasive index can predict both significant fibrosis and cirrhosis in patients with chronic hepatitis C. Hepatology 2003;38:518-526.
12. Vallet-Pichard A, Mallet V, Nalpas B, Verkarre V, Nalpas A, Dhalluin-Venier V, Fontaine H, Pol S. FIB-4: an inexpensive and accurate marker of fibrosis in HCV infection. Comparison with liver biopsy and FibroTest. Hepatology 2007;46:32-36.
13. Sandler NG, Wand H, Roque A, et al. Plasma levels of soluble CD14 independently predict mortality in HIV infection. JID 2011;203:780-790.
14. Brenchley J, Price DA, Schacker TW, Asher TE, Silvestri G, Rao S, Kazzaz Z, Bornstein E, Lambotte O, Altmann D, Blazar BR, Rodriguez B, Teixeira-Johnson L, Landay A, Martin JN, Hecht FM, Picker LJ, Lederman MM, Deeks SG, Douek DC. Microbial translocation is a cause of systemic immune activation in chronic HIV infection.Nature Medicine 2006;12:1365-1371.
15. Vajro P, Paolella G, Fasano A. Microbiota and Gut-Liver Axis: Their Influences on Obesity and Obesity-Related Liver Disease JPGN 2013;56: 461-468.
16. Roh YS, Seki E. Toll-like receptors in alcoholic liver disease, non-alcoholic steatohepatitis and carcinogenesis Journal of Gastroenterology and Hepatology 2013; 28: 38-42.
17. Ilan Y. Leaky gut and the liver: A role for bacterial translocation in nonalcoholic steatohepatitis. World J Gastroenterol 2012 June 7; 18: 2609-2618.
18. Petrasek J, Mandrekar P, Szabo G. Toll-Like Receptors in the Pathogenesis of Alcoholic Liver Disease. Gastroenterology Research and Practice 2010, Article ID 710381, 12 pages. doi:10.1155/2010/710381.
19. Sandler N, Koh C, Roque A, Eccleston J, Siegel R, Demino M, Kleiner D, Deeks S, Liang T-J, Heller T, Douek D. Host Response to Translocated Microbial Products Predicts Outcomes of Patients With HBV or HCV Infection. Gastroenterology 2011;141:1220-1230. doi:10.1053/j.gastro.2011.06.063.
20. Wiest R, Lawson M, Geuking M. Pathological bacterial translocation in liver cirrhosis. J Hepatology 2014; 60(1):197-209.
21. Shanab AA, Scully P, Crosbie O, Buckley M, O'Mahony L, Shanahan F, Gazareen S, Murphy E, Quigley EM. Small intestinal bacterial overgrowth in nonalcoholic steatohepatitis: association with toll-like receptor 4 expression and plasma levels of interleukin 8. Dig Dis Sci 2011; 56: 1524-1534
22. Henao-Mejia J, Elinav E, Jin C, Hao L, Mehal WZ, Strowig T, Thaiss CA, Kau AL, Eisenbarth SC, Jurczak MJ, Camporez J-P, Shulman GI, Gordon JI, Hoffman HM; Flavell RA. Inflammasome-mediated dysbiosis regulates progression of NAFLD and obesity. Nature 2012; 492: 179-185. doi:10.1038/nature10809.
23. Klibanov, Olga M.; Williams, Shannon H.; Iler, Cameron A (2010). "Cenicriviroc, an orally active CCR5 antagonist for the potential treatment of HIV infection". Current Opinion in Investigational Drugs 11 (8): 940-950.
24. Kleiner DE. et al., Hepatology, Design and validation of a histological scoring system for nonalcoholic fatty liver disease; 2005;41:1313-1321.
25. Fujii H et al. J. Atheroscler. Thromb. 2009;16:893.
26. Rangwala F et al. J. Pathol. 2011; 224:401.
27. Seki et al. CCR1 and CCR5 promote hepatic fibrosis in mice. J. Clin. Invest. 2009; 119(7):1858-1870.
28. Xu et al. Liver fibrosis: mechanisms of immune-mediated liver injury. Cellular & Mol. Immunol.; 2012; 9:296-301.
29. Karlmark et al, Expert Rev. Gastroenterol. Hepatol. 2(2), 233-242 (2008).
30. Mitchell C, et al. Dual role of CCR2 in the constitution and the resolution of liver fibrosis in mice. Am J Pathol. 2009;174:1766-1775.
31. Berres M-L, et al. Antagonism of the chemokine Ccl5 ameliorates experimental liver fibrosis in mice. Journal of Clin Invest. November 2010; 120(11): 4129-4140
32. Benyon, RC and MJ Arthur, Extracellular matrix degradation and the role of hepatic stellate cells.Semin Liver Dis. 2001 Aug;21(3):373-84.
33. Sheth SG, Chopra S. Natural history and management of nonalcoholic fatty liver disease in adults, UpToDate; 2014
34. Chalasani N, Younossi Z, Lavine JE, Diehl AM, Brunt EM, Cusi K, et al. The diagnosis and management of non-alcoholic fatty liver disease: Practice Guideline by the American Association for the Study of Liver Diseases, American College of Gastroenterology, and the American Gastroenterological Association. Hepatology 2012;55:2005-23.
35. McCullough AJ. The clinical features, diagnosis and natural history of nonalcoholic fatty liver disease. Clin Liver Dis 2004;8(3):521-33.
36. Loomba R, Sirlin CB, Schwimmer JB, Lavine JE. Advances in pediatric nonalcoholic fatty liver disease. Hepatology 2009;50(4):1282-93.
37. Torres DM, Williams CD, Harrison SA. Features, diagnosis, and treatment of nonalcoholic fatty liver disease. Clin Gastroenterol Hepatol 2012;10(8):837-58.
38. Schwenger KJ, Allard JP. Clinical approaches to non-alcoholic fatty liver disease. World J Gastroenterol 2014;20(7):1712-23.
39. Koo SH. Nonalcoholic fatty liver disease: molecular mechanisms for the hepatic steatosis. Clin Mol Hepatol 2013;19(3):210-5.
40. Attar BM, Van Thiel DH. Current concepts and management approaches in nonalcoholic fatty liver disease. ScientificWorld Journal 2013;2013:481893
41. Basaranoglu M, Basaranoglu G, Sentürk H. From fatty liver to fibrosis: a tale of "second hit". World J Gastroenterol 2013;19(8):1158-65.
42. Cohen JC, Horton JD, Hobbs HH. Human fatty liver disease: old questions and new insights. Science 2011;332(6037): 1519-23.
43. Matteoni CA, Younossi ZA, Gramlich T, et al. Nonalcoholic fatty liver disease: A spectrum of clinical and pathological severity. Gastroenterology 1999;116(6):1413-1419
44. World Gastroenterology Organisation Global Guidelines. Nonalcoholic Fatty liver Disease and Nonalcoholic Steatohepatitis. June 2012.
45. Ahmed MH, Abu EO, Byrne CD. Non-Alcoholic Fatty Liver Disease (NAFLD): new challenge for general practitioners and important burden for health authorities? Prim Care Diabetes. 2010;4:129-37.
46. Vernon G, Baranova A, Younossi ZM. Systematic review: the epidemiology and natural history of non-alcoholic fatty liver disease and nonalcoholic steatohepatitis in adults. Aliment Pharmacol Ther 2011;34:274-85.
47. The Gastroenterological Society of Australia/Australian Liver Association January 2013. http://static.squarespace.com/static/50ff0804e4b007d5a9abe0a5/t/53321aaee4b09f967eb0c7e5/ 1395792558684/gesa2013_revised%5B1%5D.pdf
48. Dixon JB, Bhathal PS, O'Brien PE. Nonalcoholic fatty liver disease: predictors of nonalcoholic steatohepatitis and liver fibrosis in the severely obese. Gastroenterol. 2001;121:91-100.
49. Williams CD, Stenger J, Asike MI, Torres DM, Shaw J, Contreras M, et al. Prevalence of nonalcoholic fatty liver disease and nonalcoholic steatohepatitis among a largely middle-aged population utilizing ultrasound and liver biopsy: a prospective study. Gastroenterology 2011;140:124-131.
50. Schattenberg JM, Schuppan D. Nonalcoholic steatohepatitis: the therapeutic challenge of a global epidemic. Curr Opin Lipidol 2011;22:479-88.
51. Bahrami H. Nonalcoholic fatty liver disease in developing countries. World J Gastroenterol 2005;11:3808-3809.
52. Tiniakos DG, Vos MB, Brunt EM. Nonalcoholic fatty liver disease: pathology and pathogenesis. Annu Rev Pathol 2010;5:145-71.
53. Vajro P, Paolella G, Fasano A. Microbiota and gut-liver axis: their influences on obesity and obesity-related liver disease. J Pediatr Gastroenterol Nutr 2013;56:461-8.
54. Roh YS, Seki E. Toll-like receptors in alcoholic liver disease, non-alcoholic steatohepatitis and carcinogenesis. J Gastroenterol Hepatol 2013;28 Suppl 1:38-42.
55. Ilan Y. Leaky gut and the liver: a role for bacterial translocation in nonalcoholic steatohepatitis. World J Gastroenterol 2012;18:2609-18.
56. Moschen AR, Kaser S, Tilg H. Non-alcoholic steatohepatitis: a microbiota-driven disease. Trends Endocrinol Metab 2013;24:537-45.
57. Sanyal AJ, Campbell-Sargent C, Mirshahi F, Rizzo WB, Contos MJ, Sterling RK, et al. Nonalcoholic steatohepatitis: association of insulin resistance and mitochondrial abnormalities. Gastroenterology 2001;120:1183-92.
58. McClain CJ, Mokshagundam SP, Barve SS, Song Z, Hill DB, Chen T, et al. Mechanisms of non-alcoholic steatohepatitis. Alcohol 2004;34:67-79.
59. Day CP, Saksena S. Non-alcoholic steatohepatitis: definitions and pathogenesis. J Gastroenterol Hepatol. 2002;17 Suppl 3:S377-S84.
60. Marra F, Gastaldelli A, Svegliati Baroni G, Tell G, Tiribelli C. Molecular basis and mechanisms of progression of non-alcoholic steatohepatitis. Trends Mol Med. 2008;14:72-81
61. Charlton MR, Burns JM, Pederson RA, Watt KD, Heimbach JK, Dierkhising RA. Frequency and outcomes of liver transplantation for nonalcoholic steatohepatitis in the United States. Gastroenterol. 2011;141:1249-53.
62. Vatche G. Agopian et al. Liver Transplantation for Nonalcoholic Steatohepatitis. Annals of Surgery 2012; 256(4); 624-633.
63. McCullough AJ. Epidemiology of the metabolic syndrome in the USA. J Dig Dis. 2011;12:333-40.

### Aspects of the Invention

1. A method of treating fibrosis or a fibrotic disease or condition in a subject in need thereof comprising co-administering to the subject a therapeutically effective amount of cenicriviroc or a salt or solvate thereof; and one or more additional active agents.
2. The method of 1, wherein the additional active agent is selected from the group consisting of a farnesoid X receptor (FXR) agonist, high dose vitamin E (> 400 iU/d), a peroxisome proliferator-activated receptor alpha (PPAR-α) agonist, PPAR-γ agonist, and PPAR-δ agonist, and chemokine antagonist.
3. The method of 1, wherein the additional active agent is selected from the group consisting of obeticholic acid, pioglitazone, 3-[2-[2-Chloro-4-[[3-(2,6-dichlorophenyl)-5-(1-methylethyl)-4-isoxazolyl]methoxy]phenyl]ethenyl]benzoic acid (GW4064), 2-methyl-2-[[4-[2-[[(cyclohexylamino)carbonyl](4-cyclohexylbutyl)amino]ethyl]phenyl]thio]-propanoic acid (GW7647), and 2-[2,6 dimethyl-4-[3-[4-(methylthio)phenyl]-3-oxo-1(E)-propenyl]phenoxyl]-2-methylpropanoic acid (GFT505), and BX471.
4. The method of 1, wherein the fibrosis or fibrotic disease or condition is liver fibrosis or renal fibrosis.
5. The method of 1, wherein the cenicriviroc or a salt or solvate thereof is formulated as a pharmaceutical composition comprising cenicriviroc or a salt or solvate thereof and fumaric acid.
6. The method of 4, wherein the liver fibrosis is associated with non-alcoholic steatohepatitis (NASH).
7. The method of 4, wherein the liver fibrosis is associated with non-alcoholic fatty liver disease (NAFLD).
8. The method of 4, wherein the liver fibrosis is associated with emerging cirrhosis.
9. The method of 4, wherein the liver fibrosis comprises non-cirrhotic hepatic fibrosis.
10. The method of 4, wherein the subject is infected by human immunodeficiency virus (HIV).
11. The method of any one of 1 to 10, wherein the subject has a disease or condition selected from the group consisting of alcoholic liver disease, HIV and HCV co-infection, viral hepatitis (such as HBV or HCV infection), type 2 diabetes mellitus (T2DM), metabolic syndrome (MS), and a combination thereof.
12. A method of treating NASH in a subject in need thereof comprising co-administering to the subject a therapeutically effective amount of cenicriviroc, or a salt or solvate thereof; wherein the NASH is associated with type 2 diabetes mellitus (T2DM); and one or more additional active agents.
13. A method of treating NASH in a subject in need thereof comprising co-administering to the subject a therapeutically effective amount of cenicriviroc, or a salt or solvate thereof; wherein the NASH is associated with metabolic syndrome (MS); and one or more additional active agents.
14. A method of treating NASH in a subject in need thereof comprising co-administering to the subject a therapeutically effective amount of cenicriviroc, or a salt or solvate thereof; and one or more additional active agents; wherein the NASH is associated with HIV and HCV co-infection.
15. The method of any one of 12, 13, or 14, wherein the additional active agent is selected from the group consisting of a farnesoid X receptor (FXR) agonist, high dose vitamin E (> 400 iU/d), a peroxisome proliferator-activated receptor alpha (PPAR-α) agonist, PPAR-γ agonist, PPAR-δ agonist, and chemokine antagonist.
16. The method of any one of 12, 13, or 14, wherein the additional active agent is selected from the group consisting of obeticholic acid, pioglitazone, 3-[2-[2-Chloro-4-[[3-(2,6-dichlorophenyl)-5-(1-methylethyl)-4-isoxazolyl]methoxy]phenyl]ethenyl]benzoic acid (GW4064), 2-methyl-2-[[4-[2-[[(cyclohexylamino)carbonyl](4-cyclohexylbutyl)amino]ethyl]phenyl]thio]-propanoic acid (GW7647), 2-[2,6 dimethyl-4-[3-[4-(methylthio)phenyl]-3-oxo-1(E)-propenyl]phenoxyl]-2-methylpropanoic acid (GFT505), and BX471.
17. The method of any of the preceding , wherein the cenicriviroc or salt or solvate thereof is formulated as an oral composition.
18. The method of any of the preceding , wherein the cenicriviroc or salt or solvate thereof is administered once per day or twice per day.
19. The method of any of the preceding , wherein the co-administration comprises simultaneous administration, sequential administration, overlapping administration, interval administration, continuous administration, or a combination thereof.
20. The method of 19, wherein the co-administration is carried out for one or more treatment cycles.
21. The method of 20, wherein the co-administration is carried out for 1 to 24 treatment cycles.
22. The method of 20, wherein each of the treatment cycle comprises about 7 or more days.
23. The method of 20, wherein each of the treatment cycle comprises about 28 or more days.
24. The method of any of the preceding , wherein the co-administration comprises oral administration, parenteral administration, or a combination thereof.
25. The method of 24, wherein the parenteral administration comprises intravenous administration, intraarterial administration, intramuscular administration, subcutaneous administration, intraosseous administration, intrathecal administration, or a combination thereof.
26. The method of 24, wherein cenicriviroc or a salt or solvate thereof is administered orally; and the additional active agent is administered orally or parenterally.
27. The method of any of the preceding, wherein the co-administration comprises one or more treatment cycles, and each treatment cycle comprises about 28 days.
28. The method of any of the preceding, wherein the co-administration comprises simultaneous administration.
29. The method of 28, wherein cenicriviroc or a salt or solvate thereof and the additional active agent are co-administered simultaneously for about 28 days or more.
30. The method of any of the preceding , comprising detecting a level of one or more biological molecules in the subject treated for fibrosis or the fibrotic disease or condition, and determining a treatment regimen based on an increase or decrease in the level of one or more biological molecules, wherein the biological molecule is selected from the group consisting of lipopolysaccharide (LPS), LPs-binding protein (LBP), 16S rDNA, sCD14, intestinal fatty acid binding protein (I-FABP), zonulin-1, Collagen 1a1 and 3a1, TGF-β, fibronectin-1, hs-CRP, IL-1β, IL-6, IL-33, fibrinogen, MCP-1, MIP-1α, and -1β, RANTES, sCD163, TGF-β, TNF-α, a biomarker of hepatocyte apoptosis such as CK-18 (caspase-cleaved and total), and a combination thereof.
31. The method of any of the preceding, comprising detecting a level of one or biological molecules in the subject treated for fibrosis or the fibrotic disease or condition, wherein an increase or decrease in the level of one or more biological molecules compared to a predetermined standard level is predictive of the treatment efficacy of fibrosis or the fibrotic disease or condition, wherein the biological molecule is selected from the group consisting of lipopolysaccharide (LPS), LPs-binding protein (LBP), 16S rDNA, sCD14, intestinal fatty acid binding protein (I-FABP), zonulin-1, Collagen 1a1 and 3a1, TGF-β, fibronectin-1, hs-CRP, IL-1β, IL-6, IL-33, fibrinogen, MCP-1, MIP-1α, and -1β, RANTES, sCD163, TGF-β, TNF-α, a biomarker of hepatocyte apoptosis such as CK-18 (caspase-cleaved and total), and a combination thereof.
32. The method of 30 or 31, where the one or more biological molecules are measured in a biological sample from a subject treated for fibrosis or the fibrotic disease or condition.
33. The method of 34, where the biological sample is selected from blood, skin, hair follicles, saliva, oral mucous, vaginal mucous, sweat, tears, epithelial tissues, urine, semen, seminal fluid, seminal plasma, prostatic fluid, pre-ejaculatory fluid (Cowper's fluid), excreta, biopsy, ascites, cerebrospinal fluid, lymph, brain, and tissue extract sample or biopsy sample.
34. A pharmaceutical composition comprising a therapeutically effective amount of cenicriviroc, or a salt or solvate thereof; and one or more additional active agents.
35. The pharmaceutical composition of 34, further comprising one or more pharmaceutically acceptable excipients.
36. The pharmaceutical composition of 35, wherein the pharmaceutically acceptable excipient comprises fumaric acid.
37. A combination package comprising
   (a) at least one individual dose of cenicriviroc, or a salt or solvate thereof; and
   (b) at least one individual dose of one or more additional active agent.
38. The combination package of 37, further comprising an instruction document providing a protocol for co-administering (a) and (b).
39. A method of distributing an antifibrotic agent comprising distributing to a subject a predetermined amount of a first pharmaceutical composition comprising cenicriviroc, or a salt or solvate thereof, in combination with a predetermined amount of a second pharmaceutical composition comprising at least one or more active agents.
40. A method of distributing an antifibrotic agent comprising distributing to a subject a predetermined amount of a first pharmaceutical composition comprising cenicriviroc, or a salt or solvate thereof, in combination with an instruction of administering the first pharmaceutical composition with a predetermined amount of a second pharmaceutical composition comprising at least one or more active agents.
41. The use of co-administration of a therapeutically effective amount of cenicriviroc, or a salt or solvate thereof, and one or more additional active agents for treating fibrosis or a fibrotic disease or condition in a subject in need thereof.
42. The use of co-administration of a therapeutically effective amount of cenicriviroc or a salt or solvate thereof, and one or more additional active agents for treating NASH associated with type 2 diabetes mellitus (T2DM) in a subject in need thereof.
43. The use of co-administration of a predetermined amount of a first pharmaceutical composition comprising cenicriviroc or a salt or solvate thereof, in combination with a predetermined amount of a second pharmaceutical composition comprising at least one or more active agents for distributing an antifibrotic agent in a subject.
44. The use of co-administration of a predetermined amount of a first pharmaceutical composition comprising cenicriviroc, or a salt or solvate thereof, in combination with an instruction of administering the first pharmaceutical composition with a predetermined amount of a second pharmaceutical composition comprising at least one or more active agents for distributing an antifibrotic agent in a subject.

## Claims

1. A pharmaceutical composition comprising cenicriviroc or a salt or solvate thereof; and one or more additional active agents selected from the group consisting of a farnesoid X receptor (FXR) agonist, high dose vitamin E (> 400 iU/d), a peroxisome proliferator-activated receptor alpha (PPAR-α) agonist, PPAR-γ agonist, and PPAR-δ agonist, and a chemokine antagonist, wherein said additional active agent is not obeticholic acid or 2-[2,6 dimethyl-4-[3-[4-(methylthio)phenyl]-3-oxo-1(E)-propenyl]phenoxyl]-2-methylpropanoic acid (GFT505), for use in the treatment of liver fibrosis or renal fibrosis.

2. A pharmaceutical composition according to claim 1, wherein the one or more additional active agents are selected from the group consisting of a high dose vitamin E (> 400 iU/d), pioglitazone, 3-[2-[2-Chloro-4-[[3-(2,6-dichlorophenyl)-5-(1-methylethyl)-4-isoxazolyl]methoxy]phenyl]ethenyl]benzoicacid (GW4064), 2-methyl-2-[[4-[2-[[(cyclohexylamino)carbonyl](4-cyclohexylbutyl)amino]ethyl]phenyl]thio]-propanoic acid (GW7647), and BX471.

3. The composition for use according to either claim 1 or claim 2, wherein the pharmaceutical composition further comprises fumaric acid.

4. The composition for use according to any one of claims 1 to 3, wherein the liver fibrosis is associated with non-alcoholic steatohepatitis (NASH), non-alcoholic fatty liver disease (NAFLD), emerging cirrhosis, non-cirrhotic hepatic fibrosis, or human immunodeficiency virus (HIV) infection.

5. The composition for use according to any one of claims 1 to 3, wherein the subject has a disease or condition selected from the group consisting of alcoholic liver disease, HIV and HCV co- infection, viral hepatitis (such as HBV or HCV infection), type 2 diabetes mellitus (T2DM), metabolic syndrome (MS), and a combination thereof.

6. A therapeutically effective amount of cenicriviroc, or a salt or solvate thereof, and one or more additional active agents selected from a farnesoid X receptor (FXR) agonist, high dose vitamin E (>400 iU/d), a peroxisome proliferator-activated receptor alpha (PPAR-α) agonist, PPAR-γ agonist, and PPAR-δ agonist, and a chemokine antagonist, wherein said additional active agent is not obeticholic acid or 2-[2,6 dimethyl-4-[3-[4-(methylthio)phenyl]-3-oxo-1(E)-propenyl]phenoxyl]-2-methylpropanoic acid (GFT505), for use in the treatment of NASH, wherein the NASH is associated with type 2 diabetes mellitus (T2DM), Metabolic Syndrome (MS) or HIV and HCV co-infection.

7. The cenicriviroc and one or more additional active agents for use according to claim 6, wherein the one or more additional active agents are selected from the group consisting of high dose vitamin E (>400 iU/d), pioglitazone, 3-[2-[2-Chloro-4-[[3-(2,6-dichlorophenyl)-5-(1-methylethyl)-4-isoxazolyl]methoxy]phenyl]ethenyl]benzoic acid (GW4064), 2-methyl-2-[[4-[2-[[(cyclohexylamino)carbonyl](4-cyclohexylbutyl)amino ]ethyl]phenyl]thio]-propanoic acid (GW7647), and BX471.

8. The cenicriviroc and one or more additional agents for use according to claim 6 or claim 7, wherein the cenicriviroc or salt or solvate thereof is formulated as an oral composition.

9. The cenicriviroc and one or more additional agents for use according to any one of claims 6 to 8, wherein the cenicriviroc or salt or solvate thereof is administered once per day or twice per day.

10. The cenicriviroc and one or more additional agents for use according to claim 9, wherein the administration comprises simultaneous administration, sequential administration, overlapping administration, interval administration, continuous administration, or a combination thereof.

11. The cenicriviroc and one or more additional agents for use according to claim 9, wherein the administration is carried out for one or more treatment cycles, preferably each of the treatment cycle comprises about 7 or more days.

12. The composition for use according to any one of claims 1 to 3, wherein said treatment comprises detecting a level of one or more biological molecules in the subject treated for fibrosis or the fibrotic disease or condition, and determining a treatment regimen based on an increase or decrease in the level of one or more biological molecules, wherein the biological molecule is selected from the group consisting of lipopolysaccharide (LPS), LPs-binding protein (LBP), 16S rDNA, sCD14, intestinal fatty acid binding protein (1-FABP), zonulin-1, Collagen lal and 3al, TGF-β, fibronectin-1, hs-CRP, IL-1 , IL-6, IL-33, fibrinogen, MCP-1, MIP-1α, and -1β, RANTES, sCD163, TGF-β, TNF-α, abiomarker of hepatocyte apoptosis such as CK-18 (caspase-cleaved and total), and a combination thereof.

13. The composition for use according to any one of claims 1 to 3, wherein said treatment comprises detecting a level of one or biological molecules in the subject treated for fibrosis or the fibrotic disease or condition, wherein an increase or decrease in the level of one or more biological molecules compared to a predetermined standard level is predictive of the treatment efficacy of fibrosis or the fibrotic disease or condition, wherein the biological molecule is selected from the group consisting of lipopolysaccharide (LPS), LPs-binding protein (LBP), 16S rDNA, sCD14, intestinal fatty acid binding protein (1-FABP), zonulin-1, Collagen lal and 3al, TGF- , fibronectin-1, hs-CRP, IL-1, IL-6, IL-33, fibrinogen, MCP-1, MIP-1α, and -1β, RANTES, sCD163, TGF-β, TNF-α, a biomarker of hepatocyte apoptosis such as CK-18 (caspase-cleaved and total), and a combination thereof.

14. The composition for use according to claim 12 or claim 13, where the one or more biological molecules are measured in a biological sample from a subject treated for fibrosis or the fibrotic disease or condition.

15. The composition for use according to claim 14, where the biological sample is selected from blood, skin, hair follicles, saliva, oral mucous, vaginal mucous, sweat, tears, epithelial tissues, urine, semen, seminal fluid, seminal plasma, prostatic fluid, pre-ejaculatory fluid (Cowper's fluid), excreta, biopsy, ascites, cerebrospinal fluid, lymph, brain, and tissue extract sample or biopsy sample.
